(19) Europäisches Patentamt
European Patent Office
Office européen des brevets

(11) **EP 4 112 601 A1**

(12) **EUROPEAN PATENT APPLICATION**

(43) Date of publication:
**04.01.2023 Bulletin 2023/01**

(21) Application number: **22165019.5**

(22) Date of filing: **29.03.2022**

(51) International Patent Classification (IPC):
***C07D 209/88*** *(2006.01)* ***C07D 405/12*** *(2006.01)*
***C07D 409/12*** *(2006.01)* ***H01L 51/00*** *(2006.01)*
***H05B 45/60*** *(2022.01)*

(52) Cooperative Patent Classification (CPC):
**C07D 209/88; C07D 405/12; C07D 409/12; H01L 51/0055; H01L 51/006; H01L 51/0061; H01L 51/0072; H01L 51/0073;** H01L 51/5056

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.07.2021 KR 20210087394**

(71) Applicant: **Samsung Display Co., Ltd.**
**Yongin-si, Gyeonggi-do 17113 (KR)**

(72) Inventors:
- **KIM, Dongjun**
  **17113 Yongin-si (KR)**
- **KIM, Minji**
  **17113 Yongin-si (KR)**
- **PAK, Hankyu**
  **17113 Yongin-si (KR)**
- **JEONG, Eunjae**
  **17113 Yongin-si (KR)**
- **HAN, Sanghyun**
  **17113 Yongin-si (KR)**
- **YOO, Byeongwook**
  **17113 Yongin-si (KR)**
- **JO, Sohee**
  **17113 Yongin-si (KR)**
- **PARK, Hyunbin**
  **17113 Yongin-si (KR)**

(74) Representative: **Russell, Tim et al**
**Venner Shipley LLP**
**200 Aldersgate**
**London EC1A 4HD (GB)**

(54) **CONDENSED CYCLIC COMPOUND, LIGHT-EMITTING DEVICE INCLUDING THE SAME, AND ELECTRONIC APPARATUS INCLUDING THE LIGHT-EMITTING DEVICE**

(57) A light-emitting device and an electronic apparatus including the light-emitting device include a novel condensed cyclic compound represented by Formula 1, in which $Z_1$ is a group represented by Formula 2.

**Formula 1**

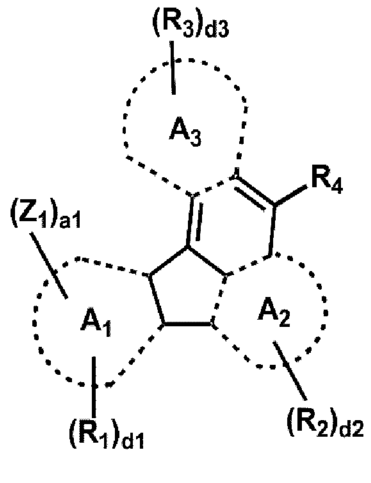

**Formula 2**

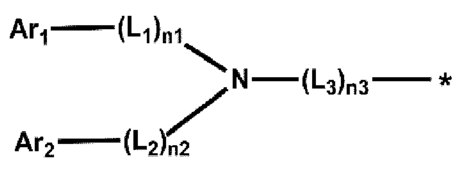
.

EP 4 112 601 A1

## Description

### CROSS-REFERENCE TO RELATED APPLICATION

This application claims priority to and the benefit of Korean Patent Application No. 10-2021-0087394, filed on July 2, 2021, in the Korean Intellectual Property Office, the entire content of which is incorporated herein by reference.

### BACKGROUND

### 1. Field

One or more aspects of embodiments of the present disclosure relate to a condensed cyclic compound, a light-emitting device including the same, and an electronic apparatus including the light-emitting device.

### 2. Description of the Related Art

From among light-emitting devices, organic light-emitting devices are self-emissive devices that, as compared with devices of the related art, may have wide viewing angles, high contrast ratios, short response times, and/or excellent or suitable characteristics in terms of luminance, driving voltage, and/or response speed, and may produce full-color images.

In an example organic light-emitting device, a first electrode is arranged on a substrate, and a hole transport region, an emission layer, an electron transport region, and a second electrode are sequentially stacked on the first electrode. Holes provided from the first electrode move toward the emission layer through the hole transport region, and electrons provided from the second electrode move toward the emission layer through the electron transport region. Carriers (such as holes and electrons) may recombine in the emission layer to produce excitons. These excitons may transition from an excited state to the ground state, thereby generating light.

### SUMMARY

The invention is defined by the claims.

One or more aspects of embodiments of the present disclosure are directed toward a condensed cyclic compound, a light-emitting device including the same, and an electronic apparatus including the light-emitting device.

Additional aspects will be set forth in part in the description that follows, and in part, will be apparent from the description, or may be learned by practice of the presented embodiments of the disclosure.

One or more embodiments of the present disclosure provide a condensed cyclic compound represented by Formula 1:

Formula 1

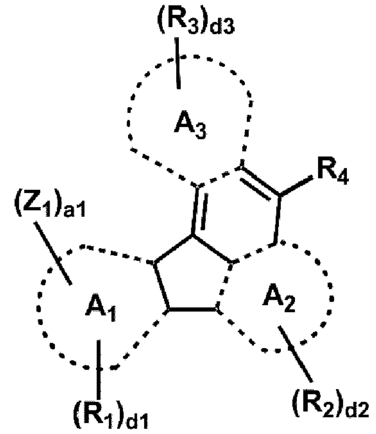

wherein in Formula 1,

$A_1$ to $A_3$ are each independently a $C_3$-$C_{60}$ carbocyclic group or a $C_1$-$C_{60}$ heterocyclic group,

$R_1$ to $R_4$ are each independently hydrogen, deuterium, -F, -Cl, -Br, -I, a hydroxyl group, a cyano group, a nitro group, a $C_1$-$C_{60}$ alkyl group unsubstituted or substituted with at least one $R_{10a}$, a $C_2$-$C_{60}$ alkenyl group unsubstituted or substituted with at least one $R_{10a}$, a $C_2$-$C_{60}$ alkynyl group unsubstituted or substituted with at least one $R_{10a}$, a $C_1$-$C_{60}$ alkoxy group unsubstituted or substituted with at least one $R_{10a}$, a $C_3$-$C_{60}$ carbocyclic group unsubstituted or substituted with at least one $R_{10a}$, a $C_1$-$C_{60}$ heterocyclic group unsubstituted or substituted with at least one $R_{10a}$, a $C_6$-$C_{60}$ aryloxy group unsubstituted or substituted with at least one $R_{10a}$, a $C_6$-$C_{60}$ arylthio group unsubstituted or substituted with at least one $R_{10a}$, -Si($Q_1$)($Q_2$)($Q_3$), -B($Q_1$)($Q_2$), - C(=O)($Q_1$), -S(=O)$_2$($Q_1$), or -P(=O)($Q_1$)($Q_2$),
d1 to d3 are each independently an integer from 0 to 10,
a1 is an integer from 1 to 4,
$Z_1$ is a group represented by Formula 2:

Formula 2

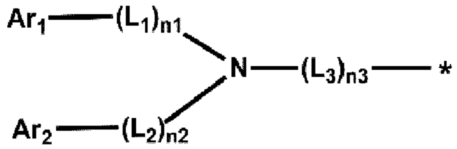

wherein in Formula 2,

$L_1$ to $L_3$ are each independently a single bond, a $C_5$-$C_{60}$ carbocyclic group unsubstituted or substituted with at least one $R_{10a}$, or a $C_1$-$C_{60}$ heterocyclic group unsubstituted or substituted with at least one $R_{10a}$,
n1 to n3 are each independently an integer from 1 to 3,
$Ar_1$ and $Ar_2$ are each independently a $C_5$-$C_{60}$ carbocyclic group unsubstituted or substituted with at least one $R_{10a}$ or a $C_1$-$C_{60}$ heterocyclic group unsubstituted or substituted with at least one $R_{10a}$,
* indicates a binding site to a neighboring atom; and
wherein each $R_{10a}$ is individually:
deuterium, -F, -Cl, -Br, -I, a hydroxyl group, a cyano group, or a nitro group;
a $C_1$-$C_{60}$ alkyl group, a $C_2$-$C_{60}$ alkenyl group, a $C_2$-$C_{60}$ alkynyl group, or a C1-$C_{60}$ alkoxy group, each unsubstituted or substituted with deuterium, -F, -Cl, -Br, -I, a hydroxyl group, a cyano group, a nitro group, a $C_3$-$C_{60}$ carbocyclic group, a $C_1$-$C_{60}$ heterocyclic group, a $C_6$-$C_{60}$ aryloxy group, a $C_6$-$C_{60}$ arylthio group, -Si($Q_{11}$)($Q_{12}$)($Q_{13}$), -N($Q_{11}$)($Q_{12}$), -B($Q_{11}$)($Q_{12}$), -C(=O)($Q_{11}$), -S(=O)$_2$($Q_{11}$), -P(=O)($Q_{11}$)($Q_{12}$), or any combination thereof;
a $C_3$-$C_{60}$ carbocyclic group, a $C_1$-$C_{60}$ heterocyclic group, a $C_6$-$C_{60}$ aryloxy group, or a $C_6$-$C_{60}$ arylthio group, each unsubstituted or substituted with deuterium, -F, -Cl, -Br, -I, a hydroxyl group, a cyano group, a nitro group, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{60}$ carbocyclic group, a $C_1$-$C_{60}$ heterocyclic group, a $C_6$-$C_{60}$ aryloxy group, a $C_6$-$C_{60}$ arylthio group, - Si($Q_{21}$)($Q_{22}$)($Q_{23}$), -N($Q_{21}$)($Q_{22}$), -B($Q_{21}$)($Q_{22}$), -C(=O)($Q_{21}$), -S(=O)$_2$($Q_{21}$), - P(=O)($Q_{21}$)($Q_{22}$), or any combination thereof; or
-Si($Q_{31}$)($Q_{32}$)($Q_{33}$), -N($Q_{31}$)($Q_{32}$), -B($Q_{31}$)($Q_{32}$), -C(=O)($Q_{31}$), -S(=O)$_2$($Q_{31}$), or - P(=O)($Q_{31}$)($Q_{32}$), and
wherein $Q_1$ to $Q_3$, $Q_{11}$ to $Q_{13}$, $Q_{21}$ to $Q_{23}$, and $Q_{31}$ to $Q_{33}$ are each independently: hydrogen; deuterium; -F; -Cl; -Br; -I; a hydroxyl group; a cyano group; a nitro group; a $C_1$-$C_{60}$ alkyl group; a $C_2$-$C_{60}$ alkenyl group; a $C_2$-$C_{60}$ alkynyl group; a C1-$C_{60}$ alkoxy group; or a $C_3$-$C_{60}$ carbocyclic group or a $C_1$-$C_{60}$ heterocyclic group, each unsubstituted or substituted with deuterium, -F, a cyano group, a $C_1$-$C_{60}$ alkyl group, a $C_1$-$C_{60}$ alkoxy group, a phenyl group, a biphenyl group, or any combination thereof.

One or more embodiments of the present disclosure provide a light-emitting device comprising a first electrode,

a second electrode facing the first electrode,
an interlayer between the first electrode and the second electrode and comprising an emission layer, and
the interlayer comrpises at least one condensed cyclic compound as described herein.

One or more embodiments of the present disclosure provide an electronic apparatus comprising the light-emitting device as described herein.
An electronic apparatus comprising: the light-emitting device as described herein; and a thin-film transistor, wherein: the thin-film transistor comprises a source electrode and a drain electrode, and the first electrode of the light-emitting device is electrically connected to the source electrode or the drain electrode.

## BRIEF DESCRIPTION OF THE DRAWINGS

The above and other aspects, features, and advantages of certain embodiments of the disclosure will be more apparent from the following description taken in conjunction with the accompanying drawings, in which:

FIG. 1 is a schematic view of a light-emitting device according to an embodiment; and
FIGS. 2 and 3 are each a schematic cross-sectional view of a light-emitting apparatus according to an embodiment of the present disclosure.

## DETAILED DESCRIPTION

Reference will now be made in more detail to embodiments, examples of which are illustrated in the accompanying drawings, wherein like reference numerals refer to like elements throughout the specification, and duplicative descriptions thereof may not be provided. In this regard, the present embodiments may have different forms and should not be construed as being limited to the descriptions set forth herein. Accordingly, the embodiments are merely described below, by referring to the drawings, to explain aspects of the present description. As used herein, the term "and/or" includes any and all combinations of one or more of the associated listed items. As used herein, expressions such as "at least one of", "one of", and "selected from", when preceding a list of elements, modify the entire list of elements and do not modify the individual elements of the list. For example, the expression "at least one of a, b and c," "at least one of a, b or c," and "at least one of a, b and/or c" may indicate only a, only b, only c, both (e.g., simultaneously) a and b, both (e.g., simultaneously) a and c, both (e.g., simultaneously) b and c, all of a, b, and c, or variations thereof.
As used herein, singular forms such as "a," "an," and "the" are intended to include the plural forms as well, unless the context clearly indicates otherwise. It will be further understood that the terms "includes," "including," "comprises," and/or "comprising," when used in this specification, specify the presence of stated features, steps, operations, elements, and/or components, but do not preclude the presence or addition of one or more other features, steps, operations, elements, components, and/or groups thereof.
As used herein, the terms "use," "using," and "used" may be considered synonymous with the terms "utilize," "utilizing," and "utilized," respectively. The term "may" will be understood to refer to "one or more embodiments," some of which include the described element and some of which exclude that element and/or include an alternate element. Similarly, alternative language such as "or" refers to "one or more embodiments," each including a corresponding listed item.
One or more embodiments of the present disclosure provide a condensed cyclic compound represented by Formula 1:

**Formula 1**

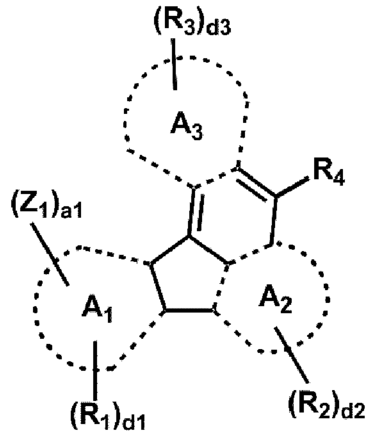

wherein, in Formula 1,
$A_1$ to $A_3$ are each independently a $C_5$-$C_{60}$ carbocyclic group or a $C_1$-$C_{60}$ heterocyclic group.
In an embodiment, $A_1$ to $A_3$ may each independently be a benzene group, a naphthalene group, an anthracene group, a phenanthrene group, a triphenylene group, a pyrene group, a chrysene group, cyclopentadiene group, a 1,2,3,4-tetrahydronaphthalene group, a thiophene group, a furan group, an indole group, a benzoborole group, a benzophosphole group, an indene group, a benzosilole group, a benzogermole group, a benzothiophene group, a benzoselenophene group, a benzofuran group, a carbazole group, a dibenzoborole group, a dibenzophosphole group, a fluorene group, a

dibenzosilole group, a dibenzogermole group, a dibenzothiophene group, a dibenzoselenophene group, a dibenzofuran group, a dibenzothiophene 5-oxide group, a 9H-fluorene-9-one group, a dibenzothiophene 5,5-dioxide group, an azaindole group, an azabenzoborole group, an azabenzophosphole group, an azaindene group, an azabenzosilole group, an azabenzogermole group, an azabenzothiophene group, an azabenzoselenophene group, an azabenzofuran group, an azacarbazole group, an azadibenzoborole group, an azadibenzophosphole group, an azafluorene group, an azadibenzosilole group, an azadibenzogermole group, an azadibenzothiophene group, an azadibenzoselenophene group, an azadibenzofuran group, an azadibenzothiophene 5-oxide group, an aza-9H-fluorene-9-one group, an azadibenzothiophene 5,5-dioxide group, an indolocarbazole group, a pyridine group, a pyrimidine group, a pyrazine group, a pyridazine group, a triazine group, a quinoline group, an isoquinoline group, a quinoxaline group, a quinazoline group, a phenanthroline group, a pyrrole group, a pyrazole group, an imidazole group, a triazole group, an oxazole group, an isooxazole group, a thiazole group, an isothiazole group, an oxadiazole group, a thiadiazole group, a benzopyrazole group, a benzimidazole group, a benzoxazole group, a benzothiazole group, a benzoxadiazole group, a benzothiadiazole group, a 5,6,7,8-tetrahydroisoquinoline group, or a 5,6,7,8-tetrahydroquinoline group.

In an embodiment, $A_1$ may be a benzene group.

$Z_1$ is a group represented by Formula 2.

Formula 2

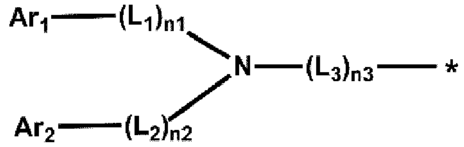

,

a1 is an integer from 1 to 4.

In one or more embodiments, a1 may be 1.

In an embodiment, the condensed cyclic compound represented by Formula 1 may be represented by one of Formulae 1-1 to 1-4:

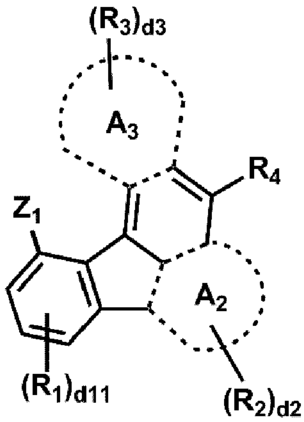

1-1

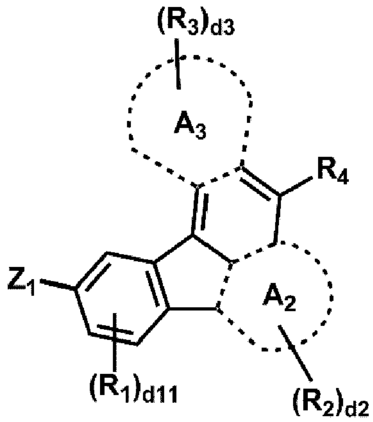

1-2

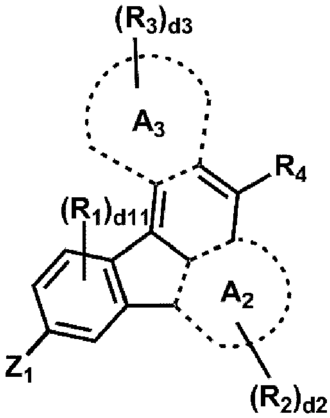

1-3

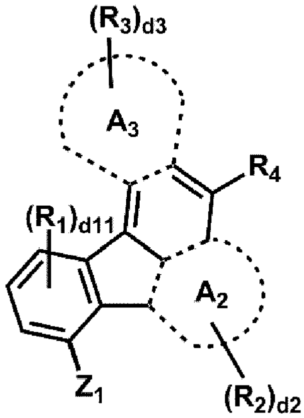

1-4

,

wherein, in Formulae 1-1 to 1-4,

$Z_1$, $A_2$, $A_3$, $R_1$ to $R_4$, d2, and d3 may each independently be the same as described herein, and d11 may be an integer from 0 to 3.

In Formula 2,

$L_1$ to $L_3$ are each independently be a single bond, a $C_5$-$C_{60}$ carbocyclic group unsubstituted or substituted with at least one $R_{10a}$, or a $C_1$-$C_{60}$ heterocyclic group unsubstituted or substituted with at least one $R_{10a}$.

In an embodiment, $L_1$ to $L_3$ may each independently be:

a single bond; or

a $\pi$ electron-rich $C_3$-$C_{60}$ cyclic group unsubstituted or substituted with at least one $R_{10a}$, and $R_{10a}$ may be the same as described herein.

In one or more embodiments, $L_1$ to $L_3$ may each independently be:

a single bond; or

a group represented by one of Formulae 3-1 to 3-40:

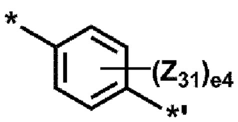

3-1

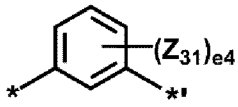

3-2

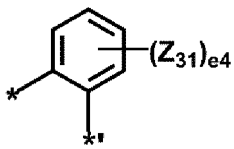

3-3

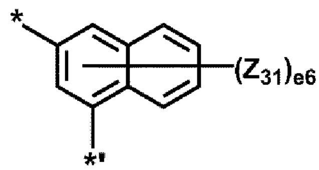

3-4

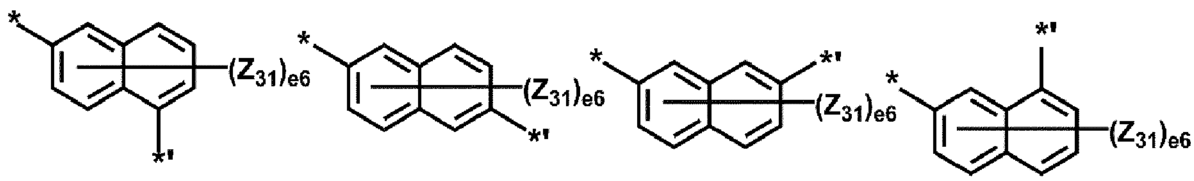

3-5 3-6 3-7 3-8

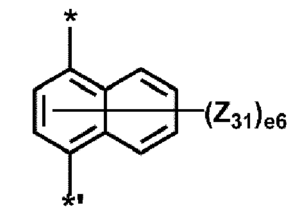
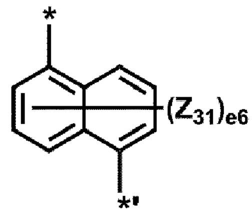
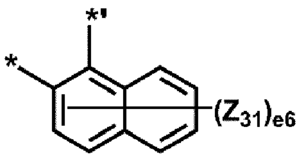
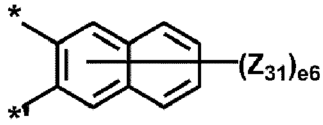

3-9 3-10 3-11 3-12

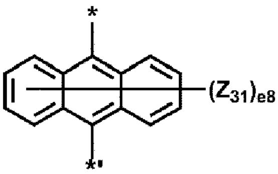
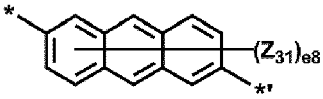
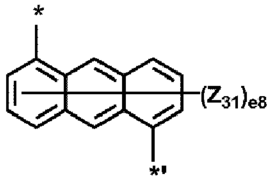

3-13 3-14 3-15

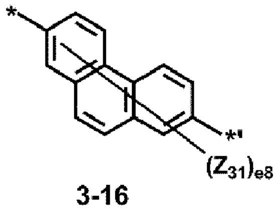
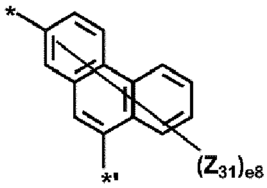
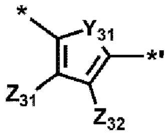
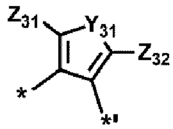

3-16 3-17 3-18 3-19

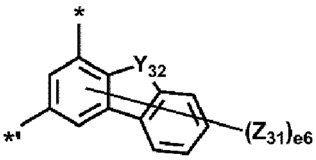
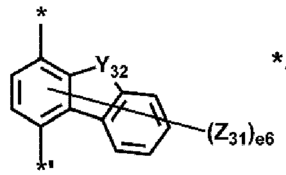
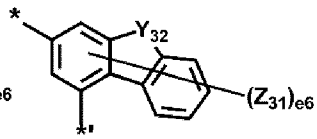
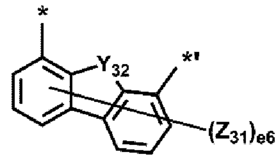

3-20 3-21 3-22 3-23

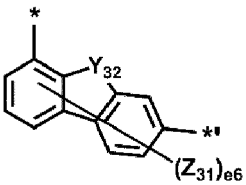
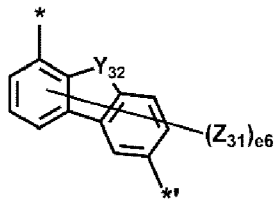
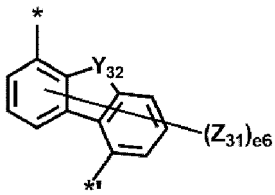
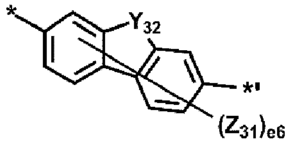

3-24 3-25 3-26 3-27

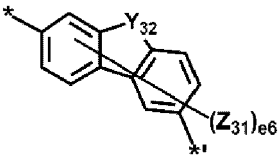
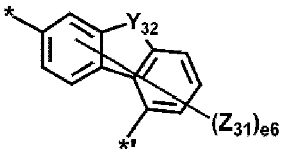
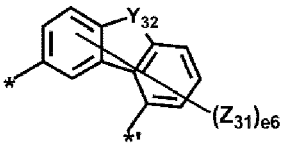
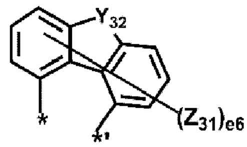

3-28 3-29 3-30 3-31

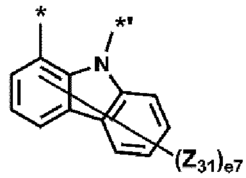

3-32

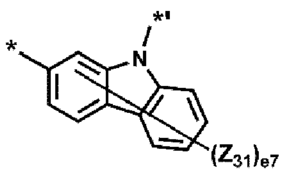

3-33

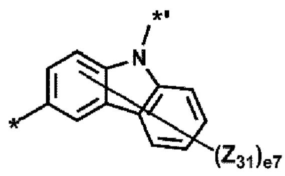

3-34

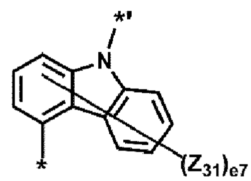

3-35

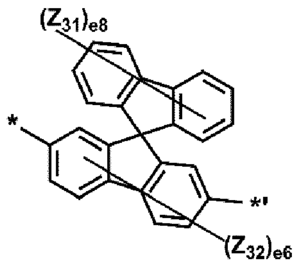

3-36

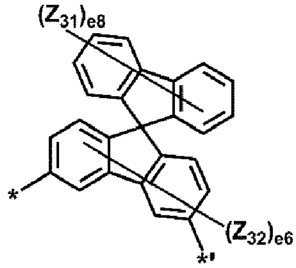

3-37

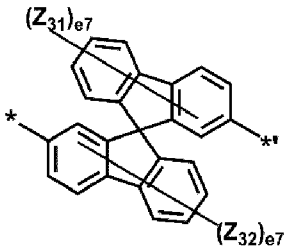

3-38

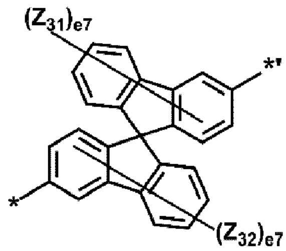

3-39

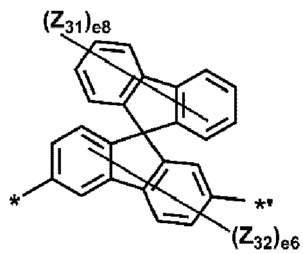

3-40

In Formulae 3-1 to 3-40,

$Y_{31}$ may be O or S,

Y32 may be O, S, N(Z33), C(Z33)(Z34), or Si(Z33)(Z34),

$Z_{31}$ to $Z_{34}$ may each independently be hydrogen, deuterium, -F, -Cl, -Br, -I, a hydroxyl group, a cyano group, a nitro group, an amidino group, a hydrazino group, a hydrazono group, -CF3, -CF2H, -CFH2, a $C_1$-$C_{20}$ alkyl group, a $C_1$-$C_{20}$ alkoxy group, a cyclopentyl group, a cyclohexyl group, a cycloheptyl group, a cyclopentenyl group, a cyclohexenyl group, a phenyl group, a biphenyl group, a naphthyl group, a fluorenyl group, a spiro-bifluorenyl group, a spiro-fluorene-benzofluorenyl group, a benzofluorenyl group, a dibenzofluorenyl group, a phenalenyl group, a phenanthrenyl group, an anthracenyl group, a fluoranthenyl group, a triphenylenyl group, a pyridinyl group, a pyrimidinyl group, a pyrazinyl group, a triazinyl group, a quinolinyl group, an isoquinolinyl group, a benzoquinolinyl group, a naphthyridinyl group, a quinoxalinyl group, a quinazolinyl group, a phenanthridinyl group, an acridinyl group, a phenanthrolinyl group, a phenazinyl group, a carbazolyl group, a dibenzofuranyl group, a dibenzothiophenyl group, a dibenzosilolyl group, -Si($Q_{31}$)($Q_{32}$)($Q_{33}$), -N($Q_{31}$)($Q_{32}$), or - B($Q_{31}$)($Q_{32}$),

e4 may be an integer from 0 to 4,

e6 may be an integer from 0 to 6,

e7 may be an integer from 0 to 7,

e8 may be an integer from 0 to 8,

$Q_{31}$ to $Q_{33}$ may each independently be a $C_1$-$C_{10}$ alkyl group, a $C_1$-$C_{10}$ alkoxy group, a phenyl group, a biphenyl group, a terphenyl group, or a naphthyl group, and

* and *' each indicate a binding site to a neighboring atom.

n1 to n3 are each independently an integer from 1 to 3.

In an embodiment, $L_3$ may be a single bond, and n3 may be 1.

In an embodiment, at least one of $L_1$ and $L_2$ may not be a single bond (e.g., may be any of the above-described groups besides a single bond).

$Ar_1$ and $Ar_2$ are each independently a $C_5$-$C_{60}$ carbocyclic group unsubstituted or substituted with at least one

$R_{10a}$ or a $C_1$-$C_{60}$ heterocyclic group unsubstituted or substituted with at least one Rioa.

In one or more embodiments, $Ar_1$ and $Ar_2$ may each independently be a π electron-rich $C_3$-$C_{60}$ cyclic group unsubstituted or substituted with at least one $R_{10a}$, and Rioa may be the same as described herein.

In an embodiment, $Ar_1$ and $Ar_2$ may each independently be a $C_3$-$C_{60}$ carbocyclic group, a 1H-pyrrole group, a silole group, a borole group, a 2H-pyrrole group, a 3H-pyrrole group, a thiophene group, a furan group, an indole group, a benzoindole group, a naphthoindole group, an isoindole group, a benzoisoindole group, a naphthoisoindole group, a benzosilole group, a benzothiophene group, a benzofuran group, a carbazole group, a dibenzosilole group, a dibenzothiophene group, a dibenzofuran group, an indenocarbazole group, an indolocarbazole group, a benzofurocarbazole group, a benzothienocarbazole group, a benzosilolocarbazole group, a benzoindolocarbazole group, a benzocarbazole group, a benzonaphthofuran group, a benzonaphthothiophene group, a benzonaphthosilole group, a benzofurodibenzofuran group, a benzofurodibenzothiophene group, or a benzothienodibenzothiophene group, each unsubstituted or substituted with at least one $R_{10a}$, and $R_{10a}$ may be the same as described herein.

In one or more embodiments, at least one of $Ar_1$ and $Ar_2$ may be a carbazole group, a dibenzosilole group, a dibenzothiophene group, or a dibenzofuran group, each unsubstituted or substituted with at least one Rioa.

In one or more embodiments, $Ar_1$ and $Ar_2$ may each independently be a group represented by one of Formulae 4-1 to 4-36:

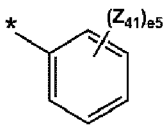
4-1
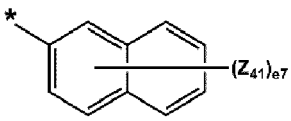
4-2
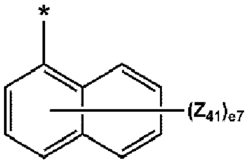
4-3
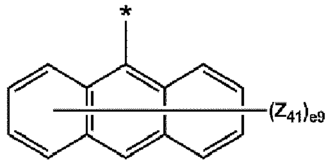
4-4

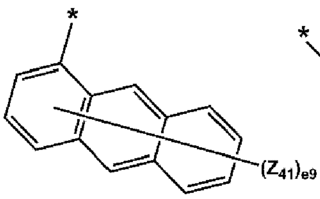
4-5
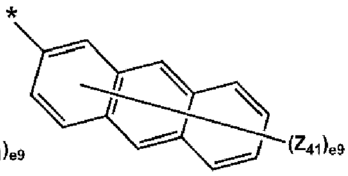
4-6
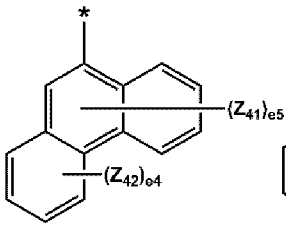
4-7
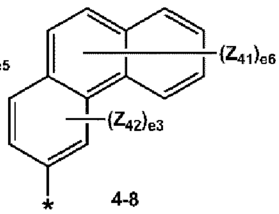
4-8

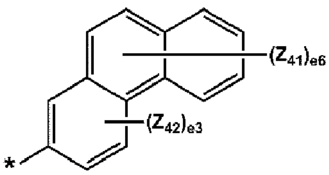
4-9
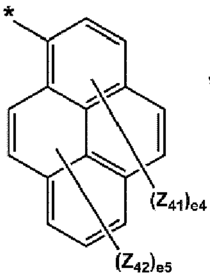
4-10
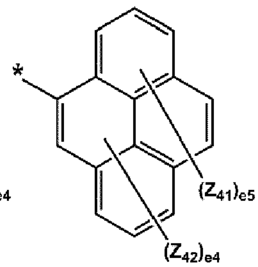
4-11
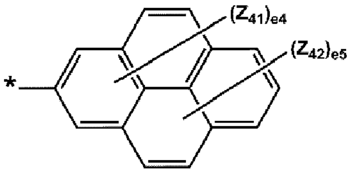
4-12

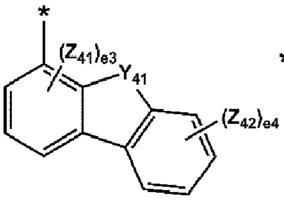
4-13
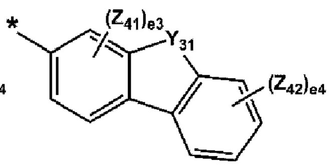
4-14
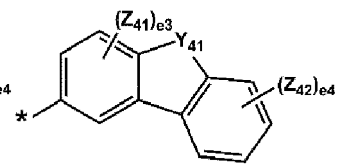
4-15
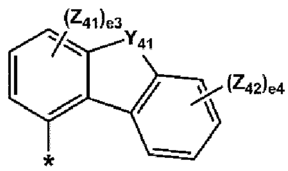
4-16

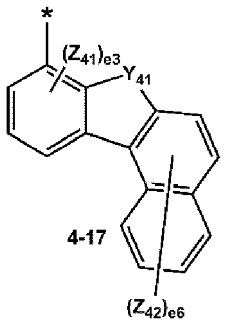
4-17

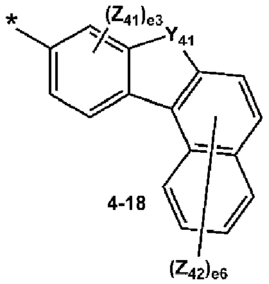
4-18

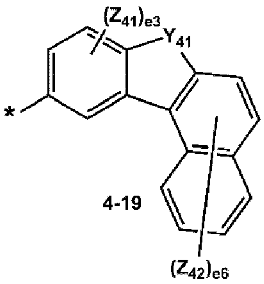
4-19

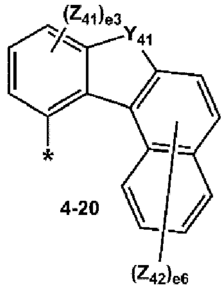
4-20

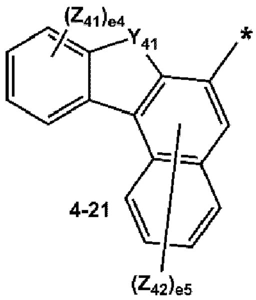
4-21

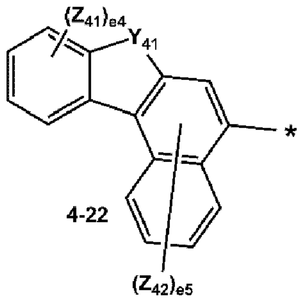
4-22

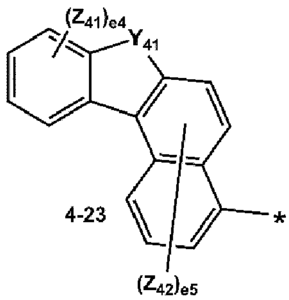
4-23

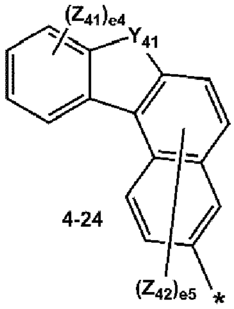
4-24

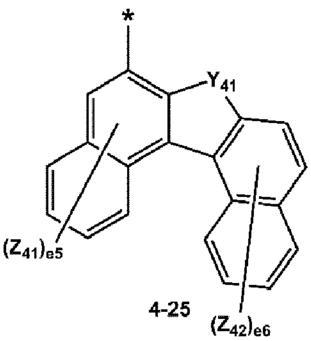
4-25

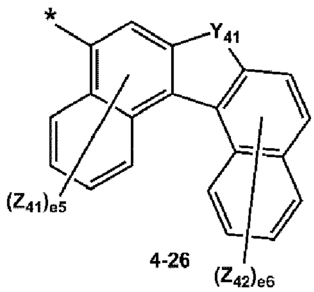
4-26

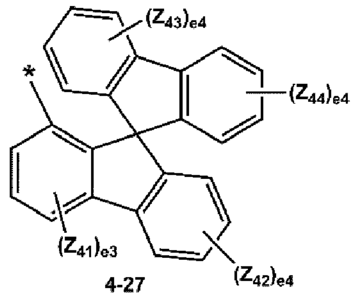
4-27

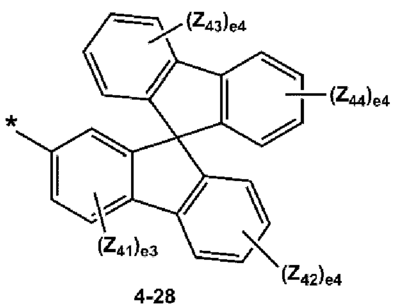
4-28

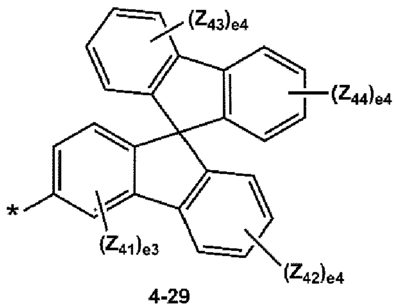
4-29

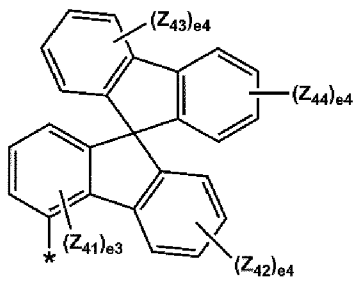
4-30

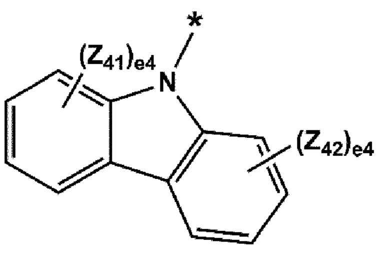
4-31

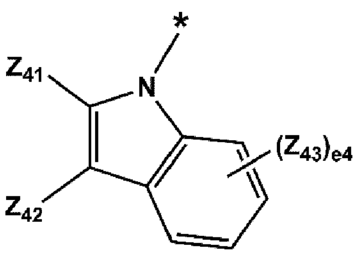
4-32

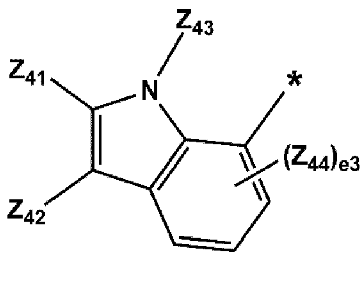
4-33

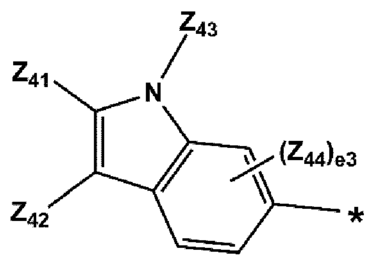

4-34

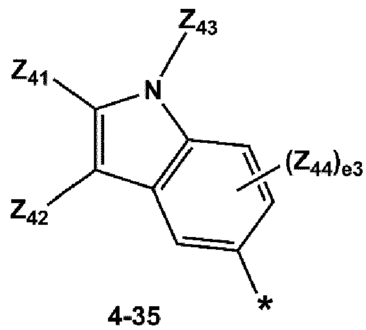

4-35

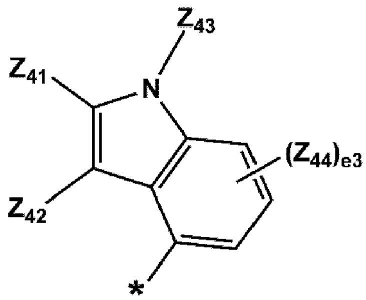

4-36

In Formulae 4-1 to 4-36,

Y41 may be O, S, N(Z45), C(Z45)(Z46), or Si(Z45)(Z46),

$Z_{41}$ to $Z_{46}$ may each independently be hydrogen, deuterium, -F, -Cl, -Br, -I, a hydroxyl group, a cyano group, a nitro group, an amidino group, a hydrazino group, a hydrazono group, -CF3, -CF2H, -CFH2, a $C_1$-$C_{20}$ alkyl group, a $C_1$-$C_{20}$ alkoxy group, a cyclopentyl group, a cyclohexyl group, a cycloheptyl group, a cyclopentenyl group, a cyclohexenyl group, a phenyl group, a biphenyl group, a naphthyl group, a fluorenyl group, a spiro-bifluorenyl group, a spiro-fluorene-benzofluorenyl group, a benzofluorenyl group, a dibenzofluorenyl group, a phenalenyl group, a phenanthrenyl group, an anthracenyl group, a fluoranthenyl group, a triphenylenyl group, a pyridinyl group, a pyrimidinyl group, a pyrazinyl group, a triazinyl group, a quinolinyl group, an isoquinolinyl group, a benzoquinolinyl group, a naphthyridinyl group, a quinoxalinyl group, a quinazolinyl group, a phenanthridinyl group, an acridinyl group, a phenanthrolinyl group, a phenazinyl group, a carbazolyl group, a dibenzofuranyl group, a dibenzothiophenyl group, a dibenzosilolyl group, -Si($Q_{31}$)($Q_{32}$)($Q_{33}$), -N($Q_{31}$)($Q_{32}$), or - B($Q_{31}$)($Q_{32}$),

e2 may be an integer from 0 to 2,

e3 may be an integer from 0 to 3,

e4 may be an integer from 0 to 4,

e5 may be an integer from 0 to 5,

e6 may be an integer from 0 to 6,

e7 may be an integer from 0 to 7,

e9 may be an integer from 0 to 9,

$Q_{31}$ to $Q_{33}$ may each independently be a $C_1$-$C_{10}$ alkyl group, a $C_1$-$C_{10}$ alkoxy group, a phenyl group, a biphenyl group, a terphenyl group, or a naphthyl group, and

* indicates a binding site to a neighboring atom.

In one or more embodiments, at least one selected from $Ar_1$ and $Ar_2$ may be a group represented by one of Formulae 4-13 to 4-26 and 4-31.

In one or more embodiments, $Ar_1$ and $Ar_2$ in Formula 2 may be different from each other.

In an embodiment, in Formula 2, *-$(L_1)_{n1}$-$Ar_1$ and *-$(L_2)_{n2}$-$Ar_2$ may be different from each other, and * indicates a binding site to N in Formula 2.

$R_1$ to $R_4$ are each independently hydrogen, deuterium, -F, -Cl, -Br, -I, a hydroxyl group, a cyano group, a nitro group, a $C_1$-$C_{60}$ alkyl group unsubstituted or substituted with at least one $R_{10a}$, a $C_2$-$C_{60}$ alkenyl group unsubstituted or substituted with at least one $R_{10a}$, a $C_2$-$C_{60}$ alkynyl group unsubstituted or substituted with at least one $R_{10a}$, a $C_1$-$C_{60}$ alkoxy group unsubstituted or substituted with at least one $R_{10a}$, a $C_3$-$C_{60}$ carbocyclic group unsubstituted or substituted with at least one $R_{10a}$, a $C_1$-$C_{60}$ heterocyclic group unsubstituted or substituted with at least one $R_{10a}$, a $C_6$-$C_{60}$ aryloxy group unsubstituted or substituted with at least one $R_{10a}$, a $C_6$-$C_{60}$ arylthio group unsubstituted or substituted with at least one $R_{10a}$, -Si($Q_1$)($Q_2$)($Q_3$), -B($Q_1$)($Q_2$), - C(=O)($Q_1$), -S$(=O)_2$($O_1$), or -P(=O)($Q_1$)($Q_2$).

In one or more embodiments, $R_1$ to $R_4$ may each independently be: hydrogen, deuterium, -F, -Cl, -Br, -I, a hydroxyl group, a cyano group, a nitro group, an amidino group, a hydrazine group, a hydrazone group, a $C_1$-$C_{20}$ alkyl group, or a $C_1$-C20 alkoxy group;

a $C_1$-$C_{20}$ alkyl group or a $C_1$-$C_{20}$ alkoxy group, each substituted with deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amidino group, a hydrazine group, a hydrazone group, a $C_1$-$C_{10}$ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a biphenyl group, a naphthyl group, a pyridinyl group, a pyrimidinyl group, or any combination thereof;

a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a biphenyl group, a $C_1$-$C_{10}$ alkylphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzoimidazolyl group, a benzofuranyl group, a benzothiophenyl group, a benzoisothiazolyl group, a benzoxazolyl group, an benzoisoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, an azacarbazolyl group, an azadibenzofuranyl group, an azadibenzothiophenyl group, an azafluorenyl group, or an azadibenzosilolyl group, each unsubstituted or substituted with deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amidino group, a hydrazine group, a hydrazone group, a $C_1$-$C_{20}$ alkyl group, a $C_1$-$C_{20}$ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a biphenyl group, a $C_1$-$C_{10}$ alkylphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzoimidazolyl group, a benzofuranyl group, a benzothiophenyl group, a benzoisothiazolyl group, a benzoxazolyl group, an benzoisoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, an azacarbazolyl group, an azadibenzofuranyl group, an azadibenzothiophenyl group, an azafluorenyl group, an azadibenzosilolyl group, $-Si(Q_{31})(Q_{32})(Q_{33})$, $-B(Q_{31})(Q_{32})$, $-P(Q_{31})(Q_{32})$, $-C(=O)(Q_{31})$, $-S(=O)_2(Q_{31})$, $-P(=O)(Q_{31})(Q_{32})$, or any combination thereof; or
$-Si(Q_1)(Q_2)(Q_3)$, $-B(Q_1)(Q_2)$, $-C(=O)(Q_1)$, $-S(=O)_2(Q_1)$, or $-P(=O)(Q_1)(Q_2)$, and
$Q_1$ to $Q_3$ and $Q_{31}$ to $Q_{33}$ may each independently be:

$-CH_3$, $-CD_3$, $-CD_2H$, $-CDH_2$, $-CH_2CH_3$, $-CH_2CD_3$, $-CH_2CD_2H$, $-CH_2CDH_2$, - $CHDCH_3$, $-CHDCD_2H$, $-CHDCDH_2$, $-CHDCD_3$, $-CD_2CD_3$, $-CD_2CD_2H$, or $-CD_2CDH_2$; or
an n-propyl group, an isopropyl group, an n-butyl group, an isobutyl group, a sec-butyl group, a tert-butyl group, an n-pentyl group, an isopentyl group, a sec-pentyl group, a tert-pentyl group, a phenyl group, a naphthyl group, a pyridinyl group, a pyrimidinyl group, a pyridazinyl group, a pyrazinyl group, a triazinyl group, a carbazolyl group, a dibenzofuranyl group, or a dibenzothiophenyl group, each unsubstituted or substituted with deuterium, a $C_1$-$C_{10}$ alkyl group, a phenyl group, a biphenyl group, a pyridinyl group, a pyrimidinyl group, pyridazinyl group, a pyrazinyl group, a triazinyl group, a carbazolyl group, a dibenzofuranyl group, a dibenzothiophenyl group, or any combination thereof.

In one or more embodiments, $R_1$ to $R_4$ may each independently be:

hydrogen, deuterium, a $C_1$-$C_{20}$ alkyl group, or a $C_1$-$C_{20}$ alkoxy group;
a $C_1$-$C_{20}$ alkyl group or a $C_1$-$C_{20}$ alkoxy group, each substituted with deuterium, $-CD_3$, $-CD_2H$, $-CDH_2$, $C_1$-$C_{10}$ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a biphenyl group, a naphthyl group, or any combination thereof;
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a biphenyl group, a $C_1$-$C_{10}$ alkylphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an isoindolyl group, an indolyl group, an indazolyl group, a purinyl group, a carbazolyl group, a benzofuranyl group, a benzothiophenyl group, a dibenzofuranyl group, a dibenzothiophenyl group, a benzocarbazolyl group, or a dibenzocarbazolyl group, each unsubstituted or substituted with deuterium, $-CD_3$, $-CD_2H$, $-CDH_2$, a $C_1$-$C_{20}$ alkyl group, a $C_1$-$C_{20}$ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a biphenyl group, a $C_1$-$C_{10}$ alkylphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an isoindolyl group, an indolyl group, an indazolyl group, a purinyl group, a carbazolyl group, a benzofuranyl group, a benzothiophenyl group, a dibenzofuranyl group, a dibenzothiophenyl group, a benzocarbazolyl group, a dibenzocarbazolyl group, -Si($Q_{31}$)($Q_{32}$)($Q_{33}$), -B($Q_{31}$)($Q_{32}$), or any combination thereof; or

-Si($Q_1$)($Q_2$)($Q_3$) or -B($Q_1$)($Q_2$), and

$Q_1$ to $Q_3$ and $Q_{31}$ to $Q_{33}$ may each independently be:

$-CH_3$, $-CD_3$, $-CD_2H$, $-CDH_2$, $-CH_2CH_3$, $-CH_2CD_3$, $-CH_2CD_2H$, $-CH_2CDH_2$, - $CHDCH_3$, $-CHDCD_2H$, $-CHDCDH_2$, $-CHDCD_3$, $-CD_2CD_3$, $-CD_2CD_2H$, or $-CD_2CDH_2$; or

an n-propyl group, an iso-propyl group, an n-butyl group, an isobutyl group, a sec-butyl group, a tert-butyl group, an n-pentyl group, an isopentyl group, a sec-pentyl group, a tert-pentyl group, a phenyl group, a naphthyl group a carbazolyl group, a dibenzofuranyl group, or a dibenzothiophenyl group, each unsubstituted or substituted with deuterium, a $C_1$-$C_{10}$ alkyl group, a phenyl group, a biphenyl group, a carbazolyl group, a dibenzofuranyl group, a dibenzothiophenyl group, or any combination thereof.

d1 to d3 are each independently an integer from 0 to 10.

In an embodiment, * and *' in Formula 2 each indicate a binding site to a neighboring atom.

each $R_{10a}$ as utilized (referenced) herein is indivdually:

deuterium, -F, -Cl, -Br, -I, a hydroxyl group, a cyano group, or a nitro group;

a $C_1$-$C_{60}$ alkyl group, a $C_2$-$C_{60}$ alkenyl group, a $C_2$-$C_{60}$ alkynyl group, or a $C_1$-$C_{60}$ alkoxy group, each unsubstituted or substituted with deuterium, -F, -Cl, -Br, -I, a hydroxyl group, a cyano group, a nitro group, a $C_3$-$C_{60}$ carbocyclic group, a $C_1$-$C_{60}$ heterocyclic group, a $C_6$-$C_{60}$ aryloxy group, a $C_6$-$C_{60}$ arylthio group, -Si($Q_{11}$)($Q_{12}$)($Q_{13}$), -N($Q_{11}$)($Q_{12}$), -B($Q_{11}$)($Q_{12}$), -C(=O)($Q_{11}$), $-S(=O)_2$($Q_{11}$), -P(=O)($Q_{11}$)($Q_{12}$), or any combination thereof;

a $C_3$-$C_{60}$ carbocyclic group, a $C_1$-$C_{60}$ heterocyclic group, a $C_6$-$C_{60}$ aryloxy group, or a $C_6$-$C_{60}$ arylthio group, each unsubstituted or substituted with deuterium, -F, -Cl, -Br, -I, a hydroxyl group, a cyano group, a nitro group, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{60}$ carbocyclic group, a $C_1$-$C_{60}$ heterocyclic group, a $C_6$-$C_{60}$ aryloxy group, a $C_6$-$C_{60}$ arylthio group, - Si($Q_{21}$)($Q_{22}$)($Q_{23}$), -N($Q_{21}$)($Q_{22}$), -B($Q_{21}$)($Q_{22}$), -C(=O)($Q_{21}$), $-S(=O)_2$($Q_{21}$), - P(=O)($Q_{21}$)($Q_{22}$), or any combination thereof; or

-Si($Q_{31}$)($Q_{32}$)($Q_{33}$), -N($Q_{31}$)($Q_{32}$), -B($Q_{31}$)($Q_{32}$), -C(=O)($Q_{31}$), $-S(=O)_2$($Q_{31}$), or - P(=O)($Q_{31}$)($Q_{32}$), and

$Q_1$ to $Q_3$, $Q_{11}$ to $Q_{13}$, $Q_{21}$ to $Q_{23}$, and $Q_{31}$ to $Q_{33}$ may each independently be: hydrogen; deuterium; -F; -Cl; -Br; -I; a hydroxyl group; a cyano group; a nitro group; $C_1$-$C_{60}$ alkyl group; $C_2$-$C_{60}$ alkenyl group; $C_2$-$C_{60}$ alkynyl group; $C_1$-$C_{60}$ alkoxy group; or a C3-C60 carbocyclic group or a $C_1$-$C_{60}$ heterocyclic group, each unsubstituted or substituted with deuterium, -F, a cyano group, a $C_1$-$C_{60}$ alkyl group, a $C_1$-$C_{60}$ alkoxy group, a phenyl group, a biphenyl group, or any combination thereof.

In an embodiment, the condensed cyclic compound represented by Formula 1 may be one of Compounds 1 to 192, but is not limited thereto:

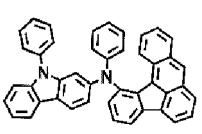
1

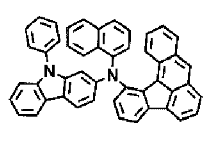
2

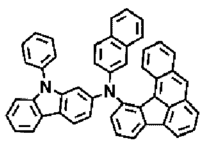
3

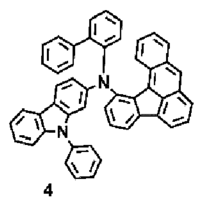
4

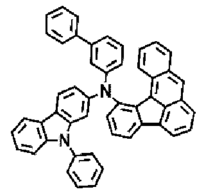
5

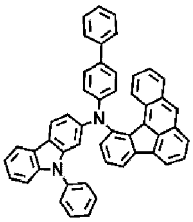
6

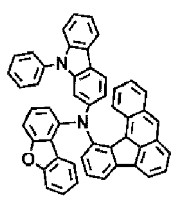
7

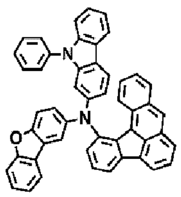
8

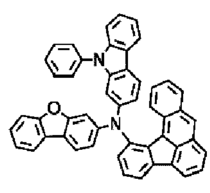
9

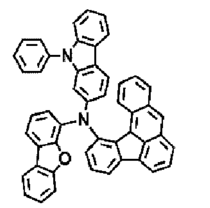
10

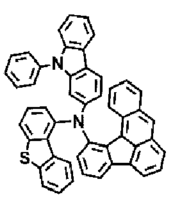 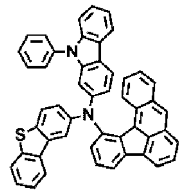 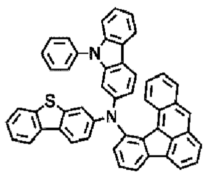 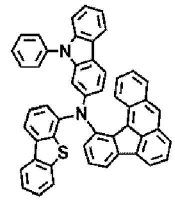 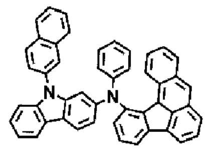

11 12 13 14 15

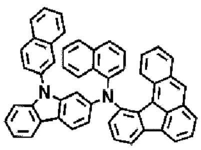 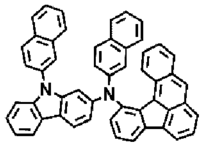 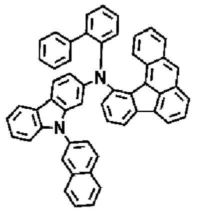 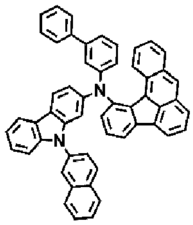 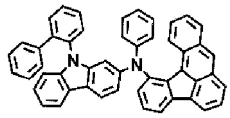

16 17 18 19 20

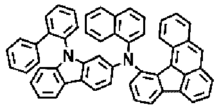 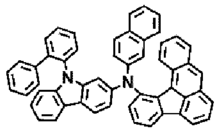 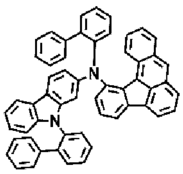 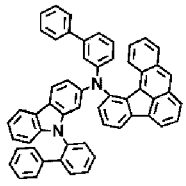 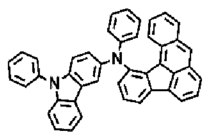

21 22 23 24 25

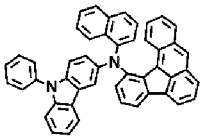 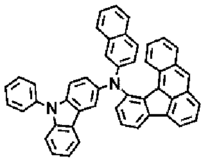 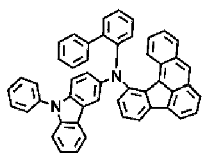 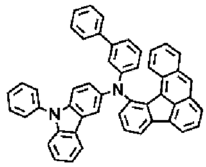 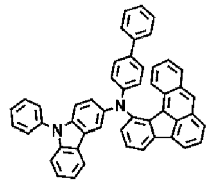

26 27 28 29 30

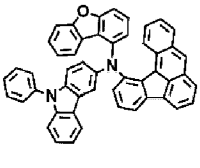 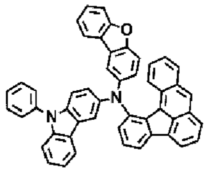 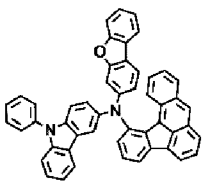 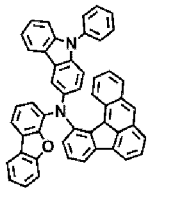 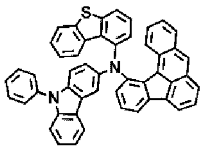

31 32 33 34 35

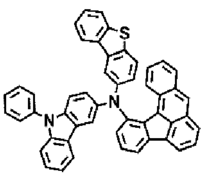 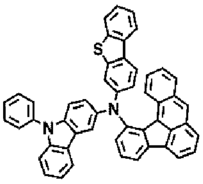 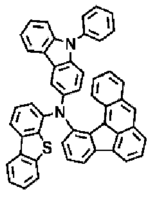 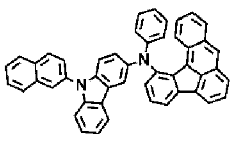 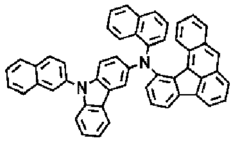

36 37 38 39 40

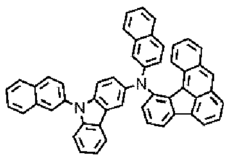
41

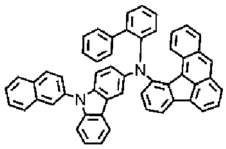
42

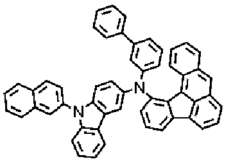
43

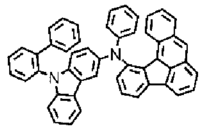
44

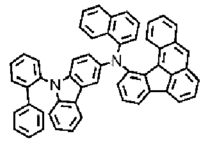
45

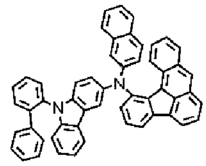
46

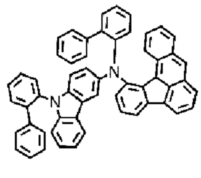
47

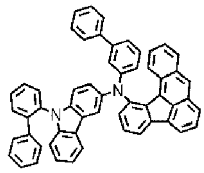
48

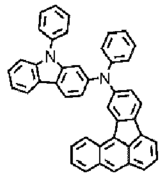
49

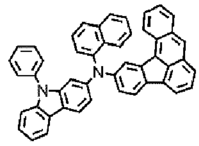
50

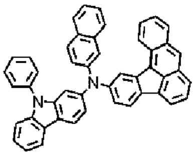
51

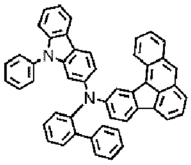
52

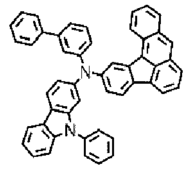
53

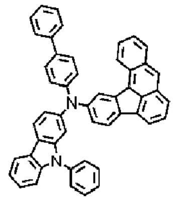
54

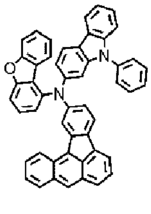
55

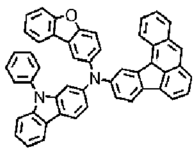
56

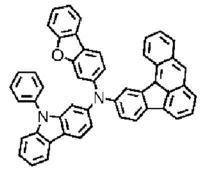
57

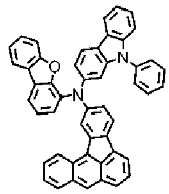
58

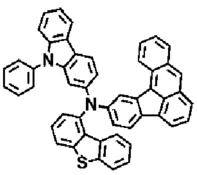
59

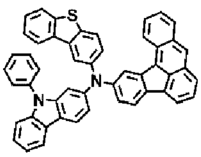
60

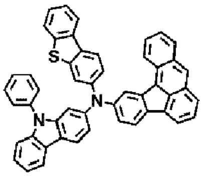
61

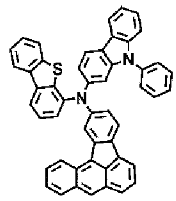
62

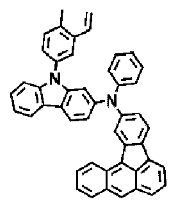
63

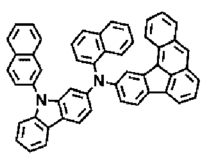
64

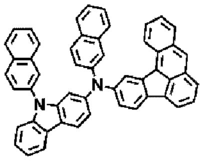
65

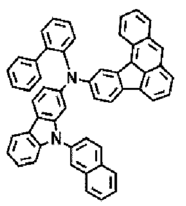
66

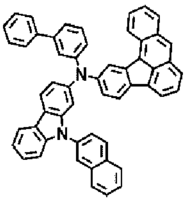
67

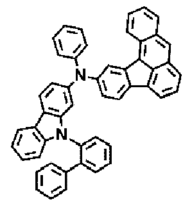
68

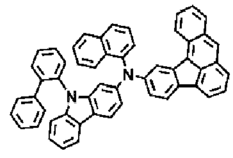
69
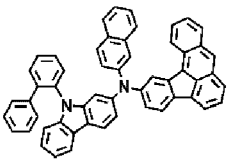
70
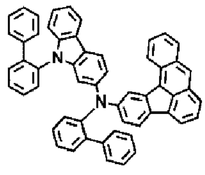
71
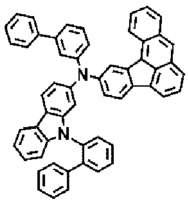
72
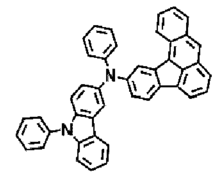
73

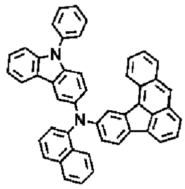
74
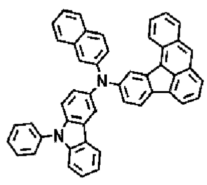
75
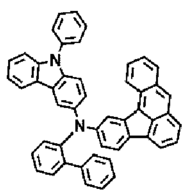
76
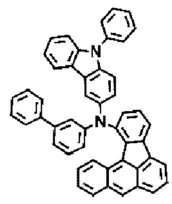
77
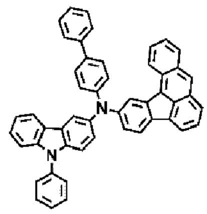
78

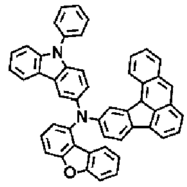
79
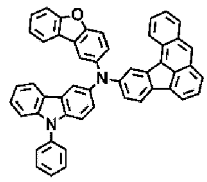
80
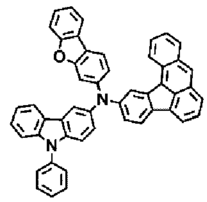
81
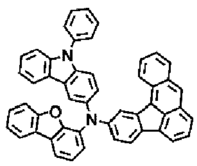
82
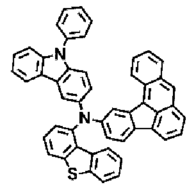
83

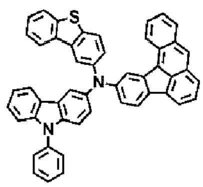
84
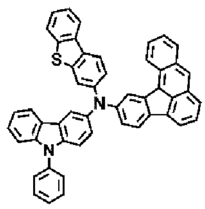
85
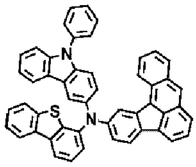
86
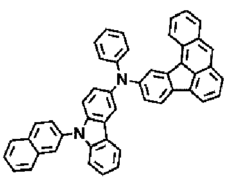
87
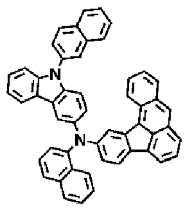
88

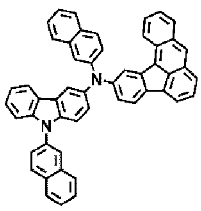
89
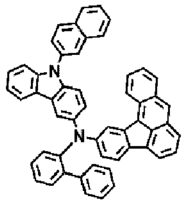
90
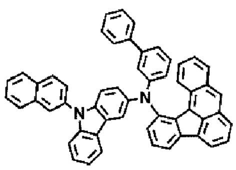
91
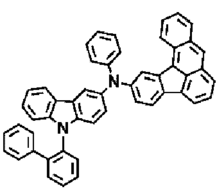
92
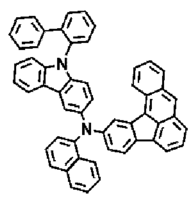
93

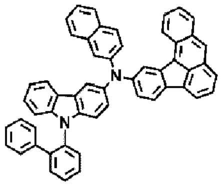
94
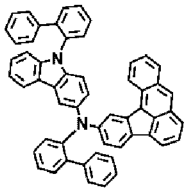
95
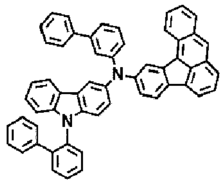
96

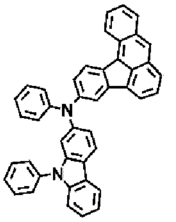
97

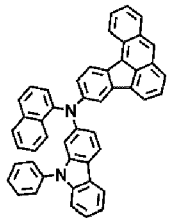
98

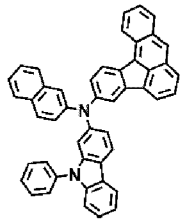
99

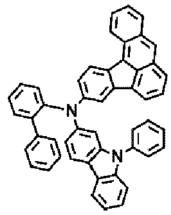
100

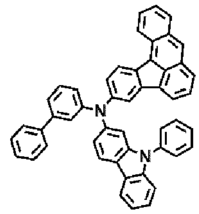
101

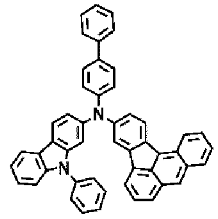
102

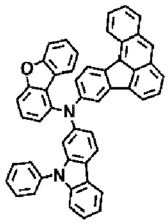
103

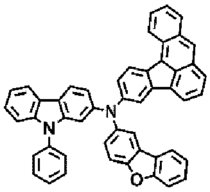
104

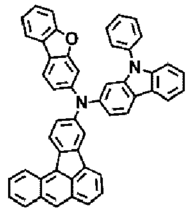
105

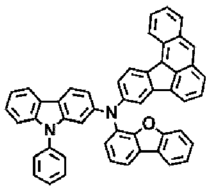
106

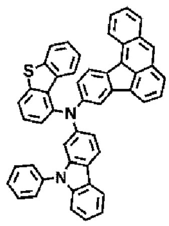
107

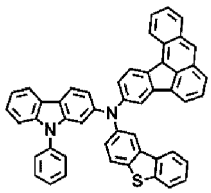
108

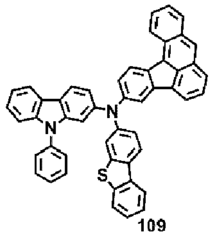
109

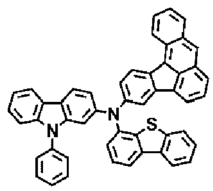
110

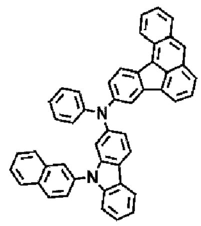
111

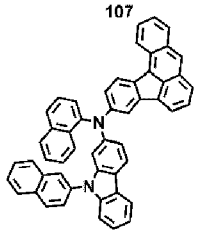
112

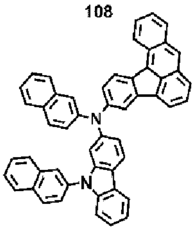
113

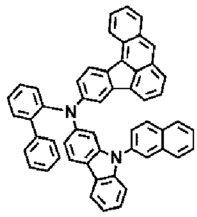
114

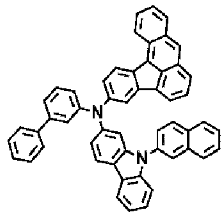
115

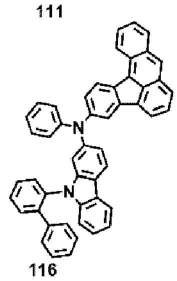
116

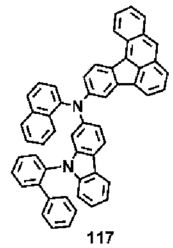
117

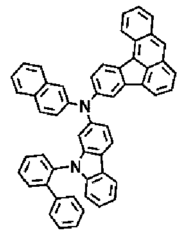
118

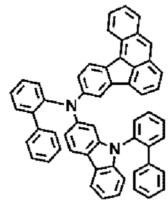
119

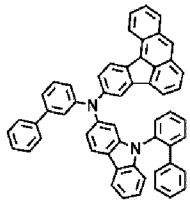
120

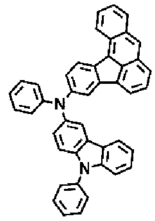
121

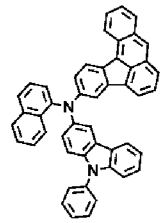
122

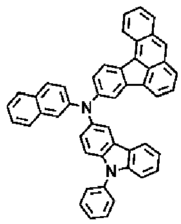
123

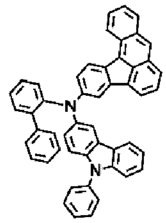
124

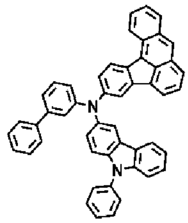
125

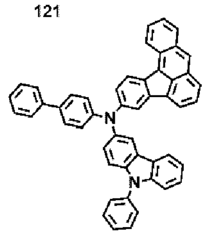
126

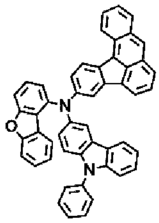
127

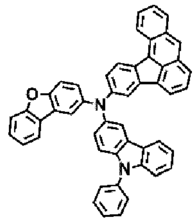
128

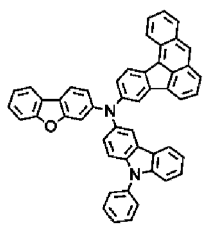
129

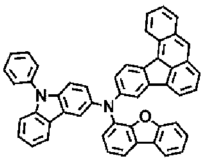
130

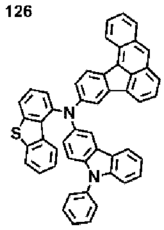
131

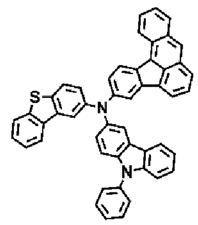
132

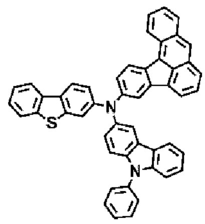
133

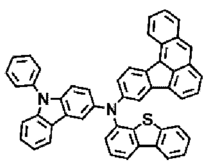
134

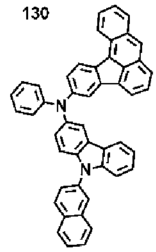
135

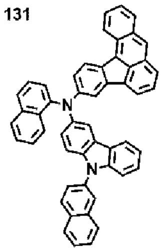
136

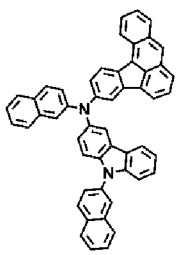
137

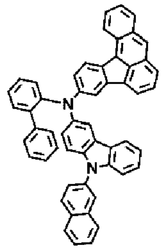
138

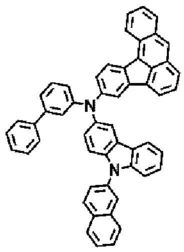
139

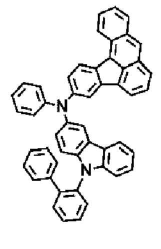
140

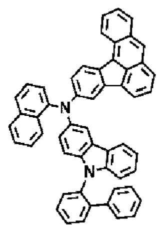
141

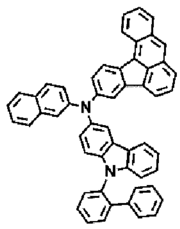
142

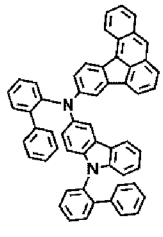
143

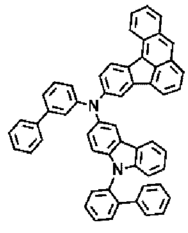
144

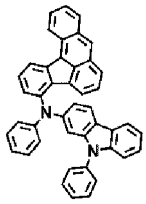
145

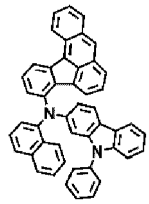
146

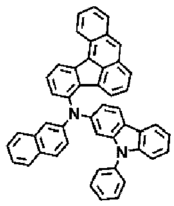
147

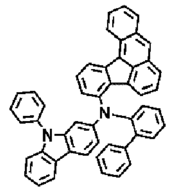
148

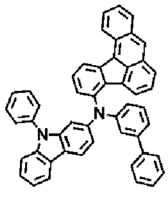
149

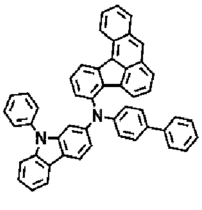
150

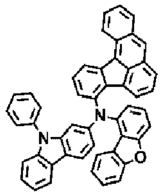
151

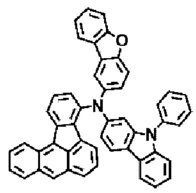
152

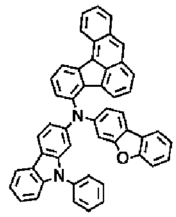
153

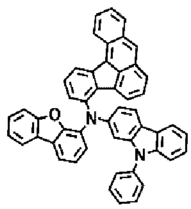
154

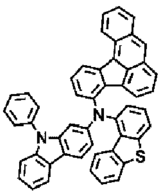
155

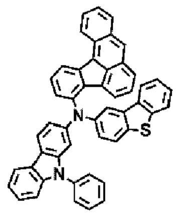
156

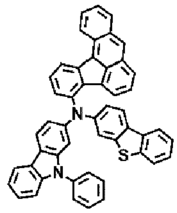
157

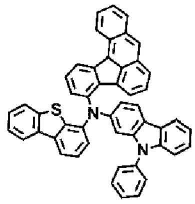
158

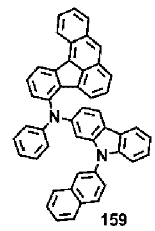
159

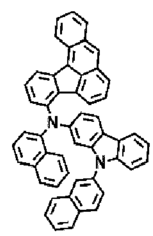
160

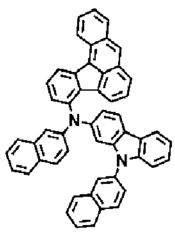
161

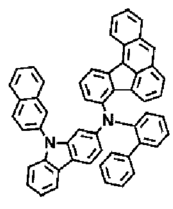
162

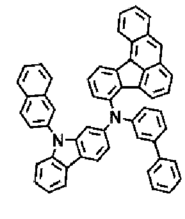
163

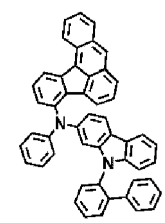
164

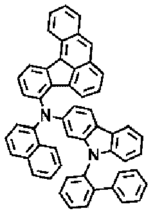
165
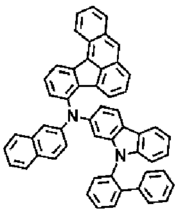
166
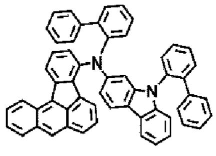
167
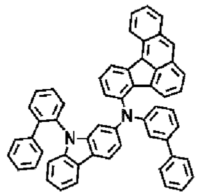
168
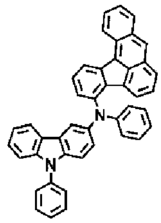
169

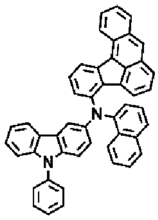
170
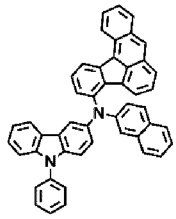
171
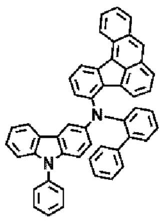
172
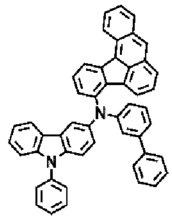
173
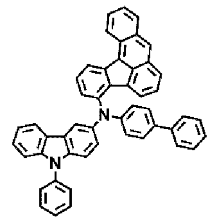
174

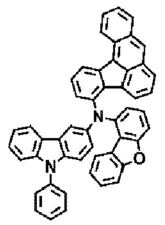
175
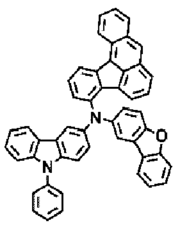
176
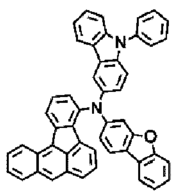
177
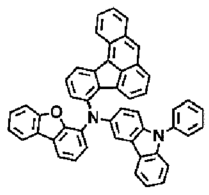
178
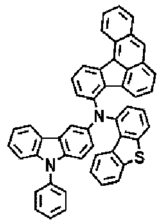
179

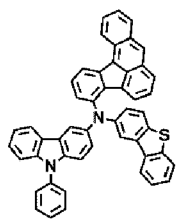
180
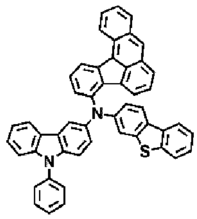
181
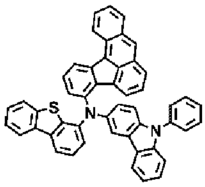
182
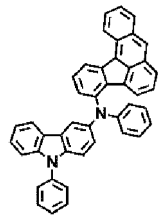
183
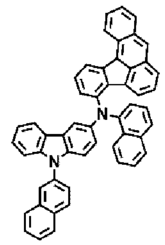
184

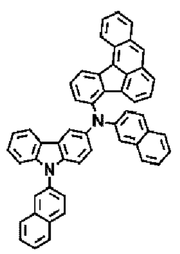
185
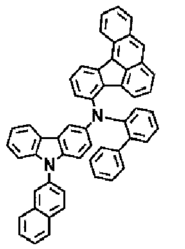
186
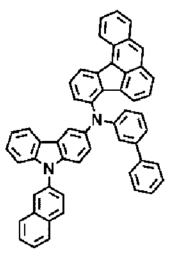
187
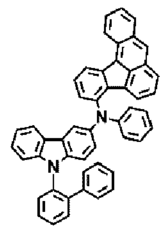
188
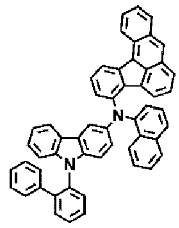
189

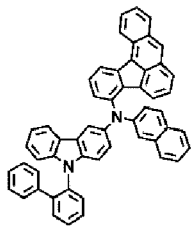

190

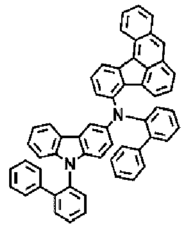

191

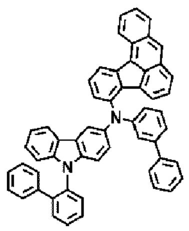

192

The condensed cyclic compound represented by Formula 1 has a structure in which three cyclic groups are condensed with a central cyclic group to form a core, and $A_1$ of the core is substituted with at least one amine group represented by Formula 2.

When the condensed cyclic compound represented by Formula 1 has such a structure including the core in which three cyclic groups are condensed with the central cyclic group, the condensed cyclic compound represented by Formula 1 may have a high glass transition temperature (Tg) and/or a high melting point. Accordingly, when a light-emitting device including the condensed cyclic compound disclosed herein emits light, resistance to a high-temperature environment and/or heat resistance to heat generated inside or between layers of the light-emitting device or between layers and electrodes may be increased. Therefore, such a light-emitting device including the condensed cyclic compound disclosed herein may have high durability during storage and/or driving.

In some embodiments, in the condensed cyclic compound represented by Formula 1, $A_1$ in Formula 1 may be substituted with at least one amine group represented by Formula 2, so that electrical stability and/or charge transport ability may be improved, thereby improving efficiency and/or lifespan of the light-emitting device.

Therefore, an electronic device, for example, a light-emitting device, including the condensed cyclic compound represented by Formula 1 may have a low driving voltage, high luminance, high efficiency, and/or a long lifespan.

Synthesis methods of the condensed cyclic compound represented by Formula 1 may be recognizable by one of ordinary skill in the art by referring to the Examples below.

At least one condensed cyclic compound represented by Formula 1 may be utilized in a light-emitting device (for example, an organic light-emitting device).

One or more embodiments of the present disclosure provide a light-emitting device including: a first electrode; a second electrode facing the first electrode; an interlayer arranged between the first electrode and the second electrode and including an emission layer; and at least one condensed cyclic compound as described herein.

In an embodiment, the first electrode may be an anode,

the second electrode may be a cathode,
the interlayer may further include a hole transport region between the emission layer and the first electrode and an electron transport region between the emission layer and the second electrode,
the hole transport region may include a hole injection layer, a hole transport layer, an emission auxiliary layer, an electron blocking layer, or any combination thereof, and
the electron transport region may include a buffer layer, a hole blocking layer, an electron control layer, an electron transport layer, an electron injection layer, or any combination thereof.

In an embodiment, the condensed cyclic compound may be included in the hole transport region.

In an embodiment, the emission layer may further include a first compound, and

the first compound may be a fluorescent compound or a TADF compound that satisfies Equation 1:

## Equation 1

$$\triangle E_{ST} = S1 - T1 \leq 0.3 \text{ eV},$$

wherein, in Equation 1, S1 is a lowest excitation singlet energy level (eV) of the TADF compound, and T1 is a lowest excitation triplet energy level (eV) of the TADF compound, and
S1 and T1 may be measured in the compound in a solid state from via photoluminescence spectroscopy, or may be measured (e.g., calculated) by utilizing DFT methods (e.g., in a program called Gaussian).

In an embodiment, the emission layer may be configured to emit blue light and/or cyan light.

In an embodiment, the emission layer may be configured to emit light having a maximum emission wavelength in a range of about 400 nm to about 500 nm.

The expression "(the interlayer) includes a condensed cyclic compound represented by Formula 1" as utilized herein may be construed as the meaning that "(the interlayer) includes one condensed cyclic compound belonging to the category of Formula 1" or at least two different condensed cyclic compounds (e.g., two or more structures) belonging to the category of Formula 1".

In an embodiment, the interlayer may include, as the condensed cyclic compound, only Compound 1. In this regard, Compound 1 may be included in the interlayer of the light-emitting device. In one or more embodiments, the interlayer may include, as the condensed cyclic compound (e.g., compounds), Compound 1 and Compound 2. In this regard, Compound 1 and Compound 2 may exist in an identical layer (for example, Compound 1 and Compound 2 may all exist in an electron transport layer), or different layer(s) (for example, Compound 1 may exist in an electron transport layer and Compound 2 may exist in a buffer layer).

The term "interlayer" as utilized herein may refer to a single layer and/or all of a plurality of layers between the first electrode and the second electrode of the light-emitting device.

One or more embodiments of the present disclosure provide an electronic apparatus including the light-emitting device.

In an embodiment, the electronic apparatus may further include a thin-film transistor. For example, the electronic apparatus may further include a thin-film transistor including a source electrode and a drain electrode, wherein the first electrode of the light-emitting device may be electrically connected to the source electrode or the drain electrode.

In one or more embodiments, the electronic apparatus may further include a color filter, a color conversion layer, a touch screen layer, a polarizing layer, or any combination thereof. For example, the electronic apparatus may be a flat panel display apparatus, but embodiments of the present disclosure are not limited thereto.

More details for the electronic apparatus are as described herein.

**Description of FIG. 1**

FIG. 1 is a schematic cross-sectional view of a light-emitting device 10 according to an embodiment. The light-emitting device 10 includes a first electrode 110, an interlayer 130, and a second electrode 150.

Hereinafter, the structure of the light-emitting device 10 according to an embodiment and a method of manufacturing the light-emitting device 10 will be described with reference to FIG. 1.

**First electrode 110**

In FIG. 1, a substrate may be additionally arranged under the first electrode 110 and/or above the second electrode 150. In an embodiment, the substrate may be a glass substrate and/or a plastic substrate. In one or more embodiments, the substrate may be a flexible substrate, and for example, may include plastics with excellent or suitable heat resistance and/or durability (such as polyimide, polyethylene terephthalate (PET), polycarbonate, polyethylene naphthalate, polyarylate (PAR), polyetherimide, or any combination thereof).

The first electrode 110 may be formed by, for example, depositing and/or sputtering a material for forming the first electrode 110 on the substrate. When the first electrode 110 is an anode, a material for forming the first electrode 110 may be a high-work function material that facilitates injection of holes.

The first electrode 110 may be a reflective electrode, a semi-transmissive electrode, or a transmissive electrode. In an embodiment, when the first electrode 110 is a transmissive electrode, a material for forming the first electrode 110 may include indium tin oxide (ITO), indium zinc oxide (IZO), tin oxide ($SnO_2$), zinc oxide (ZnO), or any combination thereof. In one or more embodiments, when the first electrode 110 is a semi-transmissive electrode or a reflective electrode, a material for forming the first electrode 110 may include magnesium (Mg), silver (Ag), aluminum (Al), aluminum-lithium (Al-Li), calcium (Ca), magnesium-indium (Mg-In), magnesium-silver (Mg-Ag), or any combination thereof.

The first electrode 110 may have a single-layered structure including (e.g., consisting of) a single layer or a multi-layered structure including a plurality of layers. For example, the first electrode 110 may have a three-layered structure of ITO/Ag/ITO.

**Interlayer 130**

The interlayer 130 may be on the first electrode 110. The interlayer 130 may include an emission layer.

The interlayer 130 may further include a hole transport region between the first electrode 110 and the emission layer and an electron transport region between the emission layer and the second electrode 150.

In an embodiment, the interlayer 130 may further include, in addition to one or more suitable organic materials, a metal-containing compound (such as an organometallic compound), an inorganic material (such as quantum dots), and/or the like.

In one or more embodiments, the interlayer 130 may further include: i) two or more emitting units sequentially

stacked between the first electrode 110 and the second electrode 150; and ii) a charge generation layer between the two or more emitting units. When the interlayer 130 includes the two or more emitting units and the charge generation layer, the light-emitting device 10 may be a tandem light-emitting device.

**Hole transport region in interlayer 130**

The hole transport region may have i) a single-layered structure including (e.g., consisting of) a single layer including (e.g., consisting of) a single material, ii) a single-layered structure including (e.g., consisting of) a single layer including (e.g., consisting of) a plurality of different materials, or iii) a multi-layered structure including a plurality of layers including different materials.

The hole transport region may include a hole injection layer, a hole transport layer, an emission auxiliary layer, an electron blocking layer, or any combination thereof.

For example, the hole transport region may have a multi-layered structure including a hole injection layer/hole transport layer structure, a hole injection layer/hole transport layer/emission auxiliary layer structure, a hole injection layer/emission auxiliary layer structure, a hole transport layer/emission auxiliary layer structure, or a hole injection layer/hole transport layer/electron-blocking layer structure, wherein layers of each structure are sequentially stacked from the first electrode 110.

The hole transport region may include a compound represented by Formula 201, a compound represented by Formula 202, or any combination thereof:

Formula 201

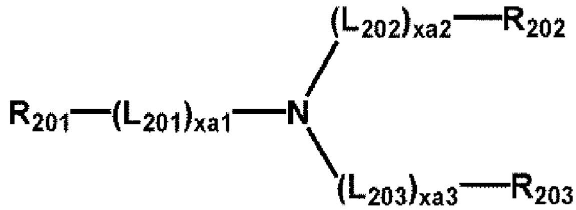

Formula 202

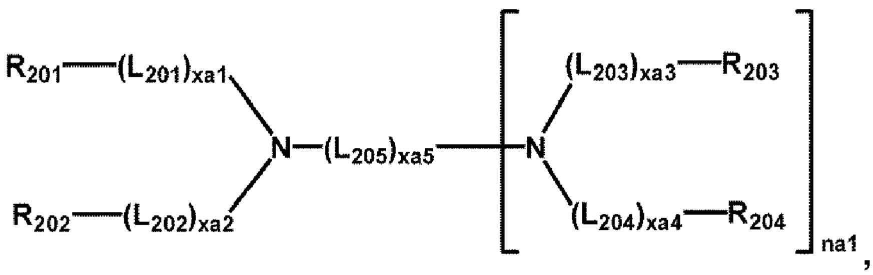

wherein, in Formulae 201 and 202,

$L_{201}$ to $L_{204}$ may each independently be a $C_3$-$C_{60}$ carbocyclic group unsubstituted or substituted with at least one $R_{10a}$ or a $C_1$-$C_{60}$ heterocyclic group unsubstituted or substituted with at least one $R_{10a}$,

$L_{205}$ may be *-O-*', *-S-*', *-N($Q_{201}$)-*', a $C_1$-$C_{20}$ alkylene group unsubstituted or substituted with at least one $R_{10a}$, a $C_2$-$C_{20}$ alkenylene group unsubstituted or substituted with at least one $R_{10a}$, a $C_3$-$C_{60}$ carbocyclic group unsubstituted or substituted with at least one $R_{10a}$, or a $C_1$-$C_{60}$ heterocyclic group unsubstituted or substituted with at least one $R_{10a}$,

xa1 to xa4 may each independently be an integer from 0 to 5,

xa5 may be an integer from 1 to 10,

$R_{201}$ to $R_{204}$ and $Q_{201}$ may each independently be a $C_3$-$C_{60}$ carbocyclic group unsubstituted or substituted with at least one $R_{10a}$ or a $C_1$-$C_{60}$ heterocyclic group unsubstituted or substituted with at least one $R_{10a}$,

$R_{201}$ and $R_{202}$ may optionally be linked to each other via a single bond, a $C_1$-$C_5$ alkylene group unsubstituted or substituted with at least one $R_{10a}$, or a $C_2$-$C_5$ alkenylene group unsubstituted or substituted with at least one $R_{10a}$ to form a $C_8$-$C_{60}$ polycyclic group (for example, a carbazole group and/or the like) unsubstituted or substituted with

at least one $R_{10a}$ (for example, Compound HT16),

$R_{203}$ and $R_{204}$ may optionally be linked to each other via a single bond, a $C_1$-$C_5$ alkylene group unsubstituted or substituted with at least one $R_{10a}$, or a $C_2$-$C_5$ alkenylene group unsubstituted or substituted with at least one $R_{10a}$ to form a $C_8$-$C_{60}$ polycyclic group unsubstituted or substituted with at least one $R_{10a}$, and

na1 may be an integer from 1 to 4.

For example, each of Formulae 201 and 202 may include at least one of groups represented by Formulae CY201 to CY217:

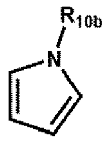
CY201
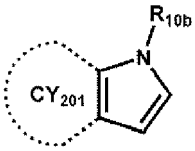
CY202
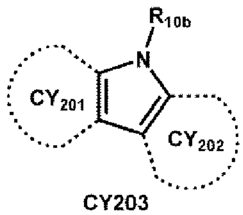
CY203
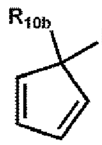
CY204
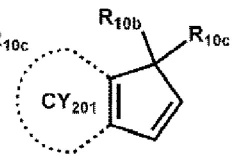
CY205
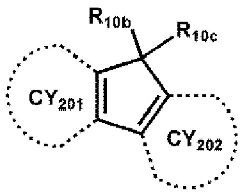
CY206

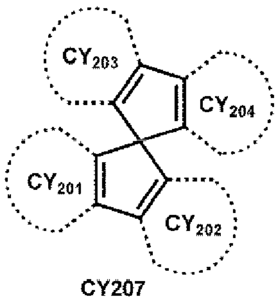
CY207
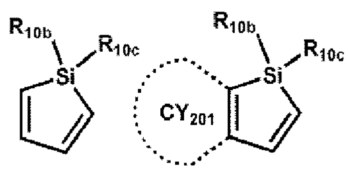
CY208
CY209
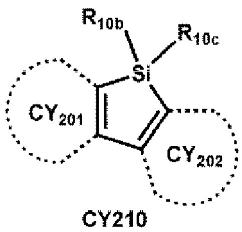
CY210
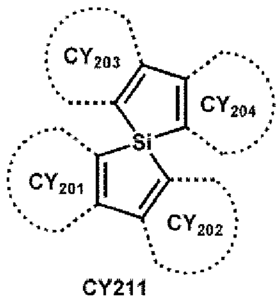
CY211

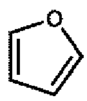
CY212
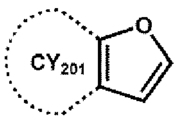
CY213
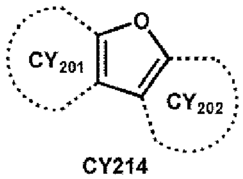
CY214
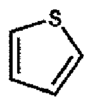
CY215
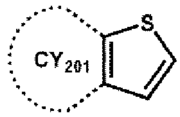
CY216
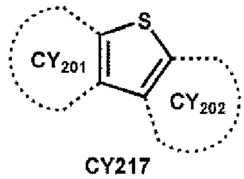
CY217 .

In Formulae CY201 to CY217, $R_{10b}$ and $R_{10c}$ may respectively be the same as described in connection with $R_{10a}$, ring $CY_{201}$ to ring $CY_{204}$ may each independently be a $C_3$-$C_{20}$ carbocyclic group or a $C_1$-$C_{20}$ heterocyclic group, and at least one hydrogen in each of Formulae CY201 to CY217 may be unsubstituted or substituted with Rioa.

In an embodiment, in Formulae CY201 to CY217, ring $CY_{201}$ to ring $CY_{204}$ may each independently be a benzene group, a naphthalene group, a phenanthrene group, or an anthracene group.

In one or more embodiments, each of Formulae 201 and 202 may include at least one of groups represented by Formulae CY201 to CY203.

In one or more embodiments, Formula 201 may include at least one of groups represented by Formulae CY201 to CY203 and at least one of groups represented by Formulae CY204 to CY217.

In one or more embodiments, in Formula 201, xa1 may be 1, $R_{201}$ may be a group represented by one of Formulae CY201 to CY203, xa2 may be 0, and $R_{202}$ may be a group represented by one of Formulae CY204 to CY207.

In one or more embodiments, each of Formulae 201 and 202 may not include (e.g., may exclude) a group represented by one of Formulae CY201 to CY203.

In one or more embodiments, each of Formulae 201 and 202 may not include (e.g., may exclude) a group represented by one of Formulae CY201 to CY203, and may include at least one of groups represented by Formulae CY204 to CY217.

In one or more embodiments, each of Formulae 201 and 202 may not include (e.g., may exclude) a group represented by one of Formulae CY201 to CY217.

For example, the hole transport region may include one of Compounds HT1 to HT46, m-MTDATA, TDATA, 2-TNATA, NPB(NPD), β-NPB, TPD, Spiro-TPD, Spiro-NPB, methylated NPB, TAPC, HMTPD, 4,4',4"-tris(N-carbazolyl)triphenylamine (TCTA), polyaniline/dodecylbenzene sulfonic acid (PANI/DBSA), poly(3,4-ethylene dioxythiophene)/poly(4-styrene sulfonate) (PEDOT/PSS), polyaniline/camphor sulfonic acid (PANI/CSA), polyaniline/poly(4-styrene sulfonate) (PANI/PSS), or any combination thereof:

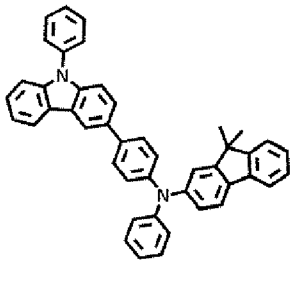

HT1

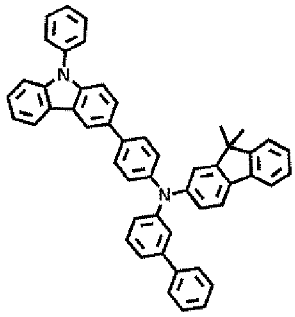

HT2

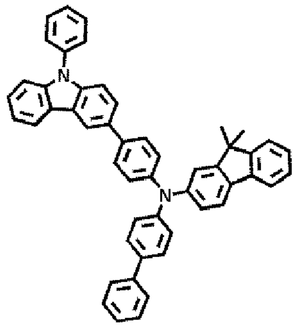

HT3

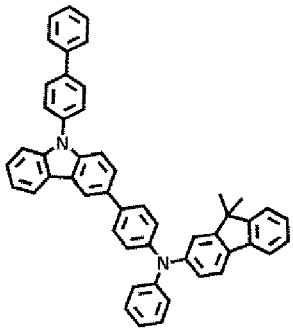

HT4

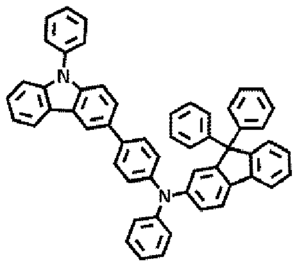

HT5

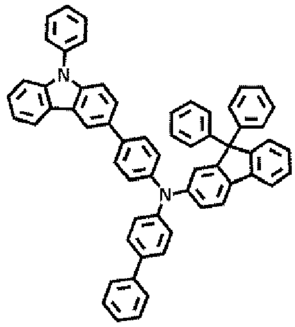

HT6

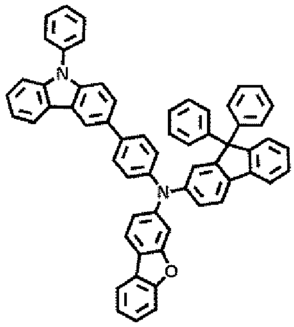

HT7

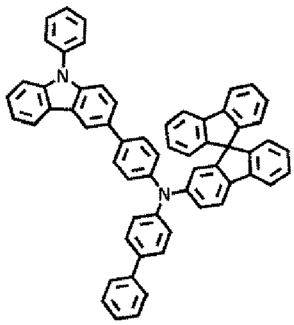

HT8

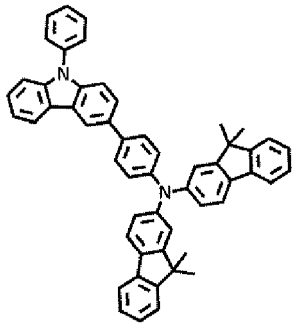

HT9

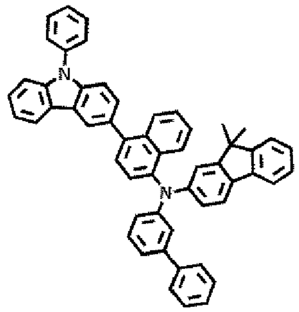

HT10

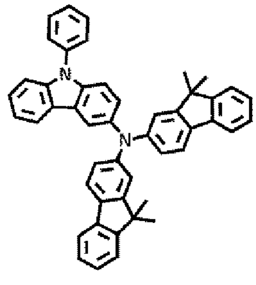

HT11

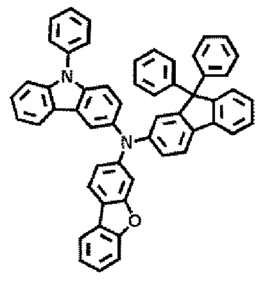

HT12

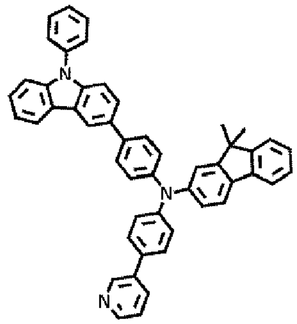

HT13

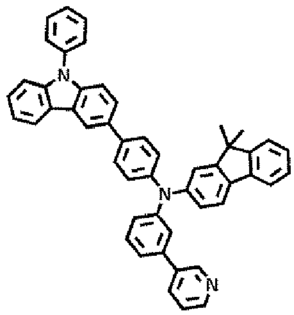

HT14

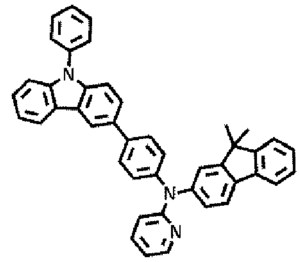

HT15

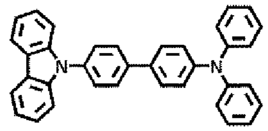

HT16

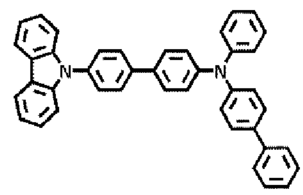

HT17

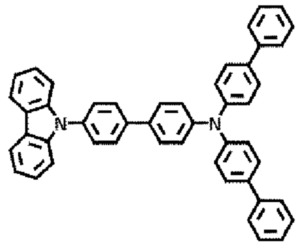

HT18

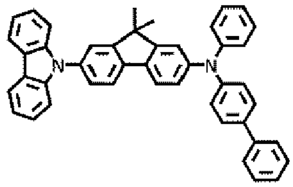

HT19

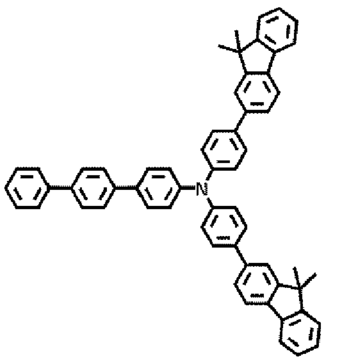

HT20

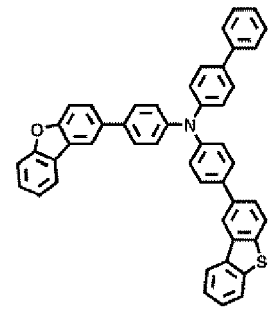

HT21

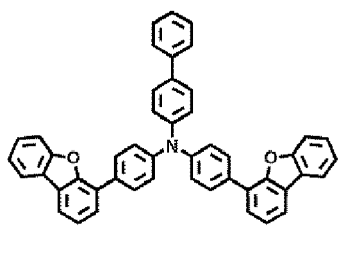

HT22

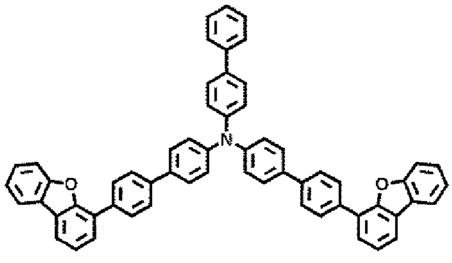

HT23

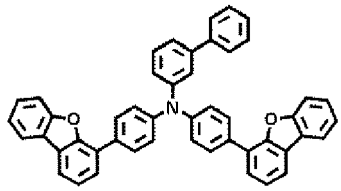

HT24

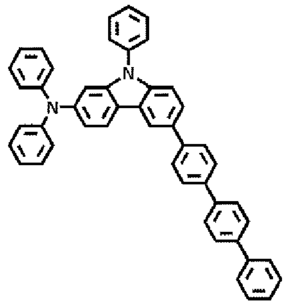

HT25

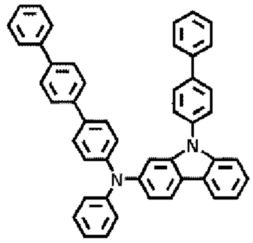

HT26

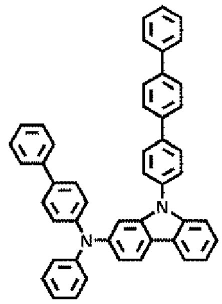

HT27

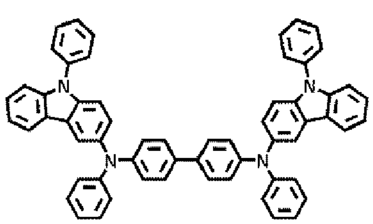

HT28

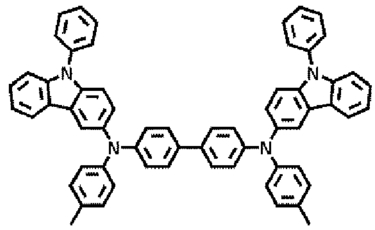

HT29

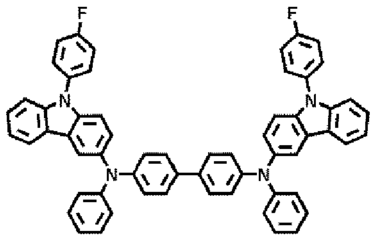

HT30

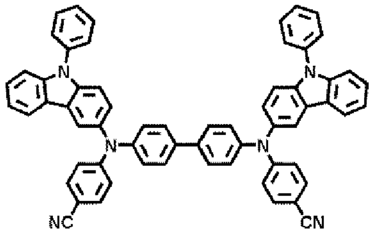

HT31

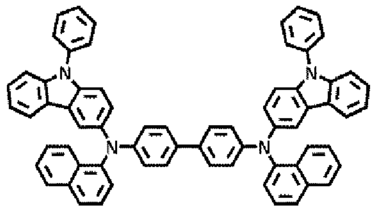

HT32

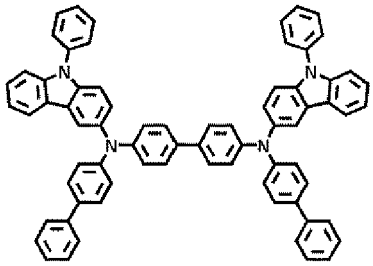

HT33

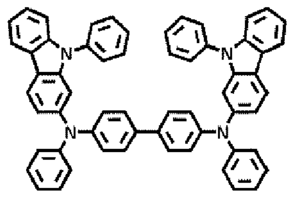

HT34

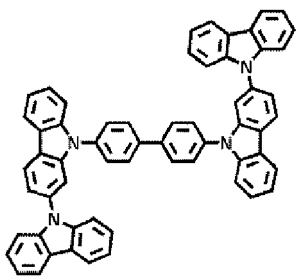

HT35

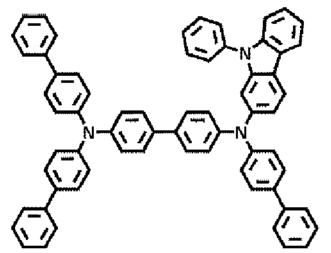

HT36

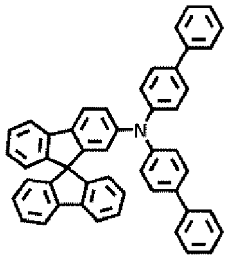

HT37

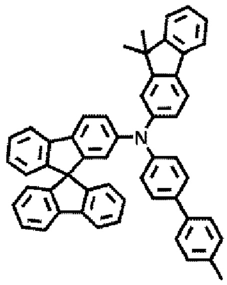

HT38

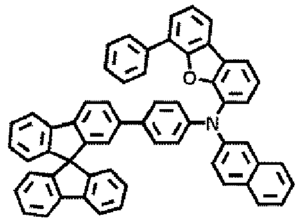

HT39

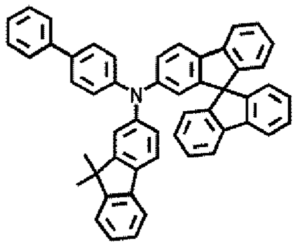

HT40

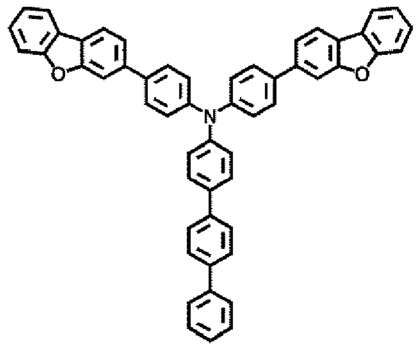

HT41

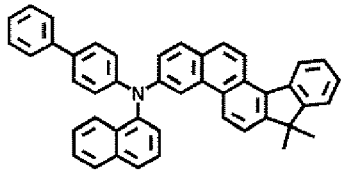

HT42

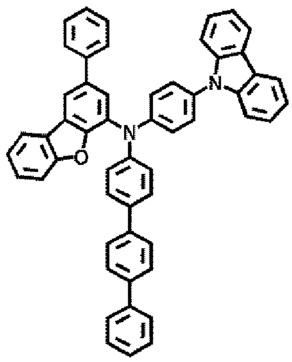

HT43

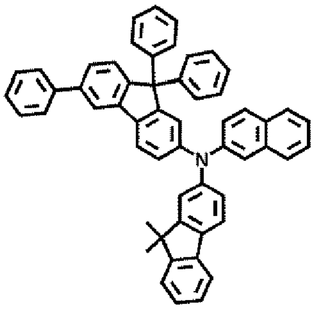

HT44

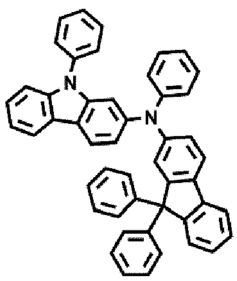

HT45

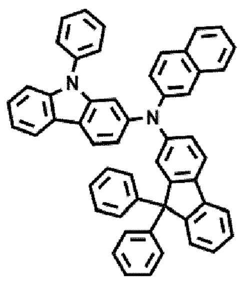

HT46

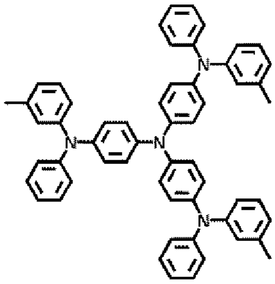
m-MTDATA

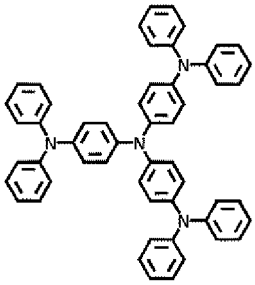
TDATA

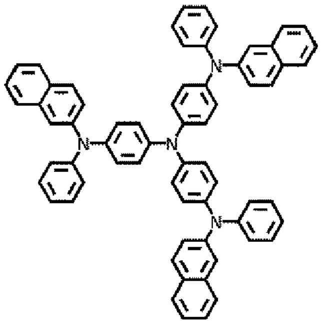
2-TNATA

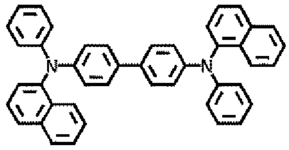
NPB

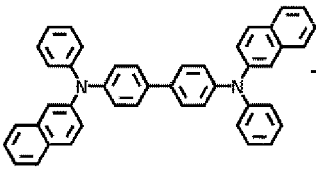
β-NPB

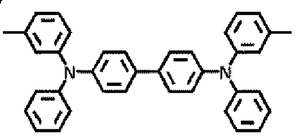
TPD

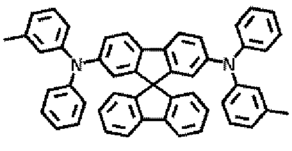
Spiro-TPD

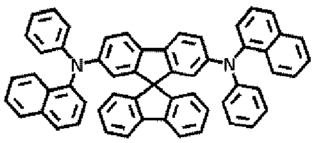
Spiro-NPB

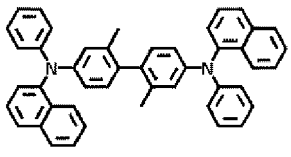
methylated-NPB

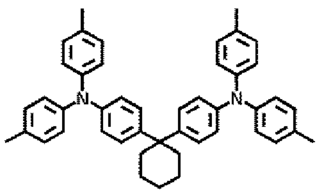
TAPC

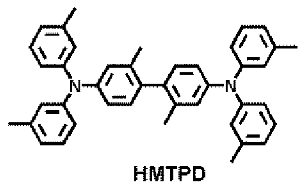
HMTPD

A thickness of the hole transport region may be in a range of about 50 Å to about 10,000 Å, for example, about 100 Å to about 4,000 Å. When the hole transport region includes a hole injection layer, a hole transport layer, or any combination thereof, a thickness of the hole injection layer may be in a range of about 100 Å to about 9,000 Å, for example, about 100 Å to about 1,000 Å, and a thickness of the hole transport layer may be in a range of about 50 Å to about 2,000 Å, for example, about 100 Å to about 1,500 Å. When the thicknesses of the hole transport region, the hole injection layer, and the hole transport layer are within these ranges, satisfactory hole transporting characteristics may be obtained without a substantial increase in driving voltage.

The emission auxiliary layer may increase the light-emission efficiency by compensating for an optical resonance distance of the wavelength of light emitted by the emission layer, and the electron blocking layer may block or reduce leakage of electrons from the emission layer to the hole transport region. Materials that may be included in the hole transport region may be included in the emission auxiliary layer and/or the electron-blocking layer.

**p-dopant**

The hole transport region may further include, in addition to these materials, a charge-generation material for the improvement of conductive properties. The charge-generation material may be substantially uniformly or non-uniformly dispersed in the hole transport region (for example, in the form of a single layer consisting of a charge-generation material).

The charge-generation material may be, for example, a p-dopant.

For example, the p-dopant may have a lowest unoccupied molecular orbital (LUMO) energy level of equal to or less than -3.5 eV.

In an embodiment, the p-dopant may include: a quinone derivative; a cyano group-containing compound; a compound containing an element EL1 and an element EL2; or any combination thereof.

Examples of the quinone derivative may include (e.g., may be) TCNQ, F4-TCNQ, and/or the like, and examples of the cyano group-containing compound may include (e.g., may be) HAT-CN, a compound represented by Formula 221, and/or the like:

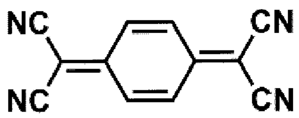

**TCNQ**

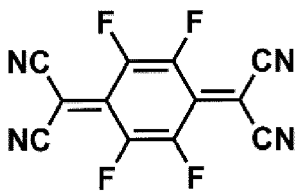

**F4-TCNQ**

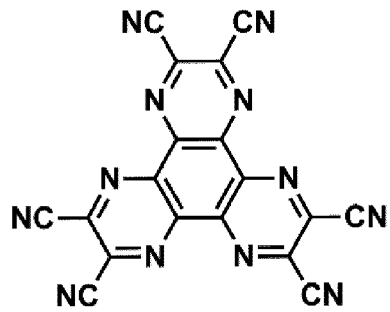

**HAT-CN**

**Formula 221**

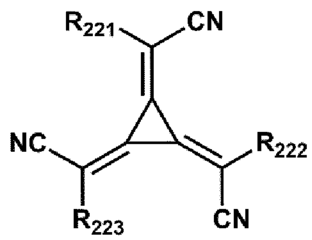

In Formula 221,

$R_{221}$ to $R_{223}$ may each independently be a $C_3$-$C_{60}$ carbocyclic group unsubstituted or substituted with at least one $R_{10a}$ or a $C_1$-$C_{60}$ heterocyclic group unsubstituted or substituted with at least one $R_{10a}$, and
at least one of $R_{221}$ to $R_{223}$ may each independently be a $C_3$-$C_{60}$ carbocyclic group or a $C_1$-$C_{60}$ heterocyclic group, each substituted with: a cyano group; -F; -Cl; -Br; -I; a $C_1$-$C_{20}$ alkyl group substituted with a cyano group, -F, -Cl, -Br, -I, or any combination thereof; or any combination thereof.

In the compound including the element EL1 and the element EL2, the element EL1 may be a metal, a metalloid, or any combination thereof, and the element EL2 may be a non-metal, a metalloid, or any combination thereof.

Examples of the metal may include (e.g., may be) an alkali metal (for example, lithium (Li), sodium (Na), potassium (K), rubidium (Rb), cesium (Cs), and/or the like); an alkaline earth metal (for example, beryllium (Be), magnesium (Mg), calcium (Ca), strontium (Sr), barium (Ba), and/or the like); a transition metal (for example, titanium (Ti), zirconium (Zr), hafnium (Hf), vanadium (V), niobium (Nb), tantalum (Ta), chromium (Cr), molybdenum (Mo), tungsten (W), manganese (Mn), technetium (Tc), rhenium (Re), iron (Fe), ruthenium (Ru), osmium (Os), cobalt (Co), rhodium (Rh), iridium (Ir), nickel (Ni), palladium (Pd), platinum (Pt), copper (Cu), silver (Ag), gold (Au), and/or the like); a post-transition metal (for example, zinc (Zn), indium (In), tin (Sn), and/or the like); and a lanthanide metal (for example, lanthanum (La), cerium (Ce), praseodymium (Pr), neodymium (Nd), promethium (Pm), samarium (Sm), europium (Eu), gadolinium (Gd), terbium (Tb), dysprosium (Dy), holmium (Ho), erbium (Er), thulium (Tm), ytterbium (Yb), lutetium (Lu), and/or the like).

Examples of the metalloid may include (e.g., may be) silicon (Si), antimony (Sb), tellurium (Te), and/or the like.

Examples of the non-metal may include (e.g., may be) oxygen (O), a halogen (for example, F, Cl, Br, I, and/or the like), and/or the like.

Examples of the compound containing the element EL1 and the element EL2 may include (e.g., may be) a metal oxide, a metal halide (for example, a metal fluoride, a metal chloride, a metal bromide, a metal iodide, and/or the like), a metalloid halide (for example, a metalloid fluoride, a metalloid chloride, a metalloid bromide, a metalloid iodide, and/or the like), a metal telluride, or any combination thereof.

Examples of the metal oxide may include (e.g., may be) a tungsten oxide (for example, WO, $W_2O_3$, $WO_2$, $WO_3$, $W_2O_5$, and/or the like), a vanadium oxide (for example, VO, $V_2O_3$, $VO_2$, $V_2O_5$, and/or the like), a molybdenum oxide (MoO, $Mo_2O_3$, $MoO_2$, $MoO_3$, $Mo_2O_5$, and/or the like), a rhenium oxide (for example, $ReO_3$, and/or the like), and/or the like.

Examples of the metal halide may include (e.g., may be) an alkali metal halide, an alkaline earth metal halide, a transition metal halide, a post-transition metal halide, a lanthanide metal halide, and/or the like.

Examples of the alkali metal halide may include (e.g., may be) LiF, NaF, KF, RbF, CsF, LiCl, NaCl, KCl, RbCl, CsCl, LiBr, NaBr, KBr, RbBr, CsBr, LiI, NaI, KI, RbI, CsI, and/or the like.

Examples of the alkaline earth metal halide may include (e.g., may be) $BeF_2$, MgF2, CaF2, SrF2, BaF2, $BeCl_2$,

$MgCl_2$, $CaCl_2$, $SrCl_2$, $BaCl_2$, BeBr2, MgBr2, CaBr2, SrBr2, BaBr2, $BeI_2$, $MgI_2$, CaI2, $SrI_2$, $BaI_2$, and/or the like.

Examples of the transition metal halide may include (e.g., may be) a titanium halide (for example, $TiF_4$, $TiCl_4$, $TiBr_4$, $TiI_4$, and/or the like), a zirconium halide (for example, $ZrF_4$, $ZrCl_4$, $ZrBr_4$, $ZrI_4$, and/or the like), a hafnium halide (for example, $HfF_4$, $HfCl_4$, $HfBr_4$, $HfI_4$, and/or the like), a vanadium halide (for example, $VF_3$, $VCl_3$, $VBr_3$, $VI_3$, and/or the like), a niobium halide (for example, $NbF_3$, $NbCl_3$, $NbBr_3$, $NbI_3$, and/or the like), a tantalum halide (for example, $TaF_3$, $TaCl_3$, $TaBr_3$, $TaI_3$, and/or the like), a chromium halide (for example, $CrF_3$, $CrCl_3$, $CrBr_3$, $CrI_3$, and/or the like), a molybdenum halide (for example, $MoF_3$, $MoCl_3$, $MoBr_3$, $MoI_3$, and/or the like), a tungsten halide (for example, $WF_3$, $WCl_3$, $WBr_3$, $WI_3$, and/or the like), a manganese halide (for example, $MnF_2$, $MnCl_2$, $MnBr_2$, $MnI_2$, and/or the like), a technetium halide (for example, $TcF_2$, $TcCl_2$, $TcBr_2$, $TcI_2$, and/or the like), a rhenium halide (for example, $ReF_2$, $ReCl_2$, $ReBr_2$, $ReI_2$, and/or the like), an iron halide (for example, $FeF_2$, $FeCl_2$, $FeBr_2$, $FeI_2$, and/or the like), a ruthenium halide (for example, $RuF_2$, $RuCl_2$, $RuBr_2$, $RuI_2$, and/or the like), an osmium halide (for example, $OsF_2$, $OsCl_2$, $OsBr_2$, $OsI_2$, and/or the like), a cobalt halide (for example, $CoF_2$, $CoCl_2$, $CoBr_2$, $CoI_2$, and/or the like), a rhodium halide (for example, $RhF_2$, $RhCl_2$, $RhBr_2$, $RhI_2$, and/or the like), an iridium halide (for example, $IrF_2$, $IrCl_2$, $IrBr_2$, $IrI_2$, and/or the like), a nickel halide (for example, $NiF_2$, $NiCl_2$, $NiBr_2$, $NiI_2$, and/or the like), a palladium halide (for example, $PdF_2$, $PdCl_2$, $PdBr_2$, $PdI_2$, and/or the like), a platinum halide (for example, $PtF_2$, $PtCl_2$, $PtBr_2$, $PtI_2$, and/or the like), a copper halide (for example, CuF, CuCl, CuBr, CuI, and/or the like), a silver halide (for example, AgF, AgCl, AgBr, AgI, and/or the like), a gold halide (for example, AuF, AuCl, AuBr, AuI, and/or the like), and/or the like.

Examples of the post-transition metal halide may include (e.g., may be) a zinc halide (for example, $ZnF_2$, $ZnCl_2$, $ZnBr_2$, $ZnI_2$, and/or the like), an indium halide (for example, $InI_3$, etc.), a tin halide (for example, $SnI_2$, and/or the like), and/or the like.

Examples of the lanthanide metal halide may include (e.g., may be) YbF, $YbF_2$, $YbF_3$, $SmF_3$, YbCl, $YbCl_2$, $YbCl_3$ $SmCl_3$, YbBr, $YbBr_2$, $YbBr_3$, $SmBr_3$, YbI, $YbI_2$, $YbI_3$, $SmI_3$, and/or the like.

Examples of the metalloid halide may include (e.g., may be) an antimony halide (for example, $SbCl_5$ and/or the like) and/or the like.

Examples of the metal telluride may include (e.g., may be) alkali metal telluride (for example, $Li_2Te$, $Na_2Te$, $K_2Te$, $Rb_2Te$, $Cs_2Te$, and/or the like), an alkaline earth metal telluride (for example, BeTe, MgTe, CaTe, SrTe, BaTe, and/or the like), a transition metal telluride (for example, TiTe2, ZrTe2, HfTe2, V2Te3, Nb2Te3, Ta2Te3, $Cr_2Te_3$, $Mo_2Te_3$, $W_2Te_3$, MnTe, TcTe, ReTe, FeTe, RuTe, OsTe, CoTe, RhTe, IrTe, NiTe, PdTe, PtTe, $Cu_2Te$, CuTe, $Ag_2Te$, AgTe, $Au_2Te$, and/or the like), a post-transition metal telluride (for example, $In_2Te_3$, SnTe, ZnTe, and/or the like), a lanthanide metal telluride (for example, LaTe, CeTe, PrTe, NdTe, PmTe, EuTe, GdTe, TbTe, DyTe, HoTe, ErTe, TmTe, YbTe, LuTe, and/or the like), and/or the like.

**Emission layer in interlayer 130**

When the light-emitting device 10 is a full-color light-emitting device, the emission layer may be patterned into a red emission layer, a green emission layer, and/or a blue emission layer, according to a sub-pixel. In an embodiment, the emission layer may have a stacked structure of two or more layers of a red emission layer, a green emission layer, and a blue emission layer, in which the two or more layers may contact each other or may be separated from each other to emit white light. In one or more embodiments, the emission layer may be a structure in which two or more materials selected from a red light-emitting material, a green light-emitting material, and a blue light-emitting material are mixed with each other in a single layer to emit white light.

The emission layer may include a host and a dopant. The dopant may include a phosphorescent dopant, a fluorescent dopant, or any combination thereof.

In the emission layer, an amount of the dopant may be in a range of about 0.01 parts by weight to about 15 parts by weight based on 100 parts by weight of the host.

In one or more embodiments, the emission layer may include quantum dots.

In one or more embodiments, the emission layer may include a delayed fluorescence material. The delayed fluorescence material may act as a host or a dopant in the emission layer.

A thickness of the emission layer may be in a range of about 100 Å to about 1,000 Å, for example, about 200 Å to about 600 Å. When the thickness of the emission layer is within these ranges, excellent or suitable luminescence characteristics may be obtained without a substantial increase in driving voltage.

**Host**

In an embodiment, the host may include a compound represented by Formula 301:

**Formula 301**

$$[Ar_{301}]_{xb11}-[(L_{301})_{xb1}-R_{301}]_{xb21},$$

wherein, in Formula 301,

$Ar_{301}$ and $L_{301}$ may each independently be a $C_3$-$C_{60}$ carbocyclic group unsubstituted or substituted with at least one $R_{10a}$ or a $C_1$-$C_{60}$ heterocyclic group unsubstituted or substituted with at least one $R_{10a}$,
xb11 may be 1, 2, or 3,
xb1 may be an integer from 0 to 5,
$R_{301}$ may be hydrogen, deuterium, -F, -Cl, -Br, -I, a hydroxyl group, a cyano group, a nitro group, a $C_1$-$C_{60}$ alkyl group unsubstituted or substituted with at least one $R_{10a}$, a $C_2$-$C_{60}$ alkenyl group unsubstituted or substituted with at least one $R_{10a}$, a $C_2$-$C_{60}$ alkynyl group unsubstituted or substituted with at least one $R_{10a}$, a $C_1$-$C_{60}$ alkoxy group unsubstituted or substituted with at least one $R_{10a}$, a $C_3$-$C_{60}$ carbocyclic group unsubstituted or substituted with at least one $R_{10a}$, a $C_1$-$C_{60}$ heterocyclic group unsubstituted or substituted with at least one $R_{10a}$,-Si($Q_{301}$)($Q_{302}$)($Q_{303}$), -N($Q_{301}$)($Q_{302}$), -B($Q_{301}$)($Q_{302}$), -C(=O)($Q_{301}$), -S(=O)$_2$($Q_{301}$), or -P(=O)($Q_{301}$)($Q_{302}$),
xb21 may be an integer from 1 to 5, and
$Q_{301}$ to $Q_{303}$ may each independently be the same as described in connection with $Q_1$.

For example, when xb11 in Formula 301 is 2 or more, two or more $Ar_{301}$(s) may be linked to each other via a single bond.

In one or more embodiments, the host may include a compound represented by Formula 301-1, a compound represented by Formula 301-2, or any combination thereof:

**Formula 301-1**

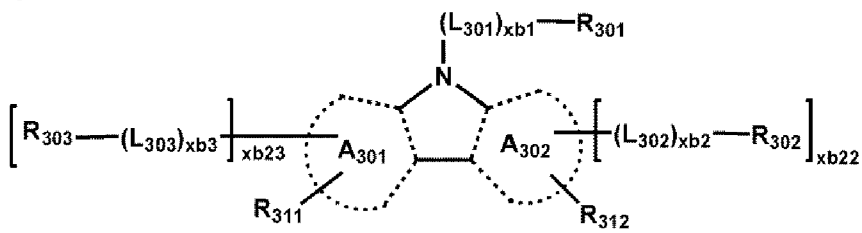

**Formula 301-2**

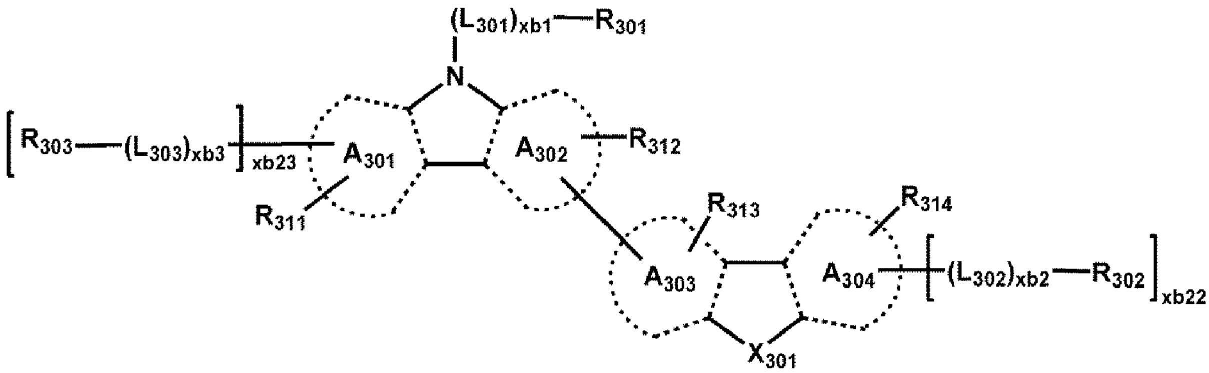

,

wherein, in Formulae 301-1 and 301-2,

ring $A_{301}$ to ring $A_{304}$ may each independently be a $C_3$-$C_{60}$ carbocyclic group unsubstituted or substituted with at least one $R_{10a}$ or a $C_1$-$C_{60}$ heterocyclic group unsubstituted or substituted with at least one $R_{10a}$,
X301 may be O, S, N-[(L304)xb4-R304], C(R304)(R305), or Si(R304)(R305),
xb22 and xb23 may each independently be 0, 1, or 2,
$L_{301}$, xb1, and $R_{301}$ may each independently be the same as described herein,
$L_{302}$ to $L_{304}$ may each independently be the same as described in connection with $L_{301}$,

xb2 to xb4 may each independently be the same as described in connection with xb1, and
$R_{302}$ to $R_{305}$ and $R_{311}$ to $R_{314}$ may each independently be the same as described in connection with $R_{301}$.

In one or more embodiments, the host may include an alkali earth metal complex, a post-transition metal complex, or any combination thereof. For example, the host may include a Be complex (for example, Compound H55), an Mg complex, a Zn complex, or any combination thereof.

In one or more embodiments, the host may include one of Compounds H1 to H124, 9,10-di(2-naphthyl)anthracene (ADN), 2-methyl-9,10-bis(naphthalen-2-yl)anthracene (MADN), 9,10-di-(2-naphthyl)-2-t-butyl-anthracene (TBADN), 4,4'-bis(N-carbazolyl)-1,1'-biphenyl (CBP), 1,3-di-9-carbazolylbenzene (mCP), 1,3,5-tri(carbazol-9-yl)benzene (TCP), or any combination thereof:

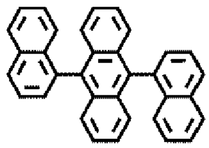
H1

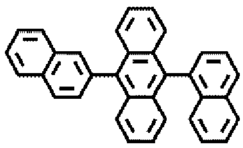
H2

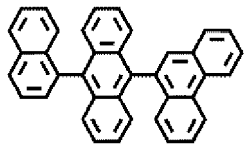
H3

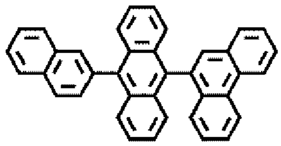
H4

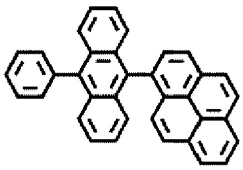
H5

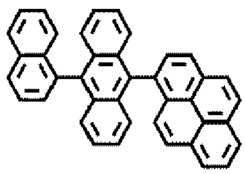
H6

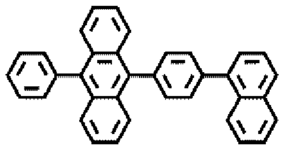
H7

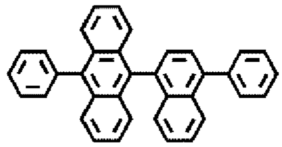
H8

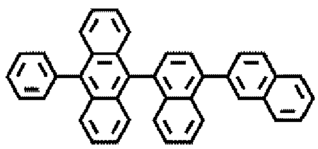
H9

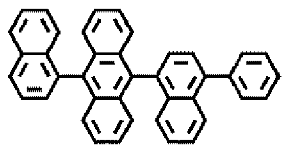
H10

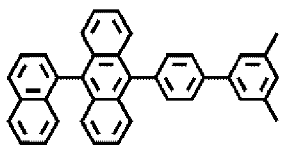
H11

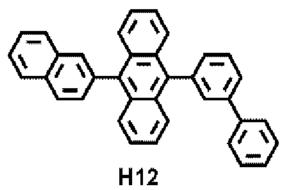
H12

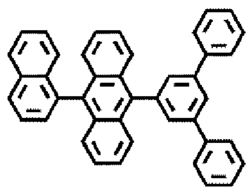
H13

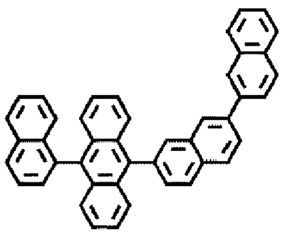
H14

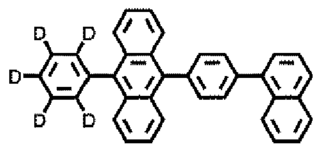
H15

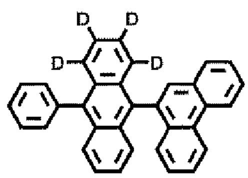
H16

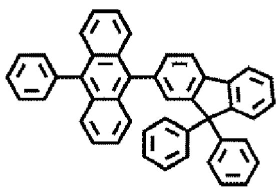
H17

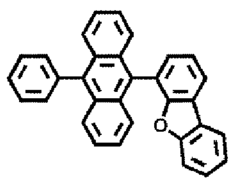
H18

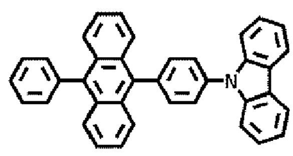
H19

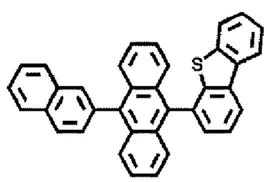
H20

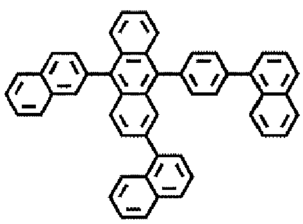
H21

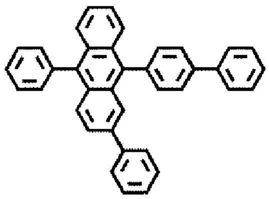
H22

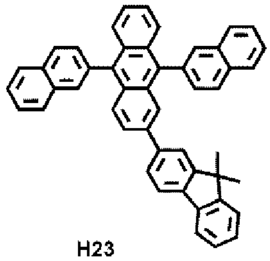
H23

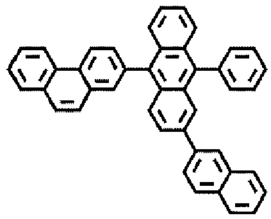
H24

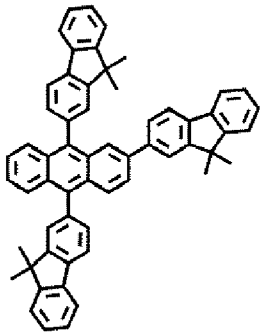
H25

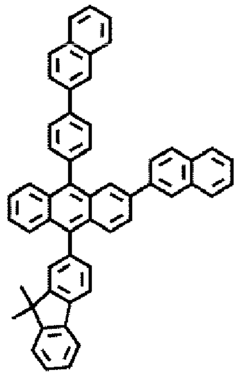
H26

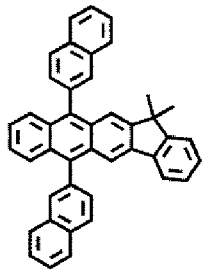
H27

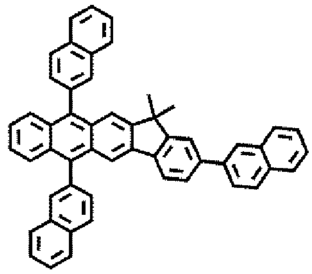
H28

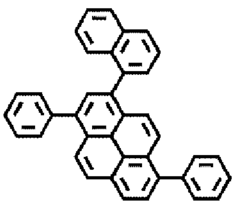
H29

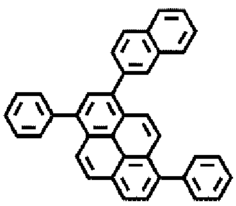
H30

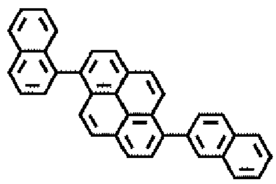
H31

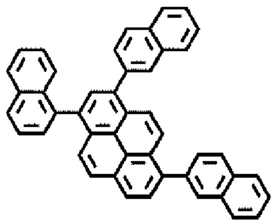
H32

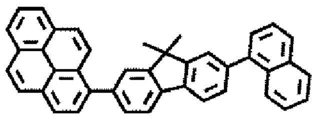
H33

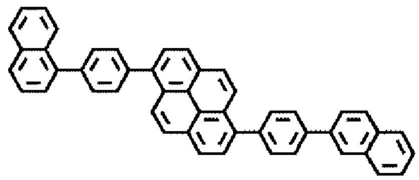
H34

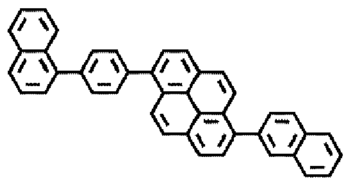
H35

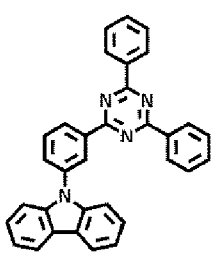
H36

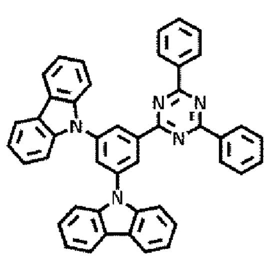
H37

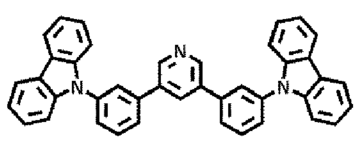
H38

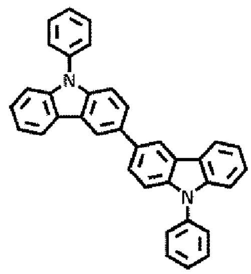
H39

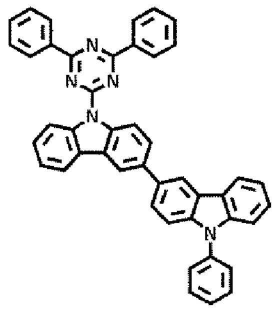
H40

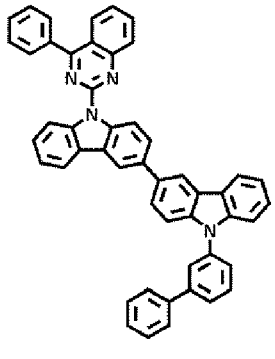
H41

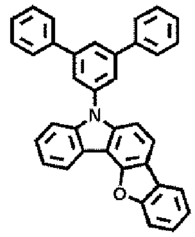
H42

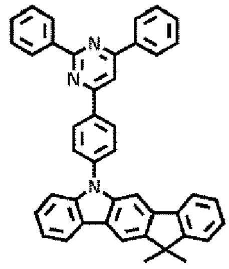
H43

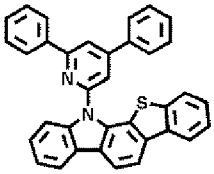
H44

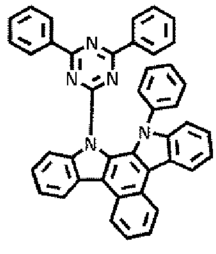
H45

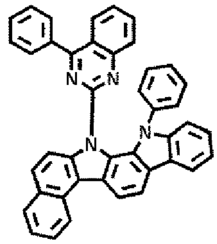
H46

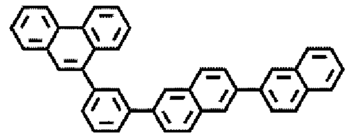
H47

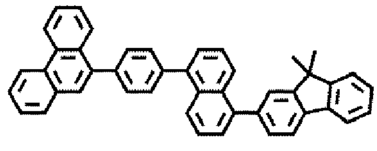
H48

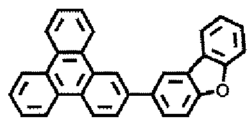
H49

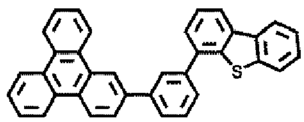
H50

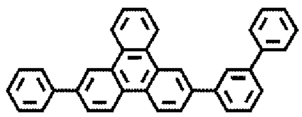
H51

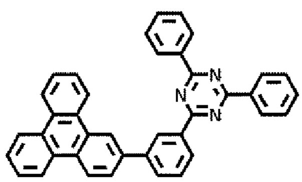
H52

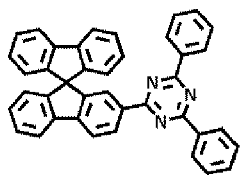
H53

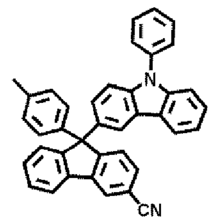
H54

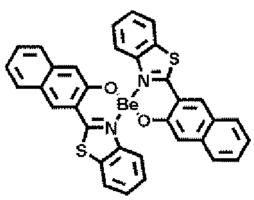
H55

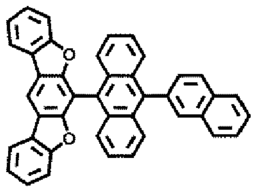
H56

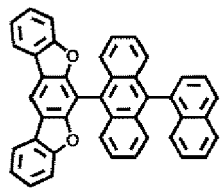
H57

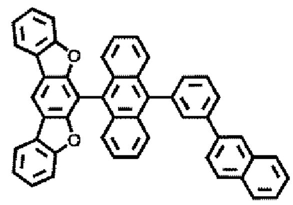
H58

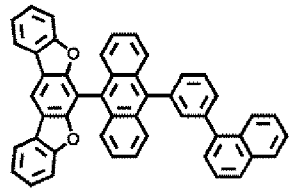
H59

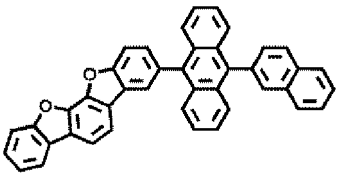
H60

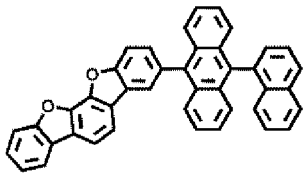
H61

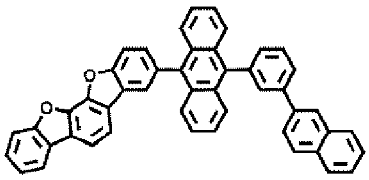
H62

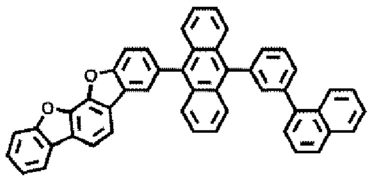
H63

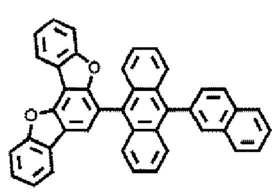
H64

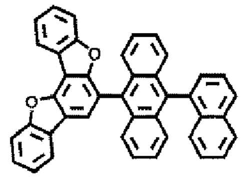
H65

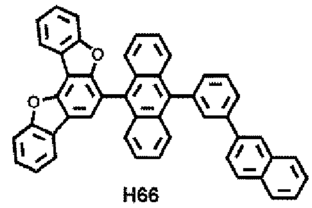
H66

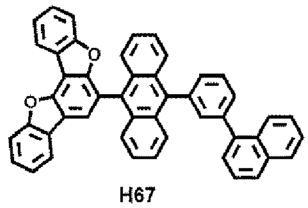
H67

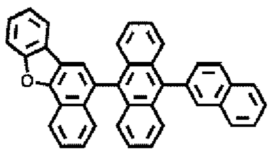
H68

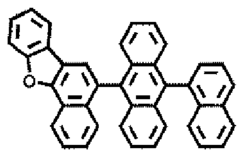
H69

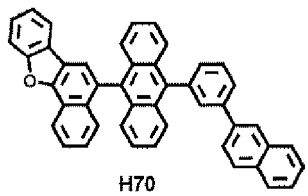
H70

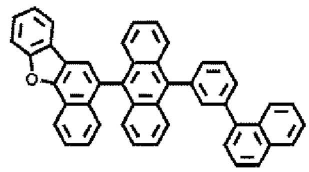
H71

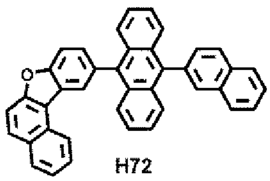
H72

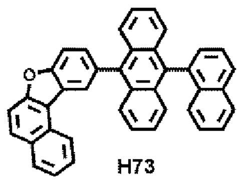
H73

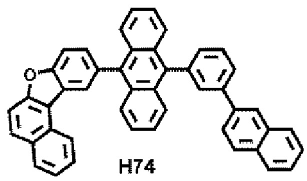
H74

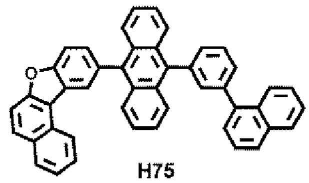
H75

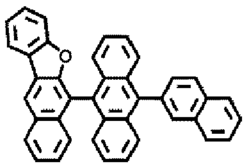
H76

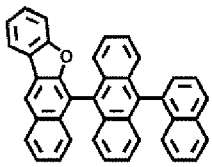
H77

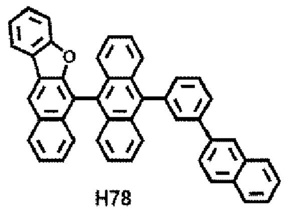
H78

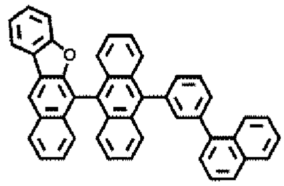
H79

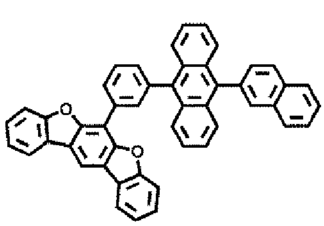
H80

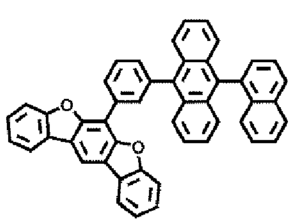
H81

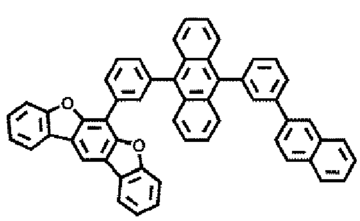
H82

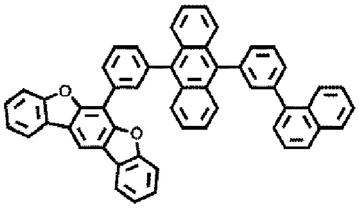

H83

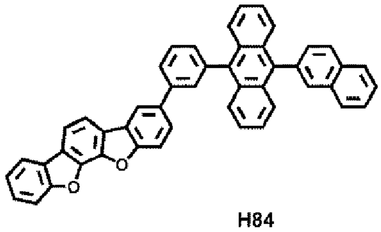

H84

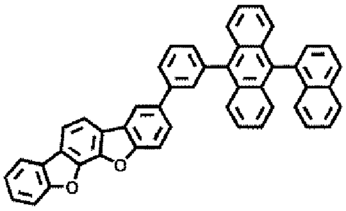

H85

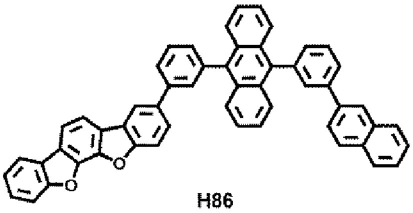

H86

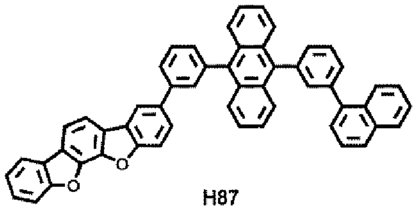

H87

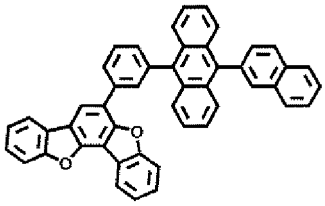

H88

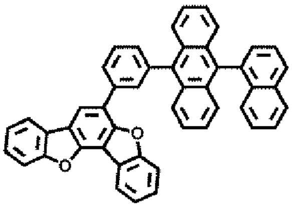

H89

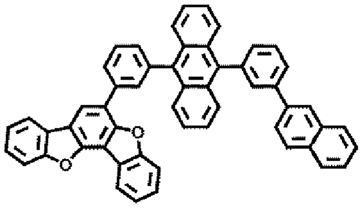

H90

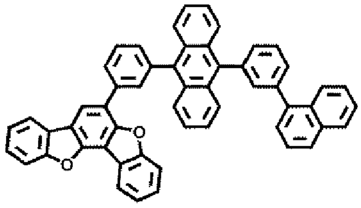

H91

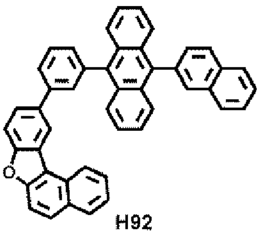

H92

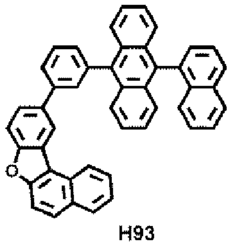

H93

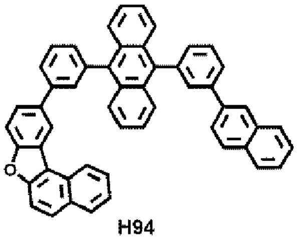

H94

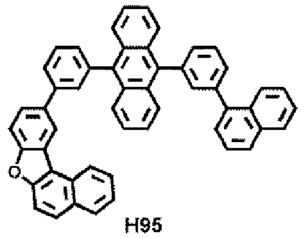

H95

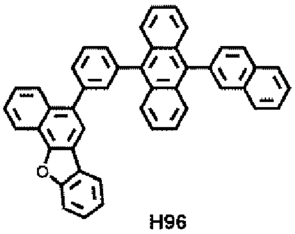

H96

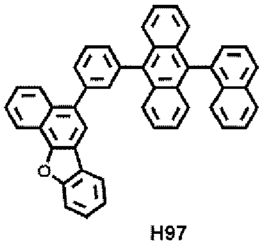

H97

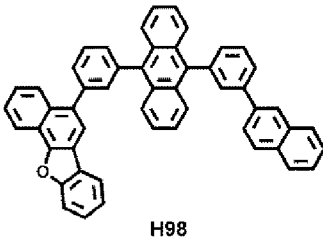

H98

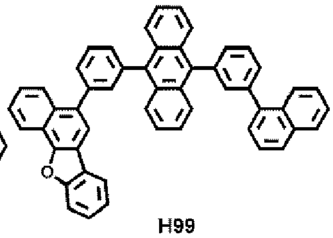

H99

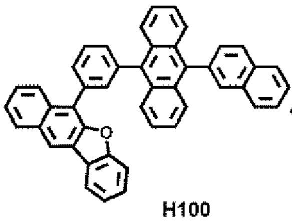

H100

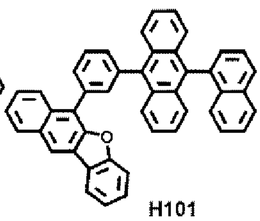

H101

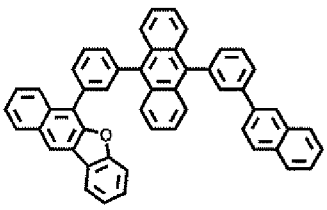

H102

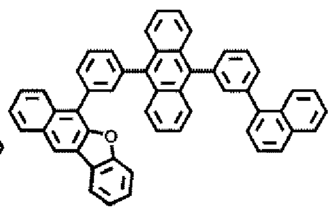

H103

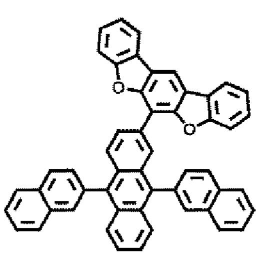
H104

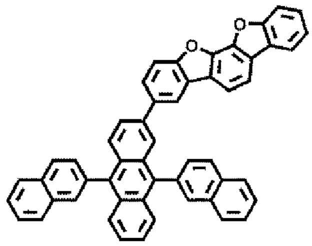
H105

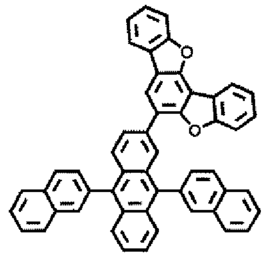
H106

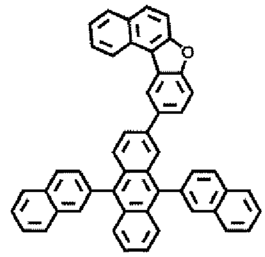
H107

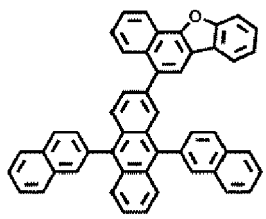
H108

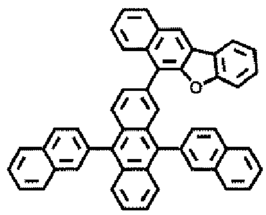
H109

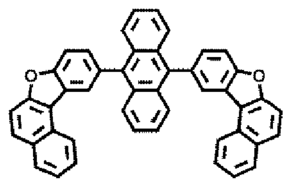
H110

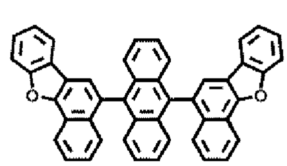
H111

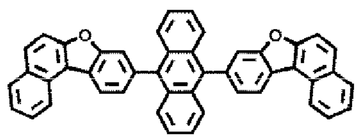
H112

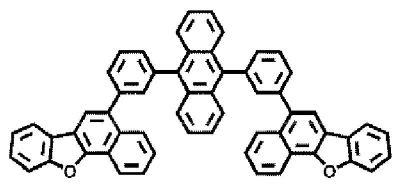
H113

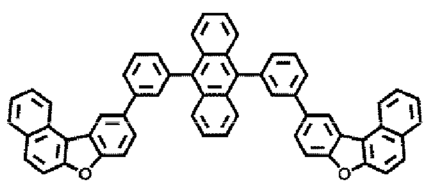
H114

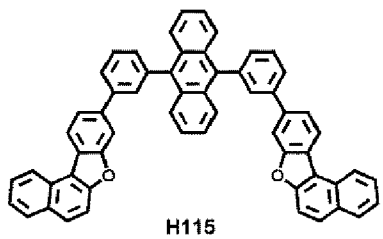
H115

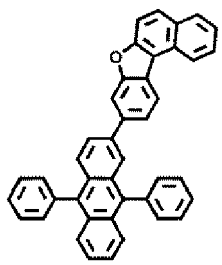
H116

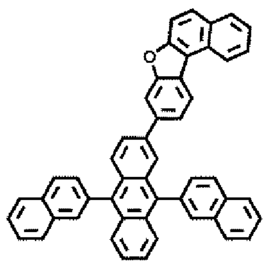
H117

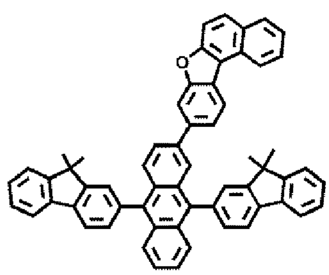
H118

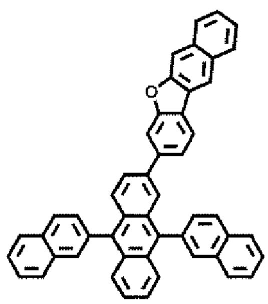
H119

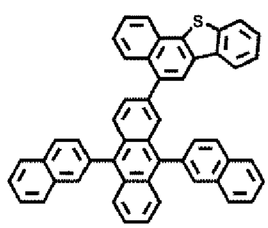
H120

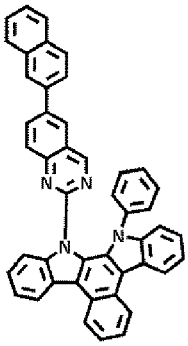

H121

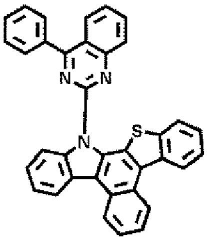

H122

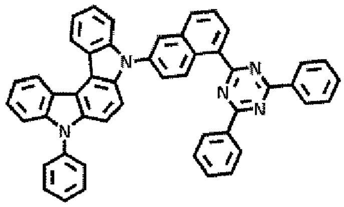

H123

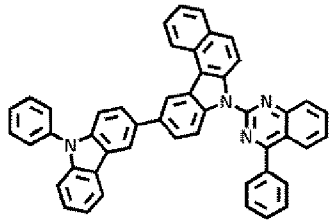

H124

.

**Phosphorescent dopant**

In an embodiment, the phosphorescent dopant may include at least one transition metal as a central metal.

In an embodiment, the phosphorescent dopant may include a monodentate ligand, a bidentate ligand, a tridentate ligand, a tetradentate ligand, a pentadentate ligand, a hexadentate ligand, or any combination thereof.

In an embodiment, the phosphorescent dopant may be electrically neutral.

In an embodiment, the phosphorescent dopant may include an organometallic compound represented by Formula 401:

**Formula 401**

$$M(L_{401})_{xc1}(L_{402})_{xc2}$$

**Formula 402**

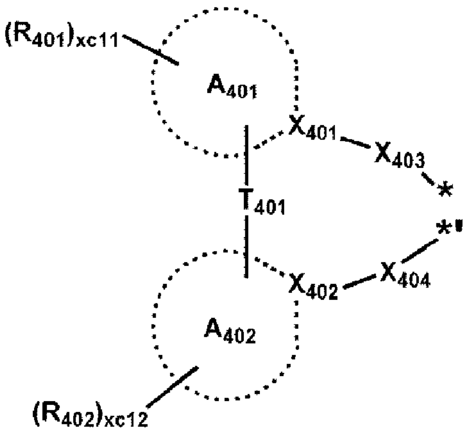

,

wherein, in Formulae 401 and 402,

- M may be a transition metal (for example, iridium (Ir), platinum (Pt), palladium (Pd), osmium (Os), titanium (Ti), gold (Au), hafnium (Hf), europium (Eu), terbium (Tb), rhodium (Rh), rhenium (Re), and/or thulium (Tm)),
- $L_{401}$ may be a ligand represented by Formula 402, and xc1 may be 1, 2, or 3, wherein, when xc1 is 2 or more, two or more $L_{401}$(s) may be identical to or different from each other,
- $L_{402}$ may be an organic ligand, and xc2 may be 0, 1, 2, 3, or 4, wherein, when xc2 is 2 or more, two or more $L_{402}$(s) may be identical to or different from each other,
- X401 and X402 may each independently be nitrogen or carbon,
- ring $A_{401}$ and ring $A_{402}$ may each independently be a $C_3$-$C_{60}$ carbocyclic group or a $C_1$-$C_{60}$ heterocyclic group,
- $T_{401}$ may be a single bond, *-O-*', *-S-*', *-C(=O)-*', *-N($Q_{411}$)-*', *-C($Q_{411}$)($Q_{412}$)-*', *-C($Q_{411}$)=C($Q_{412}$)-*', *-C($Q_{411}$)=*', or *=C=*',
- $X_{403}$ and $X_{404}$ may each independently be a chemical bond (for example, a covalent bond or a coordinate bond),

O, S, N(Q413), B(Q413), P(Q413), $C(Q_{413})(Q_{414})$, or Si(Q413)(Q414),
$Q_{411}$ to $Q_{414}$ may each independently be the same as described in connection with $Q_1$,
$R_{401}$ and $R_{402}$ may each independently be hydrogen, deuterium, -F, -Cl, -Br, - I, a hydroxyl group, a cyano group, a nitro group, a $C_1$-$C_{20}$ alkyl group unsubstituted or substituted with at least one $R_{10a}$, a $C_1$-$C_{20}$ alkoxy group unsubstituted or substituted with at least one $R_{10a}$, a $C_3$-$C_{60}$ carbocyclic group unsubstituted or substituted with at least one $R_{10a}$, a $C_1$-$C_{60}$ heterocyclic group unsubstituted or substituted with at least one $R_{10a}$, $-Si(Q_{401})(Q_{402})(Q_{403})$, $-N(Q_{401})(Q_{402})$, $-B(Q_{401})(Q_{402})$, $-C(=O)(Q_{401})$, - $S(=O)_2(Q_{401})$, or $-P(=O)(Q_{401})(Q_{402})$,
$Q_{401}$ to $Q_{403}$ may each independently be the same as described in connection with $Q_1$,
xc11 and xc12 may each independently be an integer from 0 to 10, and
* and *' in Formula 402 each indicate a binding site to M in Formula 401.

For example, in Formula 402, i) $X_{401}$ may be nitrogen and $X_{402}$ may be carbon, or ii) each of X401 and X402 may be nitrogen.

When xc1 in Formula 401 is 2 or more, two ring $A_{401}$(s) in two or more $L_{401}$(s) may optionally be linked to each other via $T_{402}$, which is a linking group, and two ring $A_{402}$(s) in two or more $L_{401}$(s) may optionally be linked to each other via $T_{403}$, which is a linking group (see Compounds PD1 to PD4 and PD7), wherein $T_{402}$ and $T_{403}$ may respectively be the same as described in connection with $T_{401}$.

In Formula 401, $L_{402}$ may be any suitable organic ligand. For example, $L_{402}$ may include a halogen group, a diketone group (for example, an acetylacetonate group), a carboxylic acid group (for example, a picolinate group), -C(=O), an isonitrile group, a -CN group, a phosphorus group (for example, a phosphine group, a phosphite group, and/or the like), or any combination thereof.

In one or more embodiments, the phosphorescent dopant may include, for example, one of Compounds PD1 to PD39, or any combination thereof:

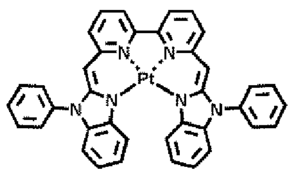

PD1

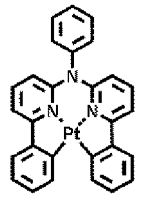

PD2

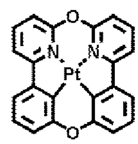

PD3

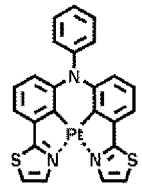

PD4

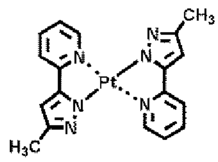

PD5

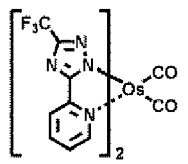

PD6

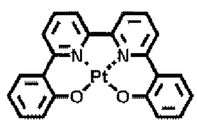

PD7

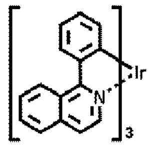

PD8

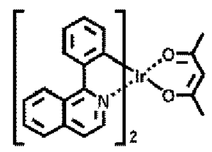

PD9

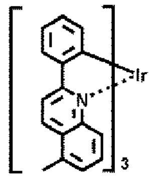

PD10

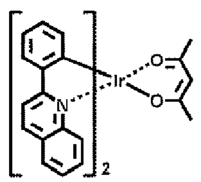

PD11

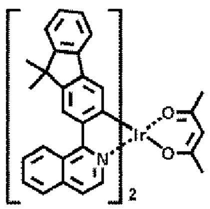

PD12

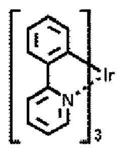

PD13

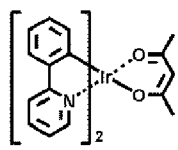

PD14

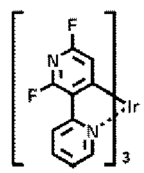

PD15

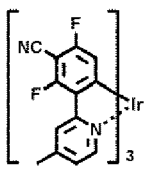

PD16

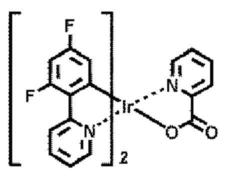

PD17

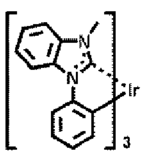

PD18

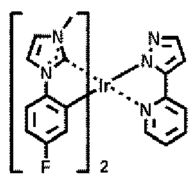

PD19

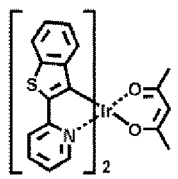

PD20

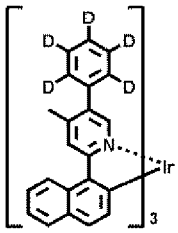
PD21

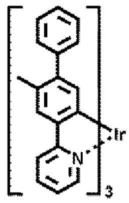
PD22

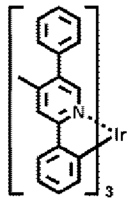
PD23

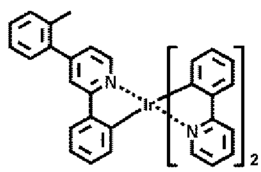
PD24

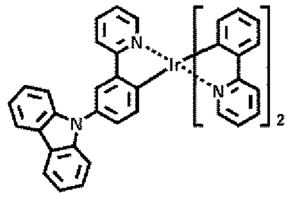
PD25

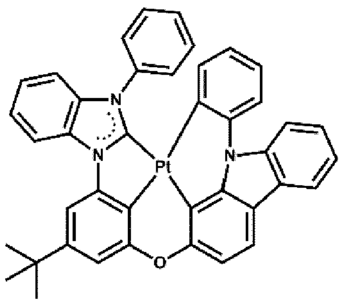
PD26

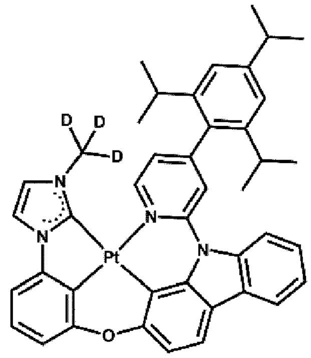
PD27

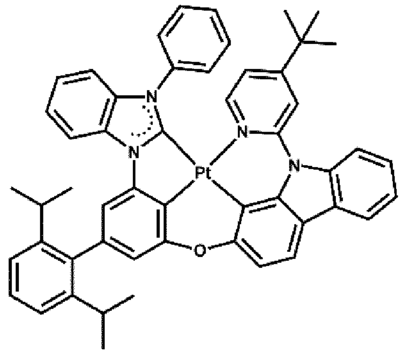
PD28

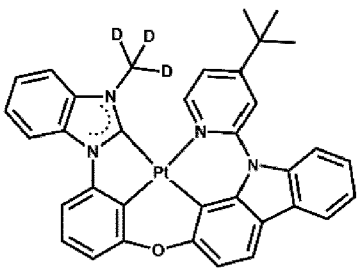
PD29

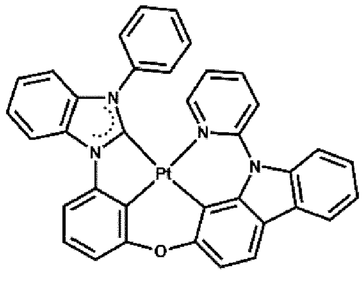
PD30

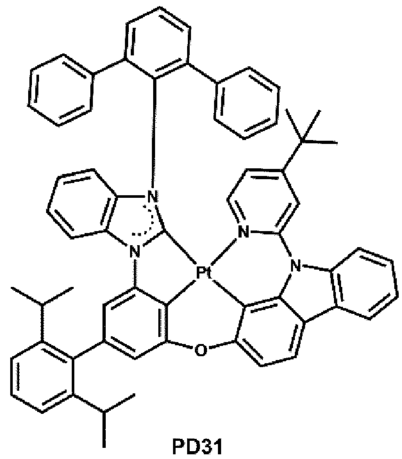
PD31

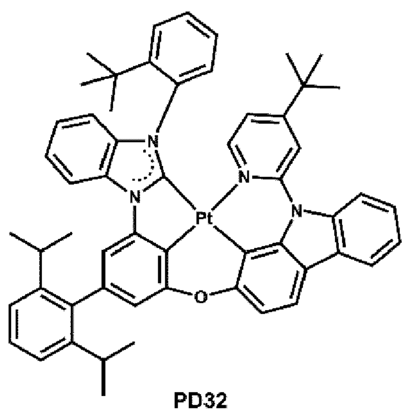
PD32

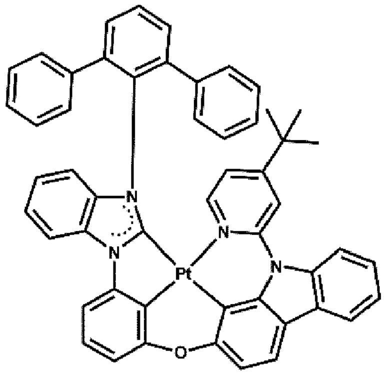
PD33

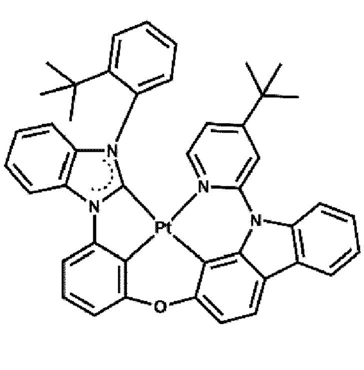
PD34

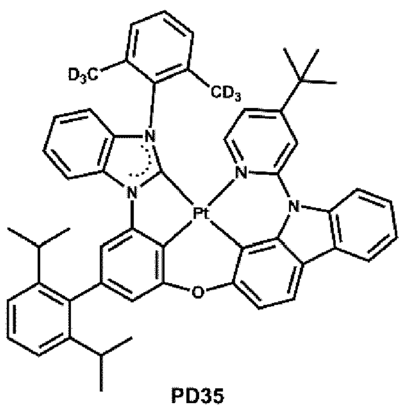

PD35

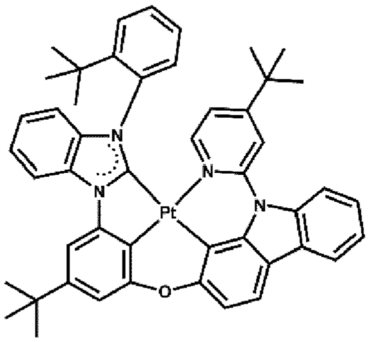

PD36

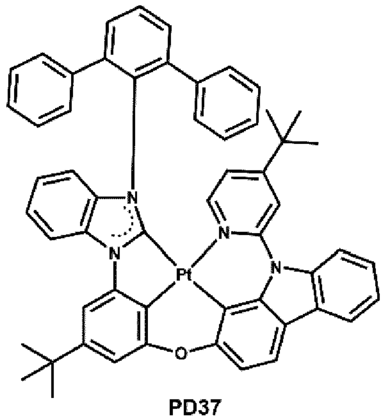

PD37

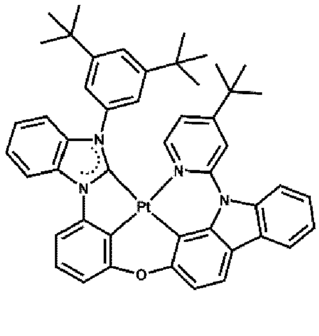

PD38

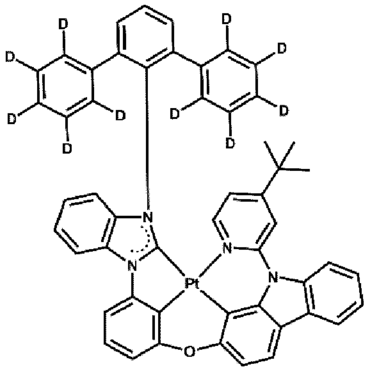

PD39

.

**Fluorescent dopant**

In an embodiment, the fluorescent dopant may include an amine group-containing compound, a styryl group-containing compound, or any combination thereof.

In one or more embodiments, the fluorescent dopant may include a compound represented by Formula 501:

## Formula 501

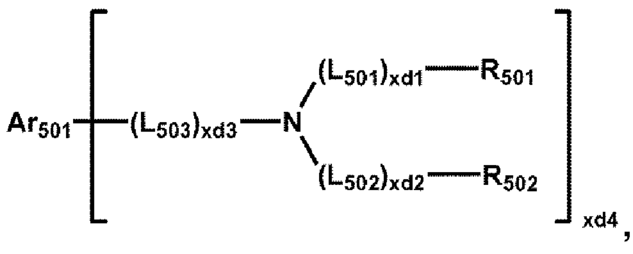

wherein, in Formula 501,

$Ar_{501}$, $L_{501}$ to $L_{503}$, $R_{501}$, and $R_{502}$ may each independently be a $C_3$-$C_{60}$ carbocyclic group unsubstituted or substituted with at least one $R_{10a}$ or a $C_1$-$C_{60}$ heterocyclic group unsubstituted or substituted with at least one $R_{10a}$,
xd1 to xd3 may each independently be 0, 1, 2, or 3, and
xd4 may be 1, 2, 3, 4, 5, or 6.

For example, $Ar_{501}$ in Formula 501 may be a condensed cyclic group (for example, an anthracene group, a chrysene group, or a pyrene group) in which three or more monocyclic groups are condensed together.

For example, xd4 in Formula 501 may be 2.

In one or more embodiments, the fluorescent dopant may include: one of Compounds FD1 to FD36; DPVBi;

DPAVBi; or any combination thereof:

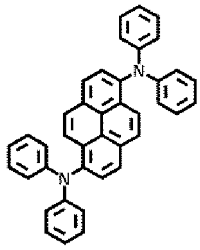

FD1

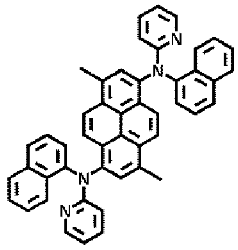

FD2

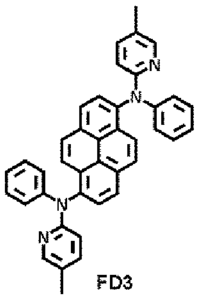

FD3

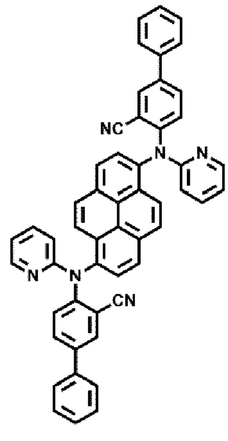

FD4

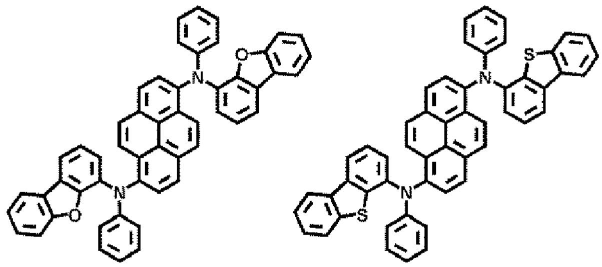

FD5

FD6

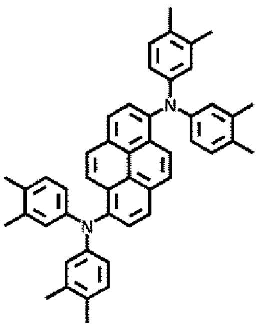

FD7

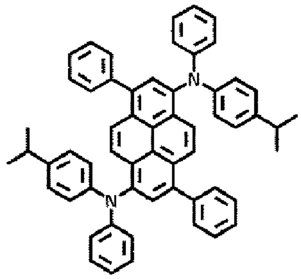

FD8

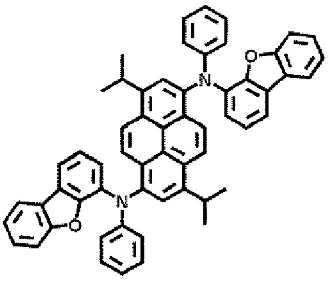

FD9

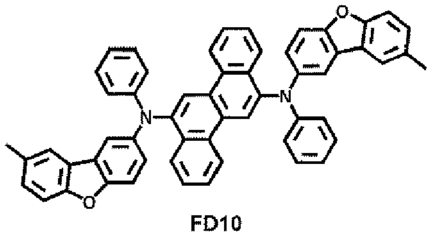

FD10

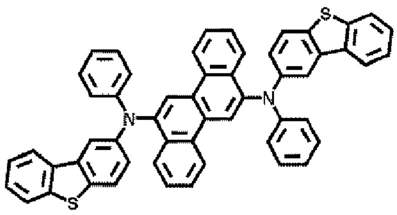

FD11

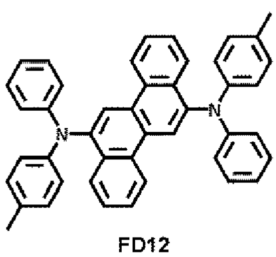

FD12

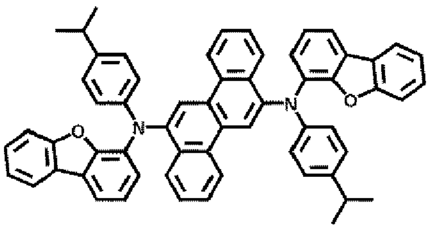

FD13

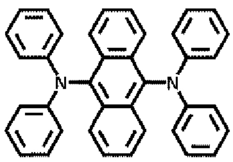

FD14

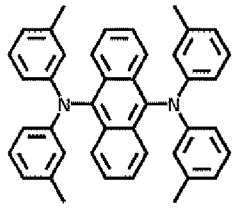

FD15

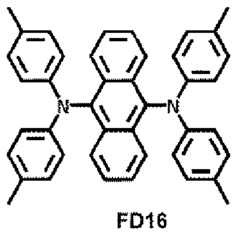

FD16

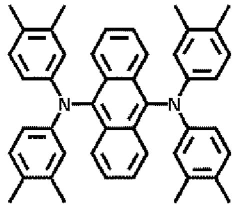

FD17

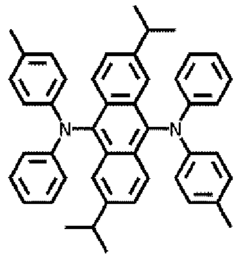

FD18

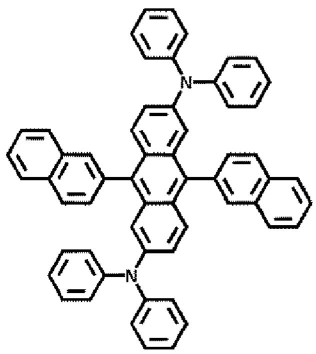

FD19

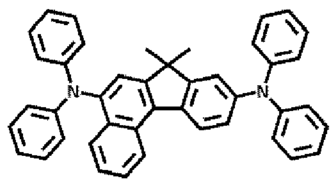

FD20

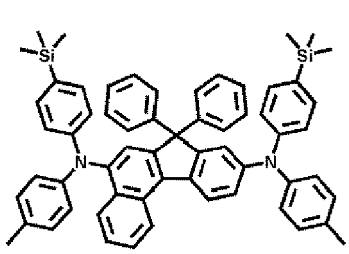

FD21

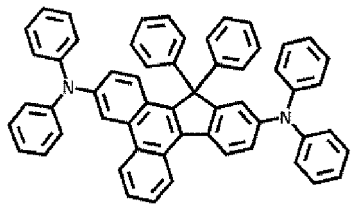

FD22

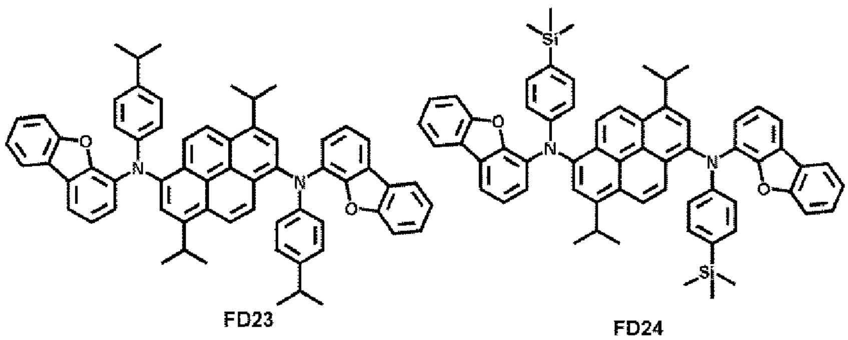

FD23

FD24

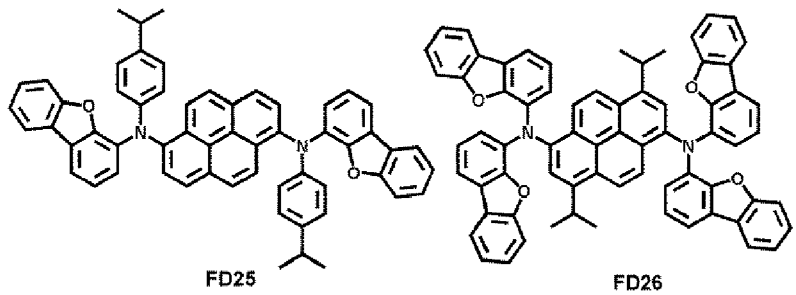

FD25

FD26

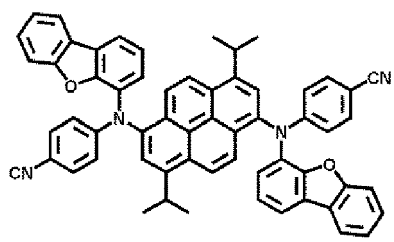

FD27

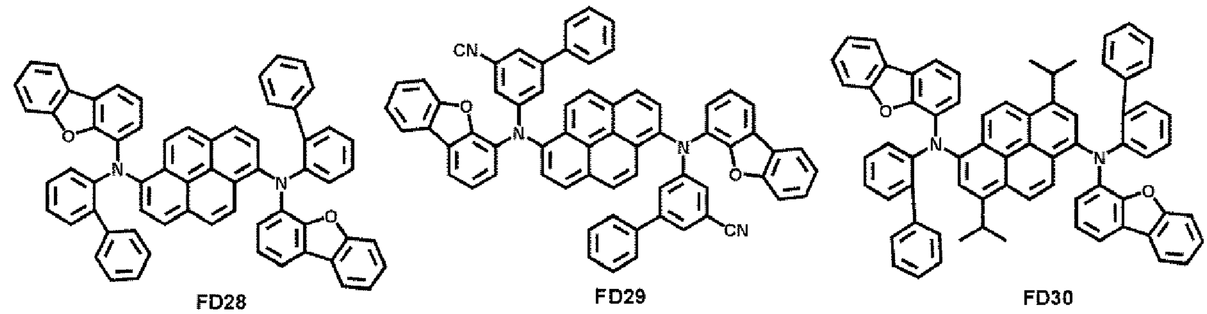

FD28 FD29 FD30

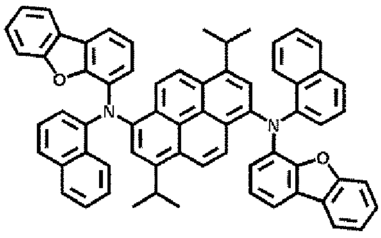

FD31

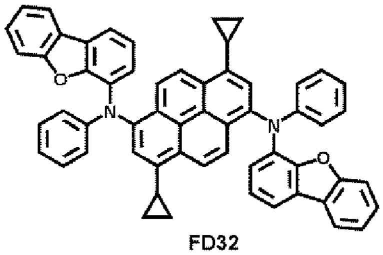

FD32

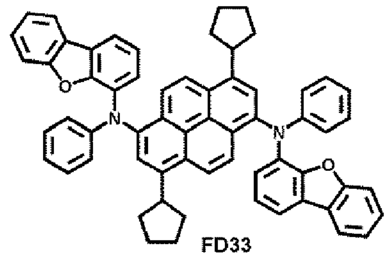

FD33

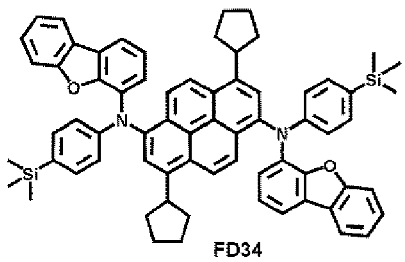

FD34

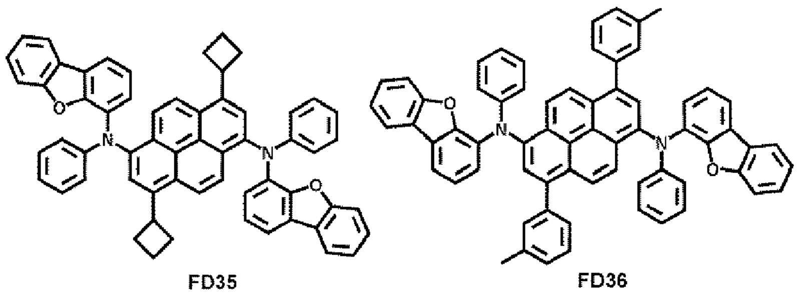

FD35 FD36

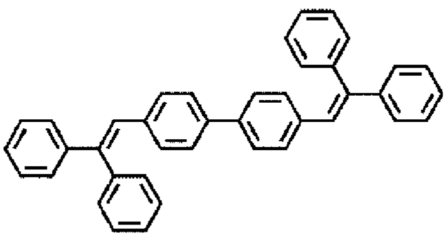

DPVBi

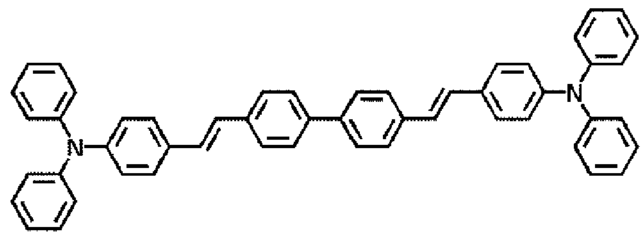

DPAVBi

.

## Delayed fluorescence material

The emission layer may include a delayed fluorescence material.

In the present specification, the delayed fluorescence material may be selected from compounds capable of emitting delayed fluorescence based on a delayed fluorescence emission mechanism.

The delayed fluorescence material included in the emission layer may act as a host or a dopant depending on the type or function of other materials included in the emission layer.

In an embodiment, a difference between a triplet energy level (eV) of the delayed fluorescence material and a singlet energy level (eV) of the delayed fluorescence material may be equal to or greater than 0 eV and equal to or less than 0.5 eV. When the difference between the triplet energy level (eV) of the delayed fluorescence material and the singlet energy level (eV) of the delayed fluorescence material is within these ranges, up-conversion from the triplet state to the singlet state of the delayed fluorescence material may effectively occur, thereby improving luminescence efficiency of the light-emitting device 10.

In an embodiment, the delayed fluorescence material may include: i) a material including at least one electron

donor (for example, a $\pi$ electron-rich $C_3$-$C_{60}$ cyclic group and/or the like, such as a carbazole group) and at least one electron acceptor (for example, a sulfoxide group, a cyano group, a $\pi$ electron-deficient nitrogen-containing $C_1$-$C_{60}$ cyclic group, and/or the like), ii) a material including a $C_8$-$C_{60}$ polycyclic group including at least two cyclic groups condensed to each other while sharing boron (B), and/or the like.

In one or more embodiments, the delayed fluorescence material may include at least one of Compounds DF1 to DF9:

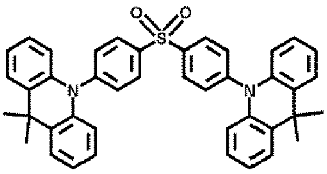
DF1(DMAC-DPS)

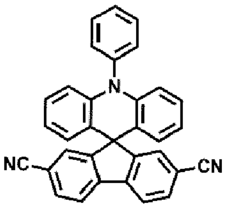
DF2(ACRFLCN)

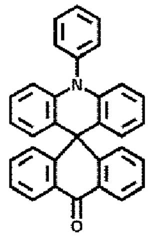
DF3(ACRSA)

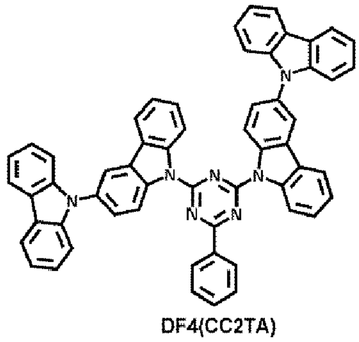
DF4(CC2TA)

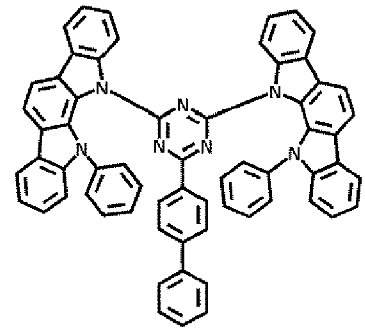
DF5(PIC-TRZ)

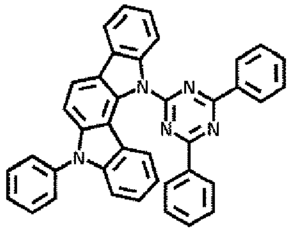
DF6(PIC-TRZ2)

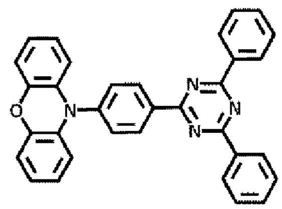
DF7(PXZ-TRZ)

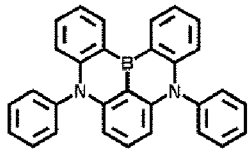
DF8(DABNA-1)

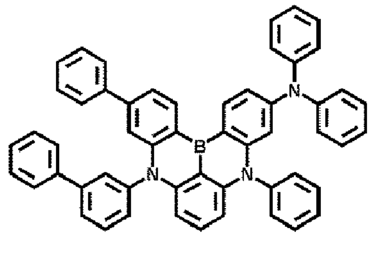
DF9(DABNA-2)

## Quantum dots

The emission layer may include quantum dots.

The term "quantum dot" as used herein refers to a crystal of a semiconductor compound, and may include any material capable of emitting light of one or more suitable emission wavelengths according to the size of the crystal.

A diameter of the quantum dot may be, for example, in a range of about 1 nm to about 10 nm.

The quantum dots may be synthesized by a wet chemical process, a metal organic chemical vapor deposition process, a molecular beam epitaxy process, or any process similar thereto.

The wet chemical process may be a method that includes mixing a precursor material with an organic solvent and then growing a quantum dot particle crystal. When the crystal grows, the organic solvent naturally acts as a dispersant coordinated on the surface of the quantum dot crystal, and thereby controls the growth of the crystal according to a process that costs lower and is easier than vapor deposition methods (such as metal organic chemical vapor deposition (MOCVD) and/or molecular beam epitaxy (MBE)).

The quantum dot may include: a Group II-VI semiconductor compound; a Group III-V semiconductor compound;

a Group III-VI semiconductor compound; a Group I-III-VI semiconductor compound; a Group IV-VI semiconductor compound; a Group IV element or compound; or any combination thereof.

Examples of the Group II-VI semiconductor compound may include (e.g., may be): a binary compound (such as CdSe, CdTe, ZnS, ZnSe, ZnTe, ZnO, HgS, HgSe, HgTe, MgSe, MgS, and/or the like); a ternary compound (such as CdSeS, CdSeTe, CdSTe, ZnSeS, ZnSeTe, ZnSTe, HgSeS, HgSeTe, HgSTe, CdZnS, CdZnSe, CdZnTe, CdHgS, CdHgSe, CdHgTe, HgZnS, HgZnSe, HgZnTe, MgZnSe, MgZnS, and/or the like); a quaternary compound (such as CdZnSeS, CdZnSeTe, CdZnSTe, CdHgSeS, CdHgSeTe, CdHgSTe, HgZnSeS, HgZnSeTe, HgZnSTe, and/or the like); or any combination thereof.

Examples of the Group III-V semiconductor compound may include (e.g., may be): a binary compound (such as GaN, GaP, GaAs, GaSb, AlN, AlP, AlAs, AlSb, InN, InP, InAs, InSb, and/or the like); a ternary compound (such as GaNP, GaNAs, GaNSb, GaPAs, GaPSb, AlNP, AlNAs, AlNSb, AlPAs, AlPSb, InGaP, InNP, InAlP, InNAs, InNSb, InPAs, InPSb, and/or the like); a quaternary compound (such as GaAlNP, GaAlNAs, GaAlNSb, GaAlPAs, GaAlPSb, GaInNP, GaInNAs, GaInNSb, GaInPAs, GaInPSb, InAlNP, InAlNAs, InAlNSb, InAlPAs, InAlPSb, and/or the like); or any combination thereof. The Group III-V semiconductor compound may further include a Group II element. Examples of the Group III-V semiconductor compound further including the Group II element may include (e.g., may be) InZnP, InGaZnP, InAlZnP, and/or the like.

Examples of the Group III-VI semiconductor compound may include (e.g., may be): a binary compound (such as GaS, GaSe, $Ga_2Se_3$, GaTe, InS, InSe, $In_2S_3$, $In_2Se_3$, InTe, and/or the like); a ternary compound (such as $InGaS_3$, $InGaSe_3$, and/or the like); or any combination thereof.

Examples of the Group I-III-VI semiconductor compound may include (e.g., may be): a ternary compound (such as AgInS, $AgInS_2$, CuInS, $CuInS_2$, $CuGaO_2$, $AgGaO_2$, $AgAlO_2$, and/or the like); or any combination thereof.

Examples of the Group IV-VI semiconductor compound may include (e.g., may be): a binary compound (such as SnS, SnSe, SnTe, PbS, PbSe, PbTe, and/or the like); a ternary compound (such as SnSeS, SnSeTe, SnSTe, PbSeS, PbSeTe, PbSTe, SnPbS, SnPbSe, SnPbTe, and/or the like); a quaternary compound (such as SnPbSSe, SnPbSeTe, SnPbSTe, and/or the like); or any combination thereof.

The Group IV element or compound may include (e.g., may be): a single element compound (such as Si, Ge, and/or the like); a binary compound (such as SiC, SiGe, and/or the like); or any combination thereof.

Each element included in a multi-element compound, (such as the binary compound, the ternary compound, and/or the quaternary compound), may exist in a particle thereof at a substantially uniform concentration or non-uniform concentration.

The quantum dot may have a single structure or a dual core-shell structure. In the case of the quantum dot having a single structure, a concentration of each element included in the corresponding quantum dot may be substantially uniform. For example, a material included in the core (e.g., the composition of the core) and a material included in the shell (e.g., the composition of the shell) may be different from each other.

The shell of the quantum dot may act as a protective layer that prevents or reduces chemical degeneration of the core to maintain semiconductor characteristics, and/or as a charging layer that imparts electrophoretic characteristics to the quantum dot. The shell may be a single layer or a multi-layer. The interface between the core and the shell may have a concentration gradient in which the concentration of an element existing in the shell decreases toward the center of the core.

Examples of the shell of the quantum dot may include (e.g., may be) a metal oxide, a metalloid oxide, a non-metal oxide, a semiconductor compound, or any combination thereof. Examples of the metal oxide, metalloid oxide, and/or non-metal oxide may include (e.g., may be): a binary compound (such as $SiO_2$, $Al_2O_3$, $TiO_2$, ZnO, MnO, $Mn_2O_3$, $Mn_3O_4$, CuO, FeO, $Fe_2O_3$, $Fe_3O_4$, CoO, $Co_3O_4$, NiO, and/or the like); a ternary compound (such as $MgAl_2O_4$, $CoFe_2O_4$, $NiFe_2O_4$, $CoMn_2O_4$, and/or the like); or any combination thereof. Examples of the semiconductor compound may include (e.g., may be): as described herein, a Group II-VI semiconductor compound; a Group III-V semiconductor compound; a Group III-VI semiconductor compound; a Group I-III-VI semiconductor compound; a Group IV-VI semiconductor compound; or any combination thereof. Examples of the semiconductor compound may include (e.g., may be) CdS, CdSe, CdTe, ZnS, ZnSe, ZnTe, ZnSeS, ZnTeS, GaAs, GaP, GaSb, HgS, HgSe, HgTe, InAs, InP, InGaP, InSb, AlAs, AlP, AlSb, or any combination thereof.

A full width at half maximum (FWHM) of an emission wavelength spectrum of the quantum dots may be equal to or less than about 45 nm, for example, equal to or less than about 40 nm, and for example, equal to or less than about 30 nm, and within these ranges, color purity and/or color reproducibility may be improved. In some embodiments, because the light emitted through the quantum dots is emitted in all directions, the wide viewing angle may be improved.

In some embodiments, the quantum dot may be, for example, spherical, pyramidal, multi-arm, and/or cubic nanoparticles, nanotubes, nanowires, nanofibers, and/or nanoplate particles.

Because the energy band gap may be adjusted by controlling the size of the quantum dots, light having various suitable wavelength bands may be obtained from the emission layer including the quantum dots. Accordingly, by utilizing the quantum dots of different sizes, a light-emitting device that emits light of various suitable wavelengths may be

implemented. For example, the size of the quantum dots may be selected to emit red light, green light, and/or blue light. In some embodiments, the sizes of the quantum dots may be selected to emit white light by a combination of light of various suitable colors.

**Electron transport region in interlayer 130**

The electron transport region may have i) a single-layered structure including (e.g., consisting of) a single layer including (e.g., consisting of) a single material, ii) a single-layered structure including (e.g., consisting of) a single layer consisting of a plurality of different materials, or iii) a multi-layered structure including a plurality of layers including different materials.

The electron transport region may include a buffer layer, a hole blocking layer, an electron control layer, an electron transport layer, an electron injection layer, or any combination thereof.

For example, the electron transport region may have an electron transport layer/electron injection layer structure, a hole blocking layer/electron transport layer/electron injection layer structure, an electron control layer/electron transport layer/electron injection layer structure, or a buffer layer/electron transport layer/electron injection layer structure, wherein layers of each structure are sequentially stacked from the emission layer.

In an embodiment, the electron transport region (for example, the buffer layer, the hole blocking layer, the electron control layer, or the electron transport layer in the electron transport region) may include a metal-free compound including at least one $\pi$ electron-deficient nitrogen-containing $C_1$-$C_{60}$ cyclic group.

In one or more embodiments, the electron transport region may include a compound represented by Formula 601:

**Formula 601**

$$[Ar_{601}]_{xe11}\text{-}[(L_{601})_{xe1}\text{-}R_{601}]_{xe21},$$

wherein, in Formula 601,

$Ar_{601}$ and $L_{601}$ may each independently be a $C_3$-$C_{60}$ carbocyclic group unsubstituted or substituted with at least one $R_{10a}$ or a $C_1$-$C_{60}$ heterocyclic group unsubstituted or substituted with at least one $R_{10a}$,
xe11 may be 1, 2, or 3,
xe1 may be 0, 1, 2, 3, 4, or 5,
$R_{601}$ may be a $C_3$-$C_{60}$ carbocyclic group unsubstituted or substituted with at least one $R_{10a}$, a $C_1$-$C_{60}$ heterocyclic group unsubstituted or substituted with at least one $R_{10a}$, $-Si(Q_{601})(Q_{602})(Q_{603})$, $-C(=O)(Q_{601})$, $-S(=O)_2(Q_{601})$, or $-P(=O)(Q_{601})(Q_{602})$,
$Q_{601}$ to $Q_{603}$ may each independently be the same as described in connection with $Q_1$,
xe21 may be 1, 2, 3, 4, or 5, and
at least one of $Ar_{601}$, $L_{601}$, and $R_{601}$ may each independently be a $\pi$ electron-deficient nitrogen-containing $C_1$-$C_{60}$ cyclic group unsubstituted or substituted with at least one $R_{10a}$.

In an embodiment, when xe11 in Formula 601 is 2 or more, two or more $Ar_{601}$(s) may be linked to each other via a single bond.

In an embodiment, $Ar_{601}$ in Formula 601 may be a substituted or unsubstituted anthracene group.

In one or more embodiments, the electron transport region may include a compound represented by Formula 601-1:

**Formula 601-1**

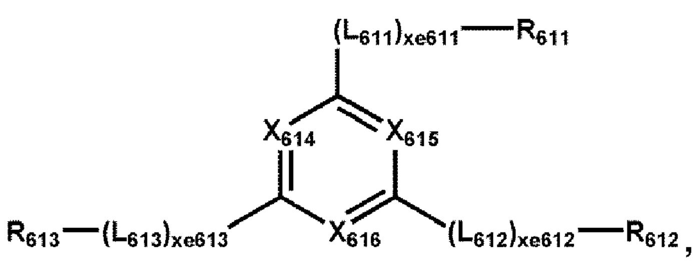

wherein, in Formula 601-1,

X614 may be N or C($R_{614}$), X615 may be N or C($R_{615}$), X616 may be N or C($R_{616}$), and at least one of $X_{614}$ to X616 may be N,
$L_{611}$ to $L_{613}$ may each independently be the same as described in connection with $L_{601}$,
xe611 to xe613 may each independently be the same as described in connection with xe1,
$R_{611}$ to $R_{613}$ may each independently be the same as described in connection with $R_{601}$, and
$R_{614}$ to $R_{616}$ may each independently be hydrogen, deuterium, -F, -Cl, -Br, -I, a hydroxyl group, a cyano group, a nitro group, a $C_1$-$C_{20}$ alkyl group, a $C_1$-$C_{20}$ alkoxy group, a $C_3$-$C_{60}$ carbocyclic group unsubstituted or substituted with at least one $R_{10a}$, or a $C_1$-$C_{60}$ heterocyclic group unsubstituted or substituted with at least one Rioa.

For example, xe1 and xe611 to xe613 in Formulae 601 and 601-1 may each independently be 0, 1, or 2.
In one or more embodiments, the electron transport region may include one of Compounds ET1 to ET45, 2,9-dimethyl-4,7-diphenyl-1,10-phenanthroline (BCP), 4,7-diphenyl-1,10-phenanthroline (Bphen), $Alq_3$, BAlq, TAZ, NTAZ, or any combination thereof:

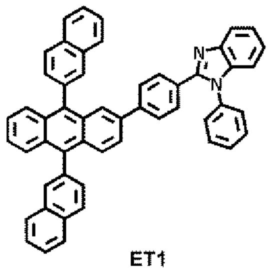
ET1

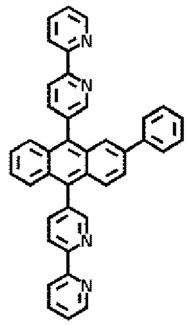
ET2

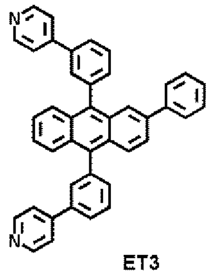
ET3

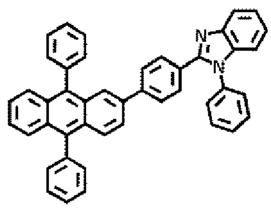
ET4

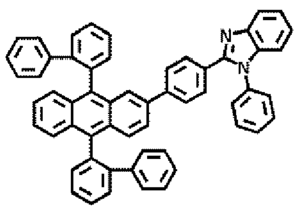
ET5

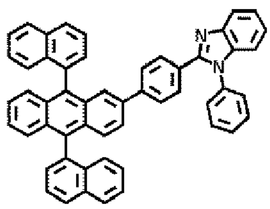
ET6

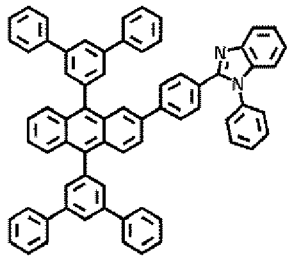
ET7

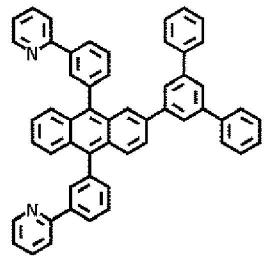
ET8

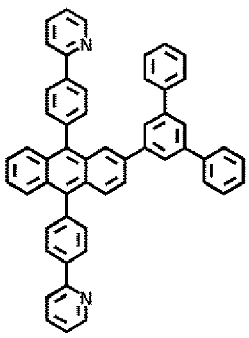
ET9

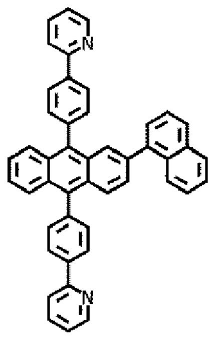
ET10

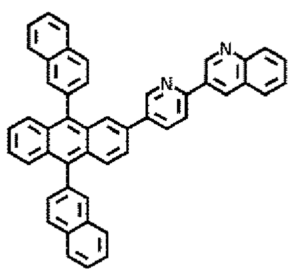
ET11

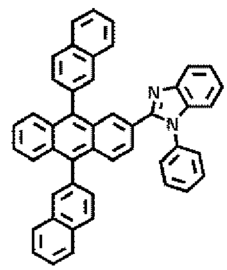
ET12

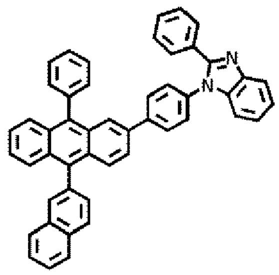
ET13

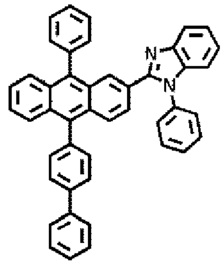
ET14

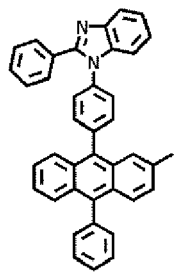
ET15

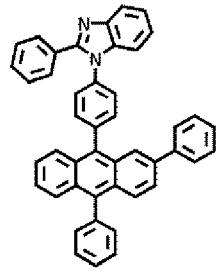
ET16

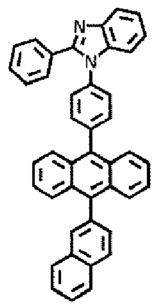
ET17

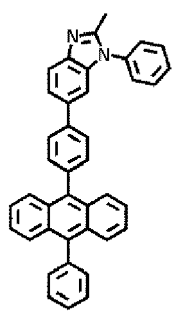
ET18

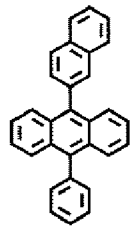
ET19

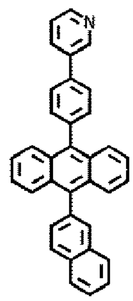
ET20

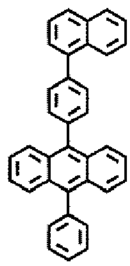
ET21

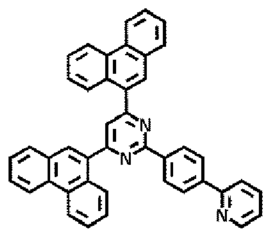
ET22

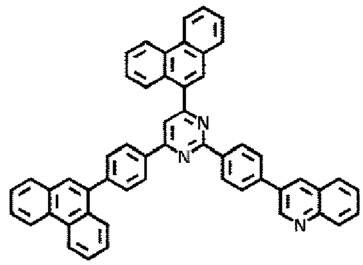
ET23

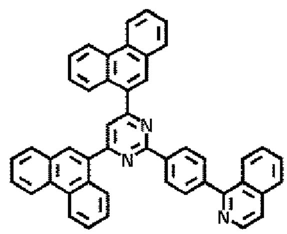
ET24

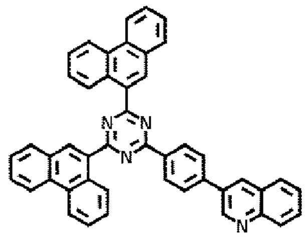
ET25

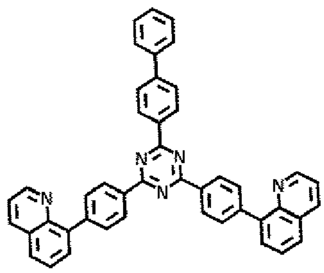
ET26

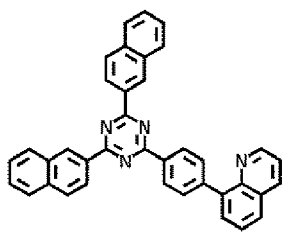
ET27

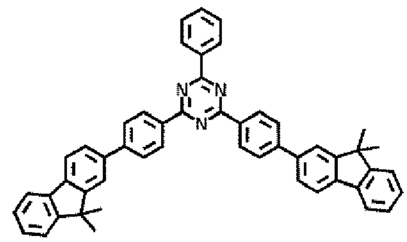
ET28

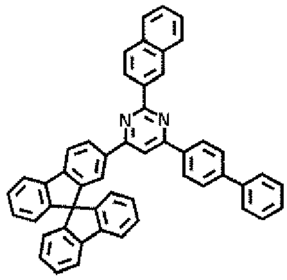
ET29

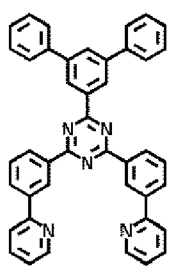
ET30

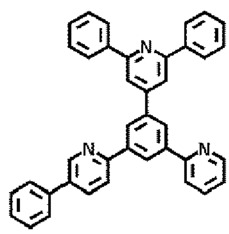
ET31

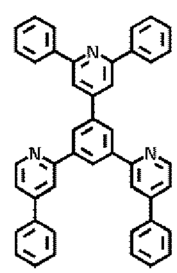
ET32

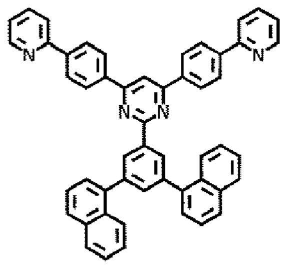
ET33

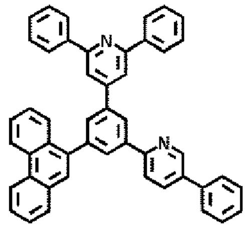
ET34

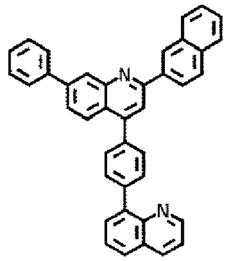
ET35

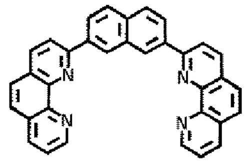
ET36

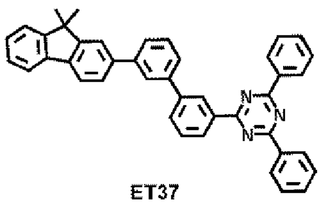
ET37

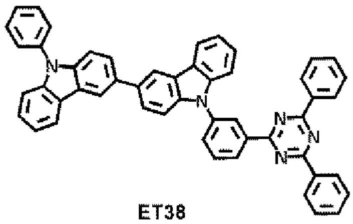
ET38

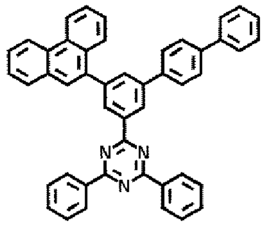
ET39

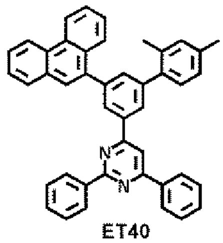
ET40

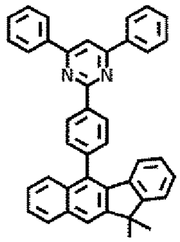
ET41

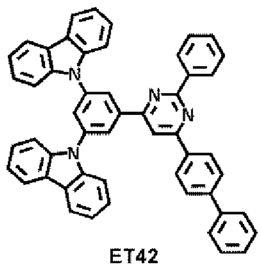
ET42

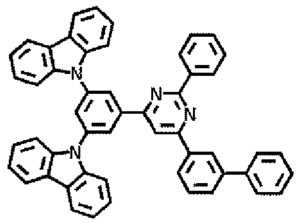
ET43

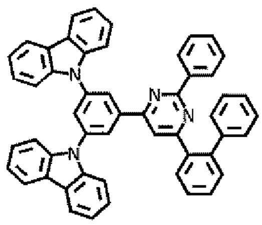
ET44

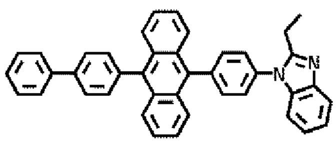
ET45

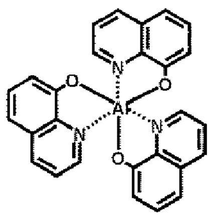
Alq3

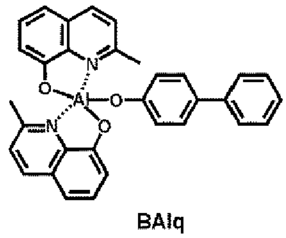
BAlq

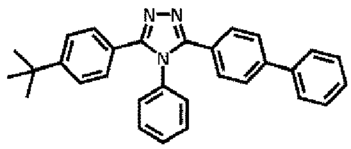
TAZ

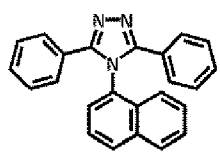
NTAZ .

A thickness of the electron transport region may be in a range of about 100 Å to about 5,000 Å, for example, about 160 Å to about 4,000 Å. When the electron transport region includes a buffer layer, a hole blocking layer, an electron control layer, an electron transport layer, or any combination thereof, a thickness of the buffer layer, the hole blocking layer, or the electron control layer may each independently be in a range of about 20 Å to about 1,000 Å, for example, about 30 Å to about 300 Å, and a thickness of the electron transport layer may be in a range of about 100 Å to about 1,000 Å, for example, about 150 Å to about 500 Å. When the thicknesses of the buffer layer, the hole blocking layer, the electron control layer, the electron transport layer, and/or the electron transport region are within these ranges, satisfactory electron transporting characteristics may be obtained without a substantial increase in driving voltage.

The electron transport region (for example, the electron transport layer in the electron transport region) may further include, in addition to the materials described above, a metal-containing material.

The metal-containing material may include an alkali metal complex, an alkaline earth metal complex, or any

combination thereof. The metal ion of the alkali metal complex may be a Li ion, a Na ion, a K ion, a Rb ion, or a Cs ion, and the metal ion of the alkaline earth metal complex may be a Be ion, a Mg ion, a Ca ion, a Sr ion, or a Ba ion. A ligand coordinated with the metal ion of the alkali metal complex or the alkaline earth-metal complex may include a hydroxyquinoline, a hydroxyisoquinoline, a hydroxybenzoquinoline, a hydroxyacridine, a hydroxyphenanthridine, a hydroxyphenyloxazole, a hydroxyphenylthiazole, a hydroxyphenyloxadiazole, a hydroxyphenylthiadiazole, a hydroxyphenylpyridine, a hydroxyphenylbenzimidazole, a hydroxyphenylbenzothiazole, a bipyridine, a phenanthroline, a cyclopentadiene, or any combination thereof.

For example, the metal-containing material may include a Li complex. The Li complex may include, for example, Compound ET-D1 (LiQ) and/or ET-D2:

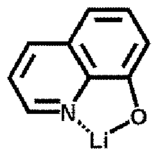

ET-D1

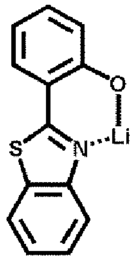

ET-D2 .

The electron transport region may include an electron injection layer to facilitate the injection of electrons from the second electrode 150. The electron injection layer may directly contact the second electrode 150.

The electron injection layer may have: i) a single-layered structure including (e.g., consisting of) a single layer including (e.g., consisting of) a single material, ii) a single-layered structure including (e.g., consisting of) a single layer including (e.g., consisting of) a plurality of different materials, or iii) a multi-layered structure including a plurality of layers including different materials.

The electron injection layer may include an alkali metal, an alkaline earth metal, a rare earth metal, an alkali metal-containing compound, an alkaline earth metal-containing compound, a rare earth metal-containing compound, an alkali metal complex, an alkaline earth metal complex, a rare earth metal complex, or any combination thereof.

The alkali metal may include lithium (Li), sodium (Na), potassium (K), rubidium (Rb), cesium (Cs), or any combination thereof. The alkaline earth metal may include magnesium (Mg), calcium (Ca), strontium (Sr), barium (Ba), or any combination thereof. The rare earth metal may include scandium (Sc), yttrium (Y), cerium (Ce), terbium (Tb), ytterbium (Yb), gadolinium (Gd), or any combination thereof.

The alkali metal-containing compound, the alkaline earth metal-containing compound, and the rare earth metal-containing compound may respectively be oxides, halides (for example, fluorides, chlorides, bromides, iodides, and/or the like), and/or tellurides of the alkali metal, the alkaline earth metal, and the rare earth metal, or any combination thereof.

The alkali metal-containing compound may include alkali metal oxides (such as $Li_2O$, $Cs_2O$, $K_2O$, and/or the like), alkali metal halides (such as LiF, NaF, CsF, KF, LiI, NaI, CsI, KI, and/or the like), or any combination thereof. The alkaline earth metal-containing compound may include an alkaline earth metal compound (such as BaO, SrO, CaO, $Ba_xSr_{1-x}O$ (wherein x is a real number satisfying the condition of 0<x<1), $Ba_xCa_{1-x}O$ (wherein x is a real number satisfying the condition of 0<x<1), and/or the like). The rare earth metal-containing compound may include $YbF_3$, $ScF_3$, $Sc_2O_3$, $Y_2O_3$, $Ce_2O_3$, $GdF_3$, $TbF_3$, $YbI_3$, $ScI_3$, $TbI_3$, or any combination thereof. For example, the rare earth metal-containing compound may include a lanthanide metal telluride. Examples of the lanthanide metal telluride may include LaTe, CeTe, PrTe, NdTe, PmTe, SmTe, EuTe, GdTe, TbTe, DyTe, HoTe, ErTe, TmTe, YbTe, LuTe, $La_2Te_3$, $Ce_2Te_3$, $Pr_2Te_3$, Nd2Te3, Pm2Te3, Sm2Te3, $Eu_2Te_3$, Gd2Te3, Tb2Te3, Dy2Te3, $Ho_2Te_3$, Er2Te3, Tm2Te3, Yb2Te3, $Lu_2Te_3$, and/or the like.

The alkali metal complex, the alkaline earth-metal complex, and the rare earth metal complex may include i) an ion of the alkali metal, the alkaline earth metal, and the rare earth metal, respectively, and ii), as a ligand bonded to the metal ion, for example, hydroxyquinoline, hydroxyisoquinoline, hydroxybenzoquinoline, hydroxyacridine, hydroxyphenanthridine, hydroxyphenyloxazole, hydroxyphenylthiazole, hydroxyphenyloxadiazole, hydroxyphenylthiadiazole, hydroxyphenylpyridine, hydroxyphenyl benzimidazole, hydroxyphenylbenzothiazole, bipyridine, phenanthroline, cyclopentadiene, or any combination thereof.

In an embodiment, the electron injection layer may include (e.g., consist of) an alkali metal, an alkaline earth metal, a rare earth metal, an alkali metal-containing compound, an alkaline earth metal-containing compound, a rare earth metal-containing compound, an alkali metal complex, an alkaline earth metal complex, a rare earth metal complex, or any combination thereof, as described above. In one or more embodiments, the electron injection layer may further include an organic material (for example, the compound represented by Formula 601).

In one or more embodiments, the electron injection layer may include (e.g., consist of): i) an alkali metal-containing compound (for example, an alkali metal halide); or ii) a) an alkali metal-containing compound (for example, an alkali metal halide), and b) an alkali metal, an alkaline earth metal, a rare earth metal, or any combination thereof. In one or more embodiments, the electron injection layer may be a KI:Yb co-deposited layer, an RbI:Yb co-deposited layer, and/or the like.

When the electron injection layer further includes an organic material, the alkali metal, alkaline earth metal, rare earth metal, an alkali metal-containing compound, an alkaline earth metal-containing compound, a rare earth metal-containing compound, an alkali metal complex, an alkaline earth-metal complex, a rare earth metal complex, or any combination thereof may be substantially homogeneously or non-homogeneously dispersed in a matrix including the organic material.

A thickness of the electron injection layer may be in a range of about 1 Å to about 100 Å, for example, about 3 Å to about 90 Å. When the thickness of the electron injection layer is within these ranges, satisfactory electron injection characteristics may be obtained without a substantial increase in driving voltage.

**Second electrode 150**

The second electrode 150 may be on the interlayer 130 having a structure as described above. The second electrode 150 may be a cathode, which is an electron injection electrode, and a material for forming the second electrode 150 may include a metal, an alloy, an electrically conductive compound, or any combination thereof, each having a low-work function.

The material for forming the second electrode 150 may include lithium (Li), silver (Ag), magnesium (Mg), aluminum (Al), aluminum-lithium (Al-Li), calcium (Ca), magnesium-indium (Mg-In), magnesium-silver (Mg-Ag), ytterbium (Yb), silver-ytterbium (Ag-Yb), ITO, IZO, or any combination thereof. The second electrode 150 may be a transmissive electrode, a semi-transmissive electrode, or a reflective electrode.

The second electrode 150 may have a single-layered structure or a multi-layered structure including a plurality of layers.

**Capping layer**

A first capping layer may be arranged outside the first electrode 110, and/or a second capping layer may be arranged outside the second electrode 150. In particular, the light-emitting device 10 may have a structure in which the first capping layer, the first electrode 110, the interlayer 130, and the second electrode 150 are sequentially stacked in the stated order, a structure in which the first electrode 110, the interlayer 130, the second electrode 150, and the second capping layer are sequentially stacked in the stated order, or a structure in which the first capping layer, the first electrode 110, the interlayer 130, the second electrode 150, and the second capping layer are sequentially stacked in the stated order.

Light generated in the emission layer of the interlayer 130 of the light-emitting device 10 may be extracted toward the outside through the first electrode 110 (which is a semi-transmissive electrode or a transmissive electrode) and the first capping layer, and light generated in the emission layer of the interlayer 130 of the light-emitting device 10 may be extracted toward the outside through the second electrode 150 (which is a semi-transmissive electrode or a transmissive electrode) and the second capping layer.

The first capping layer and the second capping layer may increase the external luminescence efficiency of the device according to the principle of constructive interference. Accordingly, the light extraction efficiency of the light-emitting device 10 may be increased, so that the luminescence efficiency of the light-emitting device 10 may be improved.

Each of the first capping layer and the second capping layer may include a material having a refractive index of equal to or greater than 1.6 (at 589 nm).

The first capping layer and the second capping layer may each independently be an organic capping layer including an organic material, an inorganic capping layer including an inorganic material, or an organic-inorganic composite capping layer including an organic material and an inorganic material.

In an embodiment, at least one selected from the first capping layer and the second capping layer may each independently include a carbocyclic compound, a heterocyclic compound, an amine group-containing compound, a porphine derivative, a phthalocyanine derivative, a naphthalocyanine derivative, an alkali metal complex, an alkaline earth metal complex, or any combination thereof. The carbocyclic compound, the heterocyclic compound, and the amine group-containing compound may each optionally be substituted with a substituent including O, N, S, Se, Si, F, Cl, Br, I, or any combination thereof. In one or more embodiments, at least one of the first capping layer and the second capping layer may each independently include an amine group-containing compound.

For example, at least one selected from the first capping layer and the second capping layer may each independently include a compound represented by Formula 201, a compound represented by Formula 202, or any combination thereof.

In one or more embodiments, at least one selected from the first capping layer and the second capping layer may each independently include one of Compounds HT28 to HT33, one of Compounds CP1 to CP6, β-NPB, or any combination thereof:

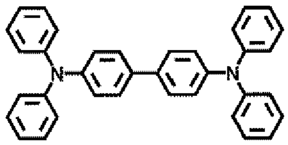
CP1

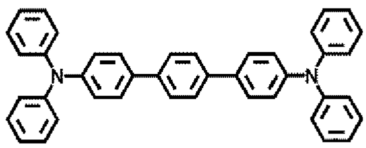
CP2

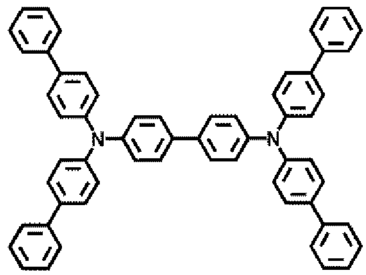
CP3

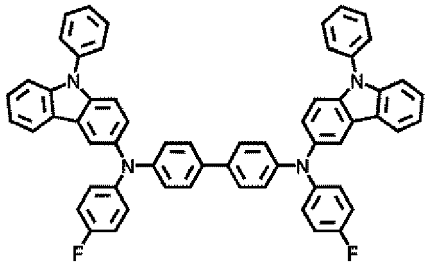
CP4

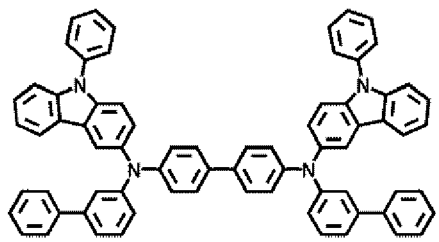
CP5

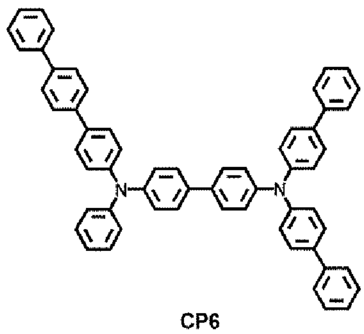
CP6

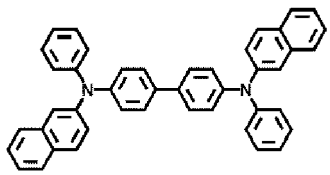
β-NPB .

## Film

The condensed cyclic compound represented by Formula 1 may be included in one or more suitable films. One or more embodiments of the present disclosure provide a film including the condensed cyclic compound represented by Formula 1. The film may be, for example, an optical member (or a light control component) (for example, a color filter, a color conversion member, a capping layer, a light extraction efficiency enhancement layer, a selective light absorbing layer, a polarizing layer, a quantum dot-containing layer, and/or the like), a light-blocking member (for example, a light reflective layer, a light absorbing layer, and/or the like), or a protective member (for example, an insulating layer, a dielectric layer, and/or the like).

## Electronic apparatus

The light-emitting device may be included in various suitable electronic apparatuses. For example, the electronic apparatus including the light-emitting device may be a light-emitting apparatus, an authentication apparatus, and/or the like.

The electronic apparatus (for example, a light-emitting apparatus) may further include, in addition to the light-emitting device, i) a color filter, ii) a color conversion layer, or iii) both (e.g., simultaneously) a color filter and a color conversion layer. The color filter and/or the color conversion layer may be arranged in at least one traveling direction of

light emitted from the light-emitting device. For example, the light emitted from the light-emitting device may be blue light or white light. The light-emitting device may be the same as described above. In an embodiment, the color conversion layer may include quantum dots. The quantum dots may be, for example, the same as described herein.

The electronic apparatus may include a first substrate. The first substrate may include a plurality of subpixel areas, the color filter may include a plurality of color filter areas respectively corresponding to the subpixel areas, and the color conversion layer may include a plurality of color conversion areas respectively corresponding to the subpixel areas.

A pixel-defining film may be arranged among the subpixel areas to define each of the subpixel areas.

The color filter including the plurality of the color filter areas may further include light-shielding patterns interposed between the color filter areas, and the color conversion layer including the plurality of the color conversion areas may further include light-shielding patterns interposed between the color conversion areas.

The plurality of color filter areas (or the plurality of color conversion areas) may include a first area to emit first color light, a second area to emit second color light, and/or a third area to emit third color light, wherein the first color light, the second color light, and/or the third color light may have different maximum emission wavelengths from one another. For example, the first color light may be red light, the second color light may be green light, and the third color light may be blue light. For example, the color filter areas (and/or the color conversion areas) may each include quantum dots. For example, the first area may include red quantum dots, the second area may include green quantum dots, and the third area may not include (e.g., may exclude) quantum dots. The quantum dots may be the same as described herein. Each of the first region, the second region, and/or the third region may further include a scatterer.

In an embodiment, the light-emitting device may be to emit first light, the first area may be to absorb the first light to emit first first-color light, the second area may be to absorb the first light to emit second first-color light, and the third area may be to absorb the first light to emit third first-color light. In this regard, the first first-color light, the second first-color light, and the third first-color light may have different maximum emission wavelengths from one another. In particular, the first light may be blue light, the first-first color light may be red light, the second-first color light may be green light, and the third-first color light may be blue light.

The electronic apparatus may further include a thin-film transistor, in addition to the light-emitting device described above. The thin-film transistor may include a source electrode, a drain electrode, and an activation layer, wherein any one of the source electrode and the drain electrode may be electrically connected to any one of the first electrode and the second electrode of the light-emitting device.

The thin-film transistor may further include a gate electrode, a gate insulating film, and/or the like.

The activation layer may include crystalline silicon, amorphous silicon, an organic semiconductor, an oxide semiconductor, and/or the like.

The electronic apparatus may further include a sealing portion for sealing the light-emitting device. The sealing portion and/or the color conversion layer may be arranged between the color filter and the light-emitting device. The sealing portion allows light from the light-emitting device to be extracted to the outside, while concurrently (e.g., simultaneously) preventing or reducing ambient air and/or moisture from penetrating into the light-emitting device. The sealing portion may be a sealing substrate including a transparent glass substrate and/or a plastic substrate. The sealing portion may be a thin-film encapsulation layer including at least one layer of an organic layer and/or an inorganic layer. When sealing portion is a thin-film encapsulating layer, the electronic apparatus may be flexible.

Various functional layers may be additionally arranged on the sealing portion, in addition to the color filter and/or the color conversion layer, according to the intended use of the electronic apparatus. Examples of the functional layers may include (e.g., may be) a touch screen layer, a polarizing layer, and/or the like. The touch screen layer may be a pressure-sensitive touch screen layer, a capacitive touch screen layer, or an infrared touch screen layer. The authentication apparatus may be, for example, a biometric authentication apparatus that authenticates an individual by utilizing biometric information of a living body (for example, fingertips, pupils, and/or the like).

The authentication apparatus may further include, in addition to the light-emitting device as described above, a biometric information collector.

The electronic apparatus may be applied to various suitable displays, light sources, lighting, personal computers (for example, a mobile personal computer), mobile phones, digital cameras, electronic organizers, electronic dictionaries, electronic game machines, medical instruments (for example, electronic thermometers, sphygmomanometers, blood glucose meters, pulse measurement devices, pulse wave measurement devices, electrocardiogram displays, ultrasonic diagnostic devices, or endoscope displays), fish finders, various suitable measuring instruments, meters (for example, meters for a vehicle, an aircraft, and a vessel), projectors, and/or the like.

## Description of FIGS. 2 and 3

FIG. 2 is a cross-sectional view showing a light-emitting apparatus according to an embodiment of the present disclosure.

The light-emitting apparatus of FIG. 2 includes a substrate 100, a thin-film transistor (TFT), a light-emitting device, and an encapsulation portion 300 that seals the light-emitting device.

The substrate 100 may be a flexible substrate, a glass substrate, and/or a metal substrate. A buffer layer 210 may be arranged on the substrate 100. The buffer layer 210 may prevent or reduce penetration of impurities through the substrate 100, and may provide a flat surface on the substrate 100.

The TFT may be arranged on the buffer layer 210. The TFT may include an activation layer 220, a gate electrode 240, a source electrode 260, and a drain electrode 270.

The activation layer 220 may include an inorganic semiconductor, (such as silicon and/or polysilicon), an organic semiconductor, and/or an oxide semiconductor, and may include a source region, a drain region, and a channel region.

A gate insulating film 230 for insulating the activation layer 220 from the gate electrode 240 may be arranged on the activation layer 220, and the gate electrode 240 may be arranged on the gate insulating film 230.

An interlayer insulating film 250 may be arranged on the gate electrode 240. The interlayer insulating film 250 may be arranged between the gate electrode 240 and the source electrode 260 and between the gate electrode 240 and the drain electrode 270, so as to provide insulation therebetween.

The source electrode 260 and the drain electrode 270 may be arranged on the interlayer insulating film 250. The interlayer insulating film 250 and the gate insulating film 230 may be formed to expose the source region and the drain region of the activation layer 220, and the source electrode 260 and the drain electrode 270 may be arranged in contact with the exposed portions of the source region and the drain region of the activation layer 220.

The TFT may be electrically connected to the light-emitting device to drive the light-emitting device, and may be covered by a passivation layer 280. The passivation layer 280 may include an inorganic insulating film, an organic insulating film, or any combination thereof. The light-emitting device may be provided on the passivation layer 280. The light-emitting device may include the first electrode 110, the interlayer 130, and the second electrode 150.

The first electrode 110 may be arranged on the passivation layer 280. The passivation layer 280 may expose a portion of the drain electrode 270 without completely covering the drain electrode 270, and the first electrode 110 may be arranged to be connected to the exposed portion of the drain electrode 270.

A pixel defining layer 290 including an insulating material may be arranged on the first electrode 110. The pixel defining layer 290 may expose a region of the first electrode 110, and the interlayer 130 may be formed in the exposed region of the first electrode 110. The pixel defining layer 290 may be a polyimide organic film and/or a polyacrylic organic film. In some embodiments, at least some layers of the interlayer 130 may extend beyond the upper portion of the pixel defining layer 290 to be arranged in the form of a common layer.

The second electrode 150 may be arranged on the interlayer 130, and a capping layer 170 may be additionally formed on the second electrode 150. The capping layer 170 may be formed to cover the second electrode 150.

The encapsulation portion 300 may be arranged on the capping layer 170. The encapsulation portion 300 may be arranged on the light-emitting device to protect the light-emitting device from moisture and/or oxygen. The encapsulation portion 300 may include: an inorganic film including silicon nitride ($SiN_x$), silicon oxide ($SiO_x$), indium tin oxide, indium zinc oxide, or any combination thereof; an organic film including polyethylene terephthalate, polyethylene naphthalate, polycarbonate, polyimide, polyethylene sulfonate, polyoxymethylene, polyarylate, hexamethyldisiloxane, an acrylic resin (for example, polymethyl methacrylate, polyacrylic acid, and/or the like), an epoxy-based resin (for example, aliphatic glycidyl ether (AGE) and/or the like), or any combination thereof; or any combination of the inorganic film and the organic film.

FIG. 3 shows a cross-sectional view showing a light-emitting apparatus according to another embodiment of the present disclosure.

The light-emitting apparatus of FIG. 3 is the same as the light-emitting apparatus of FIG. 2, except that a light-shielding pattern 500 and a functional region 400 are additionally arranged on the encapsulation portion 300. The functional region 400 may include i) a color filter area, ii) a color conversion area, or iii) a combination of a color filter area and a color conversion area. In an embodiment, a light-emitting device included in the light-emitting apparatus of FIG. 3 may be a tandem light-emitting device.

**Manufacturing method**

The respective layers included in the hole transport region, the emission layer, and the respective layers included in the electron transport region may be formed in a set or predetermined region by utilizing one or more suitable methods selected from vacuum deposition, spin coating, casting, Langmuir-Blodgett (LB) deposition, ink-jet printing, laser-printing, laser-induced thermal imaging, and/or the like.

When the respective layers included in the hole transport region, the emission layer, and the respective layers included in the electron transport region are formed by vacuum deposition, the deposition conditions may include a deposition temperature in a range of about 100 °C to about 500 °C, a vacuum degree of about $10^{-8}$ torr to about $10^{-3}$ torr, and a deposition speed in a range of about 0.01 Å/sec to about 100 Å/sec, depending on a material to be included

in a layer to be formed and the structure of a layer to be formed.

**Definition of terms**

The term "$C_3$-$C_{60}$ carbocyclic group" as utilized herein refers to a cyclic group consisting of carbon only as a ring-forming atom and having three to sixty carbon atoms, and the term "$C_1$-$C_{60}$ heterocyclic group" as utilized herein refers to a cyclic group that has one to sixty carbon atoms and further has, in addition to carbon, a heteroatom as a ring-forming atom. Each of the $C_3$-$C_{60}$ carbocyclic group and the $C_1$-$C_{60}$ heterocyclic group may be a monocyclic group consisting of one ring or a polycyclic group consisting of two or more rings that are condensed together. For example, the $C_1$-$C_{60}$ heterocyclic group may have 3 to 61 ring-forming atoms.

The term "cyclic group" as utilized herein may include both (e.g., simultaneously) the $C_3$-$C_{60}$ carbocyclic group and the $C_1$-$C_{60}$ heterocyclic group.

The term "$\pi$ electron-rich $C_3$-$C_{60}$ cyclic group" as utilized herein refers to a cyclic group that has 3 to 60 carbon atoms and does not include *-N=*' as a ring-forming moiety, and the term "$\pi$ electron-deficient nitrogen-containing $C_1$-$C_{60}$ cyclic group" as utilized herein refers to a heterocyclic group that has 1 to 60 carbon atoms and includes *-N=*' as a ring-forming moiety.

For example,

the $C_3$-$C_{60}$ carbocyclic group may be i) a T1 group (defined below) or ii) a condensed cyclic group in which at least two T1 groups are condensed with each other (for example, a cyclopentadiene group, an adamantane group, a norbornane group, a benzene group, a pentalene group, a naphthalene group, an azulene group, an indacene group, an acenaphthylene group, a phenalene group, a phenanthrene group, an anthracene group, a fluoranthene group, a triphenylene group, a pyrene group, a chrysene group, a perylene group, a pentaphene group, a heptalene group, a naphthacene group, a picene group, a hexacene group, a pentacene group, a rubicene group, a coronene group, an ovalene group, an indene group, a fluorene group, a spiro-bifluorene group, a benzofluorene group, an indenophenanthrene group, or an indenoanthracene group),

the $C_1$-$C_{60}$ heterocyclic group may be i) a T2 group (defined below), ii) a condensed cyclic group in which at least two T2 groups are condensed with each other, or iii) a condensed cyclic group in which at least one T2 group and at least one T1 group are condensed with each other (for example, a pyrrole group, a thiophene group, a furan group, an indole group, a benzoindole group, a naphthoindole group, an isoindole group, a benzoisoindole group, a naphthoisoindole group, a benzosilole group, a benzothiophene group, a benzofuran group, a carbazole group, a dibenzosilole group, a dibenzothiophene group, a dibenzofuran group, an indenocarbazole group, an indolocarbazole group, a benzofurocarbazole group, a benzothienocarbazole group, a benzosilolocarbazole group, a benzoindolocarbazole group, a benzocarbazole group, a benzonaphthofuran group, a benzonaphthothiophene group, a benzonaphthosilole group, a benzofurodibenzofuran group, a benzofurodibenzothiophene group, a benzothienodibenzothiophene group, a pyrazole group, an imidazole group, a triazole group, an oxazole group, an isoxazole group, an oxadiazole group, a thiazole group, an isothiazole group, a thiadiazole group, a benzopyrazole group, a benzimidazole group, a benzoxazole group, a benzoisoxazole group, a benzothiazole group, a benzoisothiazole group, a pyridine group, a pyrimidine group, a pyrazine group, a pyridazine group, a triazine group, a quinoline group, an isoquinoline group, a benzoquinoline group, a benzoisoquinoline group, a quinoxaline group, a benzoquinoxaline group, a quinazoline group, a benzoquinazoline group, a phenanthroline group, a cinnoline group, a phthalazine group, a naphthyridine group, an imidazopyridine group, an imidazopyrimidine group, an imidazotriazine group, an imidazopyrazine group, an imidazopyridazine group, an azacarbazole group, an azafluorene group, an azadibenzosilole group, an azadibenzothiophene group, an azadibenzofuran group, and/or the like),

the $\pi$ electron-rich $C_3$-$C_{60}$ cyclic group may be i) a T1 group, ii) a condensed cyclic group in which at least two T1 groups are condensed with each other, iii) a T3 group (defined below), iv) a condensed cyclic group in which at least two T3 groups are condensed with each other, or v) a condensed cyclic group in which at least one T3 group and at least one T1 group are condensed with each other (for example, the $C_3$-$C_{60}$ carbocyclic group, a 1H-pyrrole group, a silole group, a borole group, a 2H-pyrrole group, a 3H-pyrrole group, a thiophene group, a furan group, an indole group, a benzoindole group, a naphthoindole group, an isoindole group, a benzoisoindole group, a naphthoisoindole group, a benzosilole group, a benzothiophene group, a benzofuran group, a carbazole group, a dibenzosilole group, a dibenzothiophene group, a dibenzofuran group, an indenocarbazole group, an indolocarbazole group, a benzofurocarbazole group, a benzothienocarbazole group, a benzosilolocarbazole group, a benzoindolocarbazole group, a benzocarbazole group, a benzonaphthofuran group, a benzonaphthothiophene group, a benzonaphthosilole group, a benzofurodibenzofuran group, a benzofurodibenzothiophene group, a benzothienodibenzothiophene group, and/or the like),

the $\pi$ electron-deficient nitrogen-containing $C_1$-$C_{60}$ cyclic group may be i) a T4 group (defined below), ii) a condensed cyclic group in which at least two T4 groups are condensed with each other, iii) a condensed cyclic group in which

at least one T4 group and at least one T1 group are condensed with each other, iv) a condensed cyclic group in which at least one T4 group and at least one T3 group are condensed with each other, or v) a condensed cyclic group in which at least one T4 group, at least one T1 group, and at least one T3 group are condensed with one another (for example, a pyrazole group, an imidazole group, a triazole group, an oxazole group, an isoxazole group, an oxadiazole group, a thiazole group, an isothiazole group, a thiadiazole group, a benzopyrazole group, a benzimidazole group, a benzoxazole group, a benzoisoxazole group, a benzothiazole group, a benzoisothiazole group, a pyridine group, a pyrimidine group, a pyrazine group, a pyridazine group, a triazine group, a quinoline group, an isoquinoline group, a benzoquinoline group, a benzoisoquinoline group, a quinoxaline group, a benzoquinoxaline group, a quinazoline group, a benzoquinazoline group, a phenanthroline group, a cinnoline group, a phthalazine group, a naphthyridine group, an imidazopyridine group, an imidazopyrimidine group, an imidazotriazine group, an imidazopyrazine group, an imidazopyridazine group, an azacarbazole group, an azafluorene group, an azadibenzosilole group, an azadibenzothiophene group, an azadibenzofuran group, and/or the like),

the T1 group may be a cyclopropane group, a cyclobutane group, a cyclopentane group, a cyclohexane group, a cycloheptane group, a cyclooctane group, a cyclobutene group, a cyclopentene group, a cyclopentadiene group, a cyclohexene group, a cyclohexadiene group, a cycloheptene group, an adamantane group, a norbornane (or a bicyclo[2.2.1]heptane) group, a norbornene group, a bicyclo[1.1.1]pentane group, a bicyclo[2.1.1]hexane group, a bicyclo[2.2.2]octane group, or a benzene group,

the T2 group may be a furan group, a thiophene group, a 1H-pyrrole group, a silole group, a borole group, a 2H-pyrrole group, a 3H-pyrrole group, an imidazole group, a pyrazole group, a triazole group, a tetrazole group, an oxazole group, an isoxazole group, an oxadiazole group, a thiazole group, an isothiazole group, a thiadiazole group, an azasilole group, an azaborole group, a pyridine group, a pyrimidine group, a pyrazine group, a pyridazine group, a triazine group, a tetrazine group, a pyrrolidine group, an imidazolidine group, a dihydropyrrole group, a piperidine group, a tetrahydropyridine group, a dihydropyridine group, a hexahydropyrimidine group, a tetrahydropyrimidine group, a dihydropyrimidine group, a piperazine group, a tetrahydropyrazine group, a dihydropyrazine group, a tetrahydropyridazine group, or a dihydropyridazine group,

the T3 group may be a furan group, a thiophene group, a 1H-pyrrole group, a silole group, or a borole group, and

the T4 group may be a 2H-pyrrole group, a 3H-pyrrole group, an imidazole group, a pyrazole group, a triazole group, a tetrazole group, an oxazole group, an isoxazole group, an oxadiazole group, a thiazole group, an isothiazole group, a thiadiazole group, an azasilole group, an azaborole group, a pyridine group, a pyrimidine group, a pyrazine group, a pyridazine group, a triazine group, or a tetrazine group.

The terms "the cyclic group", "the $C_3$-$C_{60}$ carbocyclic group", "the $C_1$-$C_{60}$ heterocyclic group", "the $\pi$ electron-rich C3-C60 cyclic group", and/or "the $\pi$ electron-deficient nitrogen-containing $C_1$-$C_{60}$ cyclic group" as used herein may refer to a group condensed to any cyclic group, a monovalent group, or a polyvalent group (for example, a divalent group, a trivalent group, a tetravalent group, and/or the like) according to the structural context of a formula for which the corresponding term is used. For example, the "benzene group" may be a benzo group, a phenyl group, a phenylene group, and/or the like, which may be easily understood by one of ordinary skill in the art according to the structure of a formula including the "benzene group".

Examples of the monovalent $C_3$-$C_{60}$ carbocyclic group and the monovalent $C_1$-$C_{60}$ heterocyclic group may include a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_1$-$C_{60}$ heteroaryl group, a monovalent non-aromatic condensed polycyclic group, and/or a monovalent non-aromatic condensed heteropolycyclic group. Examples of the divalent $C_3$-$C_{60}$ carbocyclic group and the monovalent $C_1$-$C_{60}$ heterocyclic group may include a $C_3$-$C_{10}$ cycloalkylene group, a $C_1$-$C_{10}$ heterocycloalkylene group, a $C_3$-$C_{10}$ cycloalkenylene group, a $C_1$-$C_{10}$ heterocycloalkenylene group, a $C_6$-$C_{60}$ arylene group, a $C_1$-$C_{60}$ heteroarylene group, a divalent non-aromatic condensed polycyclic group, and/or a substituted or unsubstituted divalent non-aromatic condensed heteropolycyclic group.

The term "$C_1$-$C_{60}$ alkyl group" as used herein refers to a linear or branched aliphatic hydrocarbon monovalent group that has one to sixty carbon atoms, and examples thereof may include a methyl group, an ethyl group, an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, an n-hexyl group, an isohexyl group, a sec-hexyl group, a tert-hexyl group, an n-heptyl group, an isoheptyl group, a sec-heptyl group, a tert-heptyl group, an n-octyl group, an isooctyl group, a sec-octyl group, a tert-octyl group, an n-nonyl group, an iso-nonyl group, a sec-nonyl group, a tert-nonyl group, an n-decyl group, an isodecyl group, a sec-decyl group, a tert-decyl group, and/or the like. The term "$C_1$-$C_{60}$ alkylene group" as used herein refers to a divalent group having substantially the same structure as the $C_1$-$C_{60}$ alkyl group.

The term "$C_2$-$C_{60}$ alkenyl group" as used herein refers to a monovalent hydrocarbon group having at least one carbon-carbon double bond in the middle or at the terminus of the $C_2$-$C_{60}$ alkyl group, and examples thereof may include an ethenyl group, a propenyl group, a butenyl group, and/or the like. The term "$C_2$-$C_{60}$ alkenylene group" as used herein

refers to a divalent group having substantially the same structure as the $C_2$-$C_{60}$ alkenyl group.

The term "$C_2$-$C_{60}$ alkynyl group" as used herein refers to a monovalent hydrocarbon group having at least one carbon-carbon triple bond in the middle or at the terminus of the $C_2$-$C_{60}$ alkyl group, and examples thereof may include an ethynyl group, a propynyl group, and/or the like. The term "$C_2$-$C_{60}$ alkynylene group" as utilized herein refers to a divalent group having substantially the same structure as the $C_2$-$C_{60}$ alkynyl group.

The term "$C_1$-$C_{60}$ alkoxy group" as used herein refers to a monovalent group represented by $-OA_{101}$ (wherein A101 is a $C_1$-$C_{60}$ alkyl group), and examples thereof may include (e.g., may be) a methoxy group, an ethoxy group, an isopropyloxy group, and/or the like.

The term "$C_3$-$C_{10}$ cycloalkyl group" as used herein refers to a monovalent saturated hydrocarbon cyclic group having three to ten carbon atoms, and examples thereof may include (e.g., may be) a cyclopropyl group, a cyclobutyl group, a cyclopentyl group, a cyclohexyl group, a cycloheptyl group, a cyclooctyl group, an adamantanyl group, a norbornanyl group (or bicyclo[2.2.1]heptyl group), a bicyclo[1.1.1]pentyl group, a bicyclo[2.1.1]hexyl group, a bicyclo[2.2.2]octyl group, and/or the like. The term "$C_3$-$C_{10}$ cycloalkylene group" as used herein refers to a divalent group having substantially the same structure as the $C_3$-$C_{10}$ cycloalkyl group.

The term "$C_1$-$C_{10}$ heterocycloalkyl group" as used herein refers to a monovalent cyclic group that further includes, in addition to a carbon atom, at least one heteroatom as a ring-forming atom and has 1 to 10 carbon atoms, and examples thereof may include (e.g., may be) a 1,2,3,4-oxatriazolidinyl group, a tetrahydrofuranyl group, a tetrahydrothiophenyl group, and/or the like. The term "$C_1$-$C_{10}$ heterocycloalkylene group" as used herein refers to a divalent group having substantially the same structure as the $C_1$-$C_{10}$ heterocycloalkyl group.

The term "$C_3$-$C_{10}$ cycloalkenyl group" as used herein refers to a monovalent cyclic group that has 3 to 10 carbon atoms, at least one carbon-carbon double bond in the ring thereof, and no aromaticity, and examples thereof may include (e.g., may be) a cyclopentenyl group, a cyclohexenyl group, a cycloheptenyl group, and/or the like. The term "$C_3$-$C_{10}$ cycloalkenylene group" as used herein refers to a divalent group having substantially the same structure as the $C_3$-$C_{10}$ cycloalkenyl group.

The term "$C_1$-$C_{10}$ heterocycloalkenyl group" as used herein refers to a monovalent cyclic group that has, in addition to a carbon atom, at least one heteroatom as a ring-forming atom, 1 to 10 carbon atoms, and at least one carbon-carbon double bond in the cyclic structure thereof. Examples of the $C_1$-$C_{10}$ heterocycloalkenyl group may include (e.g., may be) a 4,5-dihydro-1,2,3,4-oxatriazolyl group, a 2,3-dihydrofuranyl group, a 2,3-dihydrothiophenyl group, and/or the like. The term "$C_1$-$C_{10}$ heterocycloalkenylene group" as used herein refers to a divalent group having substantially the same structure as the $C_1$-$C_{10}$ heterocycloalkenyl group.

The term "$C_6$-$C_{60}$ aryl group" as used herein refers to a monovalent group having a carbocyclic aromatic system having 6 to 60 carbon atoms, and the term "$C_6$-$C_{60}$ arylene group" as used herein refers to a divalent group having a carbocyclic aromatic system having 6 to 60 carbon atoms. Examples of the $C_6$-$C_{60}$ aryl group may include (e.g., may be) a phenyl group, a pentalenyl group, a naphthyl group, an azulenyl group, an indacenyl group, an acenaphthyl group, a phenalenyl group, a phenanthrenyl group, an anthracenyl group, a fluoranthenyl group, a triphenylenyl group, a pyrenyl group, a chrysenyl group, a perylenyl group, a pentaphenyl group, a heptalenyl group, a naphthacenyl group, a picenyl group, a hexacenyl group, a pentacenyl group, a rubicenyl group, a coronenyl group, an ovalenyl group, and/or the like. When the $C_6$-$C_{60}$ aryl group and the $C_6$-$C_{60}$ arylene group each include two or more rings, the rings may be condensed with each other.

The term "$C_1$-$C_{60}$ heteroaryl group" as used herein refers to a monovalent group having a heterocyclic aromatic system that has, in addition to a carbon atom, at least one heteroatom as a ring-forming atom, and 1 to 60 carbon atoms. The term "$C_1$-$C_{60}$ heteroarylene group" as used herein refers to a divalent group having a heterocyclic aromatic system that has, in addition to a carbon atom, at least one heteroatom as a ring-forming atom, and 1 to 60 carbon atoms. Examples of the $C_1$-$C_{60}$ heteroaryl group may include (e.g., may be) a pyridinyl group, a pyrimidinyl group, a pyrazinyl group, a pyridazinyl group, a triazinyl group, a quinolinyl group, a benzoquinolinyl group, an isoquinolinyl group, a benzoisoquinolinyl group, a quinoxalinyl group, a benzoquinoxalinyl group, a quinazolinyl group, a benzoquinazolinyl group, a cinnolinyl group, a phenanthrolinyl group, a phthalazinyl group, a naphthyridinyl group, and/or the like. When the $C_1$-$C_{60}$ heteroaryl group and the $C_1$-$C_{60}$ heteroarylene group each include two or more rings, the rings may be condensed with each other.

The term "monovalent non-aromatic condensed polycyclic group" as used herein refers to a monovalent group (for example, having 8 to 60 carbon atoms) having two or more rings condensed to each other, only carbon atoms as ring-forming atoms, and no aromaticity in its entire molecular structure (e.g., an aromatic conjugation system does not extend throughout the entire structure). Examples of the monovalent non-aromatic condensed polycyclic group may include (e.g., may be) an indenyl group, a fluorenyl group, a spiro-bifluorenyl group, a benzofluorenyl group, an indenophenanthrenyl group, and/or an indeno anthracenyl group. The term "divalent non-aromatic condensed polycyclic group" as used herein refers to a divalent group having substantially the same structure as the monovalent non-aromatic condensed polycyclic group described above.

The term "monovalent non-aromatic condensed heteropolycyclic group" as used herein refers to a monovalent

group (for example, having 1 to 60 carbon atoms) having two or more rings condensed with each other, at least one heteroatom other than carbon atoms, as a ring-forming atom, and no aromaticity in its entire molecular structure (e.g., an aromatic conjugation system does not extend throughout the entire structure). Examples of the monovalent non-aromatic condensed heteropolycyclic group may include (e.g., may be) a pyrrolyl group, a thiophenyl group, a furanyl group, an indolyl group, a benzoindolyl group, a naphthoindolyl group, an isoindolyl group, a benzoisoindolyl group, a naphthoisoindolyl group, a benzosilolyl group, a benzothiophenyl group, a benzofuranyl group, a carbazolyl group, a dibenzosilolyl group, a dibenzothiophenyl group, a dibenzofuranyl group, an azacarbazolyl group, an azafluorenyl group, an azadibenzosilolyl group, an azadibenzothiophenyl group, an azadibenzofuranyl group, a pyrazolyl group, an imidazolyl group, a triazolyl group, a tetrazolyl group, an oxazolyl group, an isoxazolyl group, a thiazolyl group, an isothiazolyl group, an oxadiazolyl group, a thiadiazolyl group, a benzopyrazolyl group, a benzimidazolyl group, a benzoxazolyl group, a benzothiazolyl group, a benzoxadiazolyl group, a benzothiadiazolyl group, an imidazopyridinyl group, an imidazopyrimidinyl group, an imidazotriazinyl group, an imidazopyrazinyl group, an imidazopyridazinyl group, an indeno carbazolyl group, an indolocarbazolyl group, a benzofurocarbazolyl group, a benzothienocarbazolyl group, a benzosilolocarbazolyl group, a benzoindolocarbazolyl group, a benzocarbazolyl group, a benzonaphthofuranyl group, a benzonaphthothiophenyl group, a benzonaphthosilolyl group, a benzofurodibenzofuranyl group, a benzofurodibenzothiophenyl group, a benzothienodibenzothiophenyl group, and/or the like. The term "divalent non-aromatic condensed heteropolycyclic group" as used herein refers to a divalent group having substantially the same structure as the monovalent non-aromatic condensed heteropolycyclic group described above.

The term "$C_6$-$C_{60}$ aryloxy group" as used herein indicates -$OA_{102}$ (wherein A102 is a $C_6$-$C_{60}$ aryl group), and the term "$C_6$-$C_{60}$ arylthio group" as used herein indicates -SA103 (wherein A103 is a $C_6$-$C_{60}$ aryl group).

The term "$C_7$-$C_{60}$ arylalkyl group" as used herein refers to -$A_{104}A_{105}$ (wherein A104 is a $C_1$-$C_{54}$ alkylene group and A105 is a $C_6$-$C_{59}$ aryl group), and the term "$C_2$-$C_{60}$ heteroarylalkyl group" as used herein refers to -$A_{106}A_{107}$ (wherein A106 is a $C_1$-$C_{59}$ alkylene group and A107 is a $C_1$-$C_{59}$ heteroaryl group).

The term "$R_{10a}$" as used herein may be:

deuterium, -F, -Cl, -Br, -I, a hydroxyl group, a cyano group, or a nitro group;

a $C_1$-$C_{60}$ alkyl group, a $C_2$-$C_{60}$ alkenyl group, a $C_2$-$C_{60}$ alkynyl group, or a $C_1$-$C_{60}$ alkoxy group, each unsubstituted or substituted with deuterium, -F, -Cl, -Br, -I, a hydroxyl group, a cyano group, a nitro group, a $C_3$-$C_{60}$ carbocyclic group, a $C_1$-$C_{60}$ heterocyclic group, a $C_6$-$C_{60}$ aryloxy group, a $C_6$-$C_{60}$ arylthio group, a $C_7$-$C_{60}$ arylalkyl group, a $C_2$-$C_{60}$ heteroarylalkyl group, -Si($Q_{11}$)($Q_{12}$)($Q_{13}$), -N($Q_{11}$)($Q_{12}$), -B($Q_{11}$)($Q_{12}$), - C(=O)($Q_{11}$), -S(=O)$_2$($Q_{11}$), -P(=O)($Q_{11}$)($Q_{12}$), or any combination thereof,

a $C_3$-$C_{60}$ carbocyclic group, a $C_1$-$C_{60}$ heterocyclic group, a $C_6$-$C_{60}$ aryloxy group, a $C_6$-$C_{60}$ arylthio group, a $C_7$-$C_{60}$ arylalkyl group, or a $C_2$-$C_{60}$ heteroaryl alkyl group, each unsubstituted or substituted with deuterium, -F, -Cl, -Br, -I, a hydroxyl group, a cyano group, a nitro group, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{60}$ carbocyclic group, a $C_1$-$C_{60}$ heterocyclic group, a $C_6$-$C_{60}$ aryloxy group, a $C_6$-$C_{60}$ arylthio group, a $C_7$-$C_{60}$ arylalkyl group, a $C_2$-$C_{60}$ heteroaryl alkyl group, -Si($Q_{21}$)($Q_{22}$)($Q_{23}$), -N($Q_{21}$)($Q_{22}$), -B($Q_{21}$)($Q_{22}$), - C(=O)($Q_{21}$), -S(=O)$_2$($Q_{21}$), -P(=O)($Q_{21}$)($Q_{22}$), or any combination thereof; or

-Si($Q_{31}$)($Q_{32}$)($Q_{33}$), -N($Q_{31}$)($Q_{32}$), -B($Q_{31}$)($Q_{32}$), -C(=O)($Q_{31}$), -S(=O)$_2$($Q_{31}$), or - P(=O)($Q_{31}$)($Q_{32}$).

In the present specification, $Q_1$ to $Q_3$, $Q_{11}$ to $Q_{13}$, $Q_{21}$ to $Q_{23}$, and $Q_{31}$ to $Q_{33}$ may each independently be: hydrogen; deuterium; -F; -Cl; -Br; -I; a hydroxyl group; a cyano group; a nitro group; a $C_1$-$C_{60}$ alkyl group; a $C_2$-$C_{60}$ alkenyl group; a $C_2$-$C_{60}$ alkynyl group; a $C_1$-$C_{60}$ alkoxy group; or a $C_3$-$C_{60}$ carbocyclic group, a $C_1$-$C_{60}$ heterocyclic group, a $C_7$-$C_{60}$ aryl alkyl group, or a $C_2$-$C_{60}$ heteroaryl alkyl group, each unsubstituted or substituted with deuterium, -F, a cyano group, a $C_1$-$C_{60}$ alkyl group, a $C_1$-$C_{60}$ alkoxy group, a phenyl group, a biphenyl group, or any combination thereof.

The term "heteroatom" as used herein refers to any atom other than a carbon atom. Examples of the heteroatom may include (e.g., may be) oxygen (O), sulfur (S), nitrogen (N), phosphorus (P), silicon (Si), boron (B), germanium (Ge), selenium (Se), or any combination thereof.

The term "third-row transition metal" as used herein includes hafnium (Hf), tantalum (Ta), tungsten (W), rhenium (Re), osmium (Os), iridium (Ir), platinum (Pt), gold (Au), and/or the like.

"Ph" as used herein refers to a phenyl group, "Me" as used herein refers to a methyl group, "Et" as used herein refers to an ethyl group, "ter-Bu" or "$Bu^t$" as used herein refers to a tert-butyl group, and "OMe" as used herein refers to a methoxy group.

The term "biphenyl group" as used herein refers to "a phenyl group substituted with a phenyl group". In other words, the "biphenyl group" belongs to "a substituted phenyl group having a $C_6$-$C_{60}$ aryl group as a substituent".

The term "terphenyl group" as used herein refers to "a phenyl group substituted with a biphenyl group". In other words, the "terphenyl group" belongs to "a substituted phenyl group having, as a substituent, a $C_6$-$C_{60}$ aryl group substituted with a $C_6$-$C_{60}$ aryl group".

In the present specification, * and *', unless defined otherwise, each refer to a binding site to a neighboring atom in a corresponding formula or moiety.

Hereinafter, compounds according to embodiments and light-emitting devices according to embodiments will be described in more detail with reference to the following synthesis examples and examples. The wording "B was utilized instead of A" used in describing Synthesis Examples indicates that an identical molar equivalent of B was used in place of A.

**Examples**

**Synthesis Example 1: Synthesis of Compound 1**

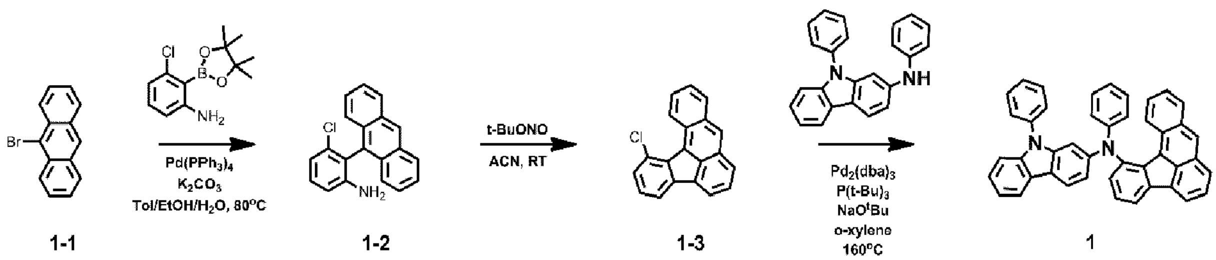


1-1 1-2 1-3 1

**Synthesis of Intermediate 1-2**

Intermediate 1-1 (2.57 g), $Pd(PPh_3)_4$ (0.56 g), $K_2CO_3$ (3.45 g), and 3-chloro-2-(4,4,5,5-tetramethyl-1,3,2-dioxaborolan-2-yl)aniline (2.53 g) were dissolved in toluene/EtOH/$H_2O$ (100 mL /25 mL /25 mL), and stirred at a temperature of 80 °C for 12 hours. After the reaction solution was cooled to room temperature, the reaction was terminated by utilizing water. Then, an extraction process was performed thereon three times by utilizing ethyl ether. An organic layer extracted therefrom was dried by utilizing anhydrous magnesium sulfate and distilled under reduced pressure, and a residue thus obtained was separated and purified by column chromatography, thereby obtaining Intermediate 1-2 (1.91 g, yield: 63 %).

**Synthesis of Intermediate 1-3**

Intermediate 1-2 (3.03 g) was dissolved in acetonitrile (50 mL), and t-BuONO (1.56 g) was added thereto. The reaction solution was then stirred at room temperature for 12 hours. After the reaction solution was cooled to room temperature, the reaction was terminated by utilizing water. Then, an extraction process was performed thereon three times by utilizing ethyl ether. An organic layer extracted therefrom was dried by utilizing anhydrous magnesium sulfate and distilled under reduced pressure, and a residue thus obtained was separated and purified by column chromatography, thereby obtaining Intermediate 1-3 (0.95 g, yield: 33 %).

**Synthesis of Compound 1**

Intermediate 1-3 (2.86 g), N,9-diphenyl-9H-carbazol-2-amine (3.35 g), $Pd_2(dba)_3$ (0.46 g), $P(t\text{-}Bu)_3$ (0.21 g), and $NaO^tBu$ (2.44 g) were dissolved in o-xylene (50 mL), and then stirred at a temperature of 160 °C for 1 hour. After the reaction solution was cooled to room temperature, the reaction was terminated by utilizing water. Then, an extraction process was performed thereon three times by utilizing ethyl ether. An organic layer extracted therefrom was dried by utilizing anhydrous magnesium sulfate and distilled under reduced pressure, and a residue thus obtained was separated and purified by column chromatography, thereby obtaining Compound 1 (4.91 g, yield: 83 %).

**Synthesis Example 2: Synthesis of Compound 4**

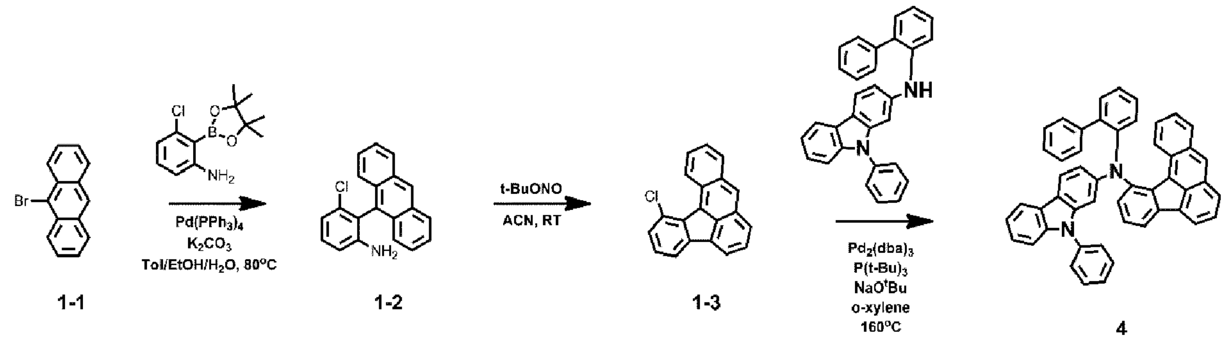


1-1 1-2 1-3 4

**Synthesis of Compound 4**

Intermediate 1-3 (2.86 g), N-([1,1'-biphenyl]-2-yl)-9-phenyl-9H-carbazol-2-amine (4.10 g), $Pd_2(dba)_3$ (0.46 g), $P(t\text{-}Bu)_3$ (0.21 g), and $NaO^tBu$ (2.44 g) were dissolved in o-xylene (50 mL), and then stirred at a temperature of 160 °C for 1 hour. After the reaction solution was cooled to room temperature, the reaction was terminated by utilizing water. Then, an extraction process was performed thereon three times by utilizing ethyl ether. An organic layer extracted therefrom was dried by utilizing anhydrous magnesium sulfate and distilled under reduced pressure, and a residue thus obtained was separated and purified by column chromatography, thereby obtaining Compound 4 (4.91 g, yield: 83 %).

**Synthesis Example 3: Synthesis of Compound 21**

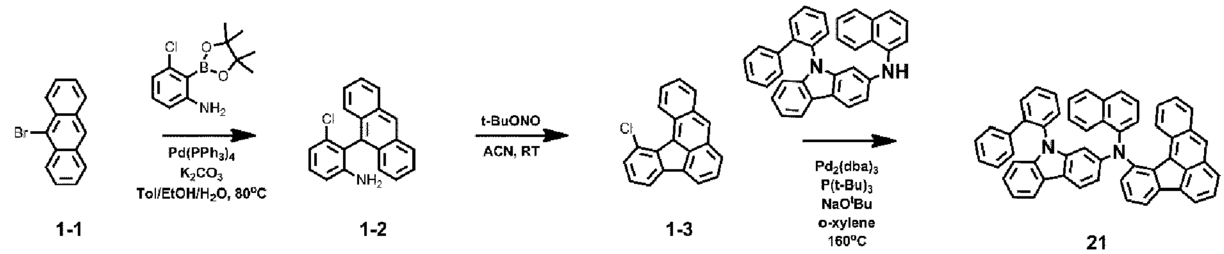


1-1 1-2 1-3 21

**Synthesis of Compound 21**

Intermediate 1-3 (2.86 g), 9-([1,1'-biphenyl]-2-yl)-N-(naphthalen-1-yl)-9H-carbazol-2-amine (4.60 g), $Pd_2(dba)_3$ (0.46 g), $P(t\text{-}Bu)_3$ (0.21 g), and $NaO^tBu$ (2.44 g) were dissolved in o-xylene (50 mL), and then stirred at a temperature of 160 °C for 1 hour. After the reaction solution was cooled to room temperature, the reaction was terminated by utilizing water. Then, an extraction process was performed thereon three times by utilizing ethyl ether. An organic layer extracted therefrom was dried by utilizing anhydrous magnesium sulfate and distilled under reduced pressure, and a residue thus obtained was separated and purified by column chromatography, thereby obtaining

Compound 21 (5.32 g, yield: 75 %).

**Synthesis Example 4: Synthesis of Compound 52**

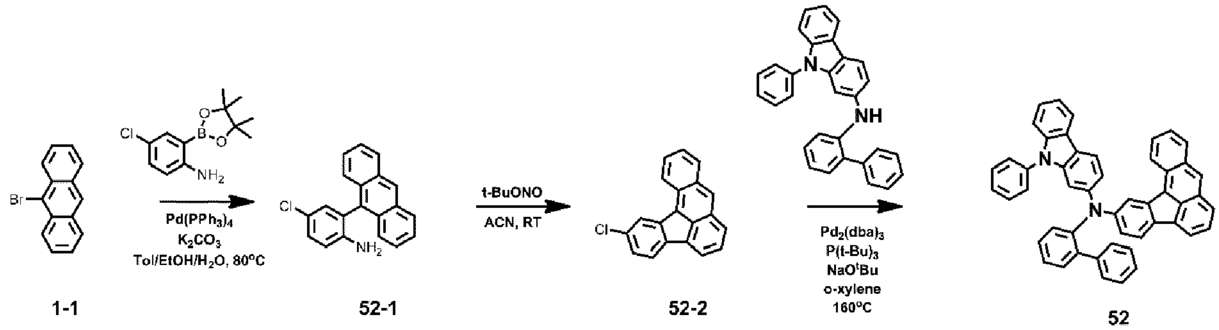


1-1 52-1 52-2 52

### Synthesis of Intermediate 52-1

Intermediate 1-1 (2.57 g), $Pd(PPh_3)_4$ (0.56 g), $K_2CO_3$ (3.45 g), and 4-chloro-2-(4,4,5,5-tetramethyl-1,3,2-dioxaborolan-2-yl)aniline (2.53 g) were dissolved in toluene/EtOH/$H_2O$ (100 mL /25 mL /25 mL), and stirred at a temperature of 80 °C for 12 hours. The reaction solution was cooled to room temperature, and the reaction was terminated by utilizing water. Then, an extraction process was performed thereon three times by utilizing ethyl ether. An organic layer extracted therefrom was dried by utilizing anhydrous magnesium sulfate and distilled under reduced pressure, and a residue thus obtained was separated and purified by column chromatography, thereby obtaining Intermediate 52-1 (1.81 g, yield: 60 %).

### Synthesis of Intermediate 52-2

Intermediate 52-1 (3.03 g) was dissolved in acetonitrile (50 mL), and t-BuONO (1.56 g) was added thereto. The reaction solution was then stirred at room temperature for 12 hours. After the reaction solution was cooled to room temperature, the reaction was terminated by utilizing water. Then, an extraction process was performed thereon three times by utilizing ethyl ether. An organic layer extracted therefrom was dried by utilizing anhydrous magnesium sulfate and distilled under reduced pressure, and a residue thus obtained was separated and purified by column chromatography, thereby obtaining Intermediate 52-2 (1.00 g, yield: 35 %).

### Synthesis of Compound 52

Intermediate 52-2 (2.86 g), N-([1,1'-biphenyl]-2-yl)-9-phenyl-9H-carbazol-2-amine (4.10 g), $Pd_2(dba)_3$ (0.46 g), $P(t\text{-}Bu)_3$ (0.21 g), and $NaO^tBu$ (2.44 g) were dissolved in o-xylene (50 mL), and then stirred at a temperature of 160 °C for 1 hour. After the reaction solution was cooled to room temperature, the reaction was terminated by utilizing water. Then, an extraction process was performed thereon three times by utilizing ethyl ether. An organic layer extracted therefrom was dried by utilizing anhydrous magnesium sulfate and distilled under reduced pressure, and a residue thus obtained was separated and purified by column chromatography, thereby obtaining Compound 52 (5.02 g, yield: 76 %).

### Synthesis Example 5: Synthesis of Compound 68

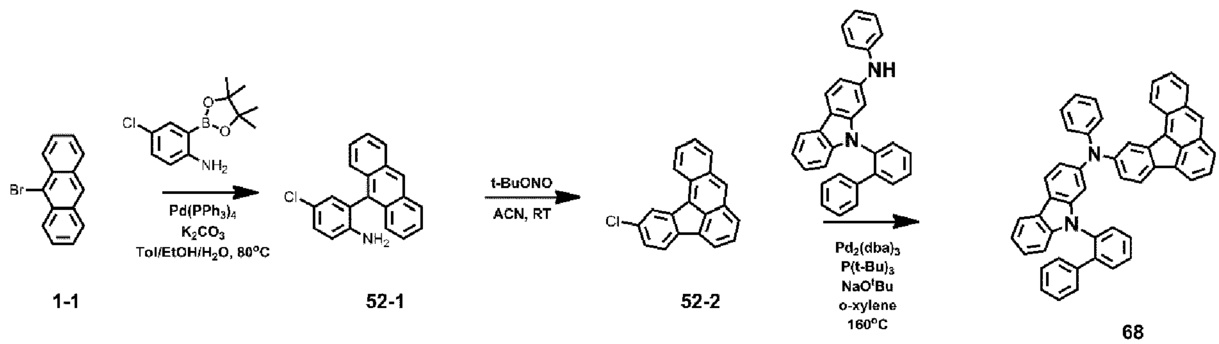


1-1 52-1 52-2 68

### Synthesis of Compound 68

Intermediate 52-2 (2.86 g), 9-([1,1'-biphenyl]-2-yl)-N-phenyl-9H-carbazol-2-amine (4.10 g), $Pd_2(dba)_3$ (0.46 g),

P(t-Bu)$_3$ (0.21 g), and NaO$^t$Bu (2.44 g) were dissolved in o-xylene (50 mL), and then stirred at a temperature of 160 °C for 1 hour. After the reaction solution was cooled to room temperature, the reaction was terminated by utilizing water. Then, an extraction process was performed thereon three times by utilizing ethyl ether. An organic layer extracted therefrom was dried by utilizing anhydrous magnesium sulfate and distilled under reduced pressure, and a residue thus obtained was separated and purified by column chromatography, thereby obtaining Compound 68 (5.08 g, yield: 77 %).

**Synthesis Example 6: Synthesis of Compound 93**

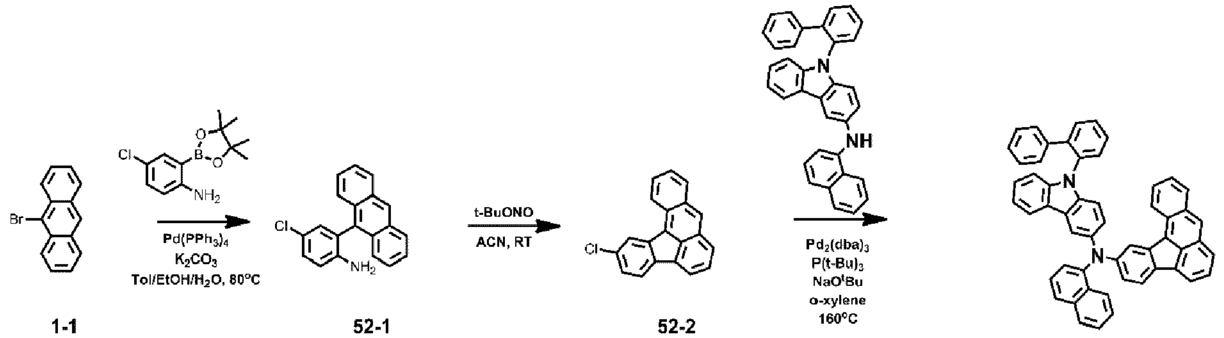


1-1 52-1 52-2

93

**Synthesis of Compound 93**

Intermediate 52-2 (2.86 g), 9-([1,1'-biphenyl]-2-yl)-N-(naphthalen-1-yl)-9H-carbazol-3-amine (4.60 g), Pd$_2$(dba)$_3$ (0.46 g), P(t-Bu)$_3$ (0.21 g), and NaO$^t$Bu (2.44 g) were dissolved in o-xylene (50 mL), and then stirred at a temperature of 160 °C for 1 hour. After the reaction solution was cooled to room temperature, the reaction was terminated by utilizing water. Then, an extraction process was performed thereon three times by utilizing ethyl ether. An organic layer extracted therefrom was dried by utilizing anhydrous magnesium sulfate and distilled under reduced pressure, and a residue thus obtained was separated and purified by column chromatography, thereby obtaining Compound 93 (4.90 g, yield: 69 %).

**Synthesis Example 7: Synthesis of Compound 104**

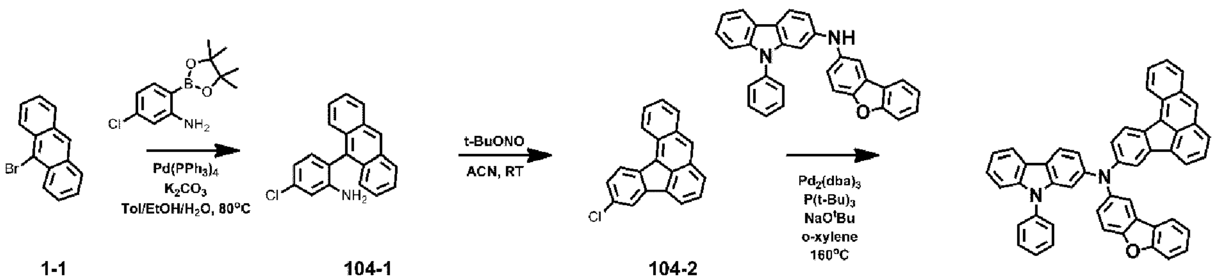


1-1 104-1 104-2

104

**Synthesis of Intermediate 104-1**

Intermediate 1-1 (2.57 g), Pd(PPh$_3$)$_4$ (0.56 g), $K_2CO_3$ (3.45 g), and 5-chloro-2-(4,4,5,5-tetramethyl-1,3,2-dioxaborolan-2-yl)aniline (2.53 g) were dissolved in toluene/EtOH/$H_2O$ (100 mL /25 mL /25 mL), and stirred at a temperature of 80 °C for 12 hours. After the reaction solution was cooled to room temperature, the reaction was terminated by utilizing water. Then, an extraction process was performed thereon three times by utilizing ethyl ether. An organic layer extracted therefrom was dried by utilizing anhydrous magnesium sulfate and distilled under reduced pressure, and a residue thus obtained was separated and purified by column chromatography, thereby obtaining Intermediate 104-1 (1.67 g, yield: 55 %).

**Synthesis of Intermediate 104-2**

Intermediate 104-1 (3.03 g) was dissolved in acetonitrile (50 mL), and t-BuONO (1.56 g) was added thereto. The reaction solution was then stirred at room temperature for 12 hours. After the reaction solution was cooled to room temperature, the reaction was terminated by utilizing water. Then, an extraction process was performed thereon three times by utilizing ethyl ether. An organic layer extracted therefrom was dried by utilizing anhydrous magnesium sulfate and distilled under reduced pressure, and a residue thus obtained was separated and purified by column chromatography, thereby obtaining Intermediate 104-2 (1.00 g, yield: 35 %).

**Synthesis of Compound 104**

Intermediate 104-2 (2.86 g), N-(dibenzo[b,d]furan-2-yl)-9-phenyl-9H-carbazol-2-amine (4.24 g), $Pd_2(dba)_3$ (0.46 g), $P(t\text{-}Bu)_3$ (0.21 g), and $NaO^tBu$ (2.44 g) were dissolved in o-xylene (50 mL), and then stirred at a temperature of 160 °C for 1 hour. After the reaction solution was cooled to room temperature, the reaction was terminated by utilizing water. Then, an extraction process was performed thereon three times by utilizing ethyl ether. An organic layer extracted therefrom was dried by utilizing anhydrous magnesium sulfate and distilled under reduced pressure, and a residue thus obtained was separated and purified by column chromatography, thereby obtaining Compound 104 (6.07 g, yield: 90 %).

**Synthesis Example 8: Synthesis of Compound 126**

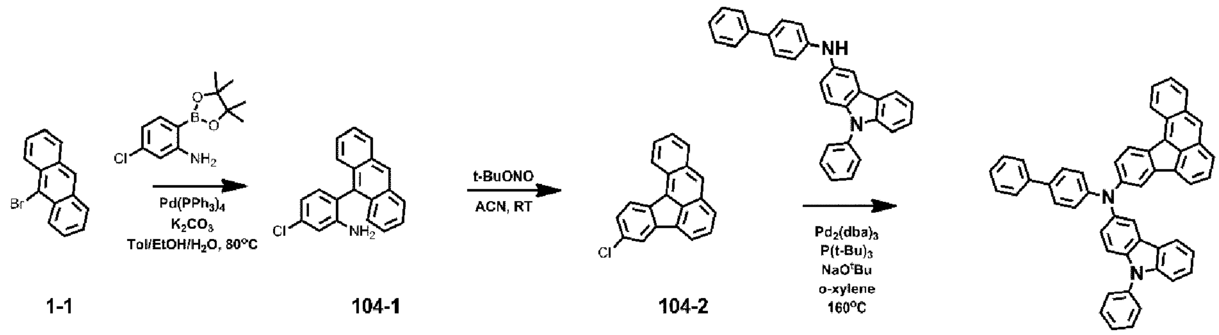


1-1 104-1 104-2 126

**Synthesis of Compound 126**

Intermediate 104-2 (2.86 g), N-([1,1'-biphenyl]-4-yl)-9-phenyl-9H-carbazol-3-amine (4.10 g), $Pd_2(dba)_3$ (0.46 g), $P(t\text{-}Bu)_3$ (0.21 g), and $NaO^tBu$ (2.44 g) were dissolved in o-xylene (50 mL), and then stirred at a temperature of 160 °C for 1 hour. After the reaction solution was cooled to room temperature, the reaction was terminated by utilizing water. Then, an extraction process was performed thereon three times by utilizing ethyl ether. An organic layer extracted therefrom was dried by utilizing anhydrous magnesium sulfate and distilled under reduced pressure, and a residue thus obtained was separated and purified by column chromatography, thereby obtaining Compound 126 (5.81 g, yield: 88 %).

**Synthesis Example 9: Synthesis of Compound 148**

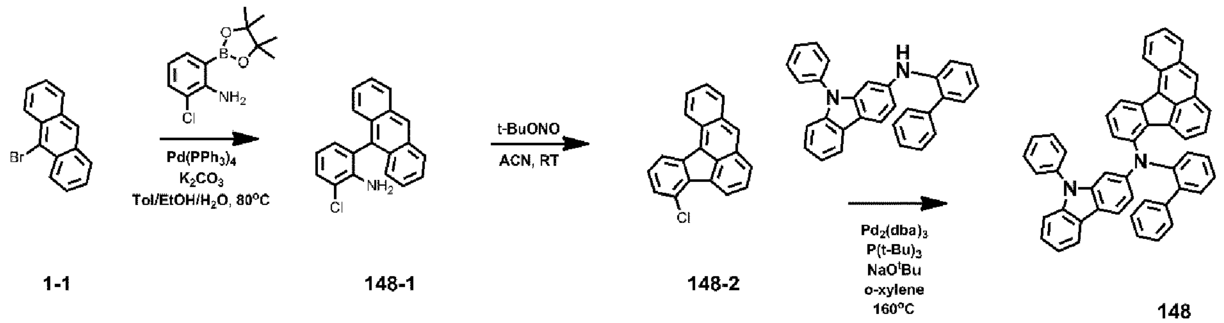


1-1 148-1 148-2 148

**Synthesis of Intermediate 148-1**

Intermediate 1-1 (2.57 g), $Pd(PPh_3)_4$ (0.56 g), $K_2CO_3$ (3.45 g), and 2-chloro-6-(4,4,5,5-tetramethyl-1,3,2-dioxaborolan-2-yl)aniline (2.53 g) were dissolved in toluene/EtOH/$H_2O$ (100 mL /25 mL /25 mL), and stirred at a temperature of 80 °C for 12 hours. After the reaction solution was cooled to room temperature, the reaction was terminated by utilizing water. Then, an extraction process was performed thereon three times by utilizing ethyl ether. An organic layer extracted therefrom was dried by utilizing anhydrous magnesium sulfate and distilled under reduced pressure, and a residue thus obtained was separated and purified by column chromatography, thereby obtaining Intermediate 148-1 (1.88 g, yield: 62 %).

**Synthesis of Intermediate 148-2**

Intermediate 148-1 (3.03 g) was dissolved in acetonitrile (50 mL), and t-BuONO (1.56 g) was added thereto. The reaction solution was then stirred at room temperature for 12 hours. After the reaction solution was cooled to room temperature, the reaction was terminated by utilizing water. Then, an extraction process was performed thereon three times by utilizing ethyl ether. An organic layer extracted therefrom was dried by utilizing anhydrous magnesium sulfate and distilled under reduced pressure, and a residue thus obtained was separated and purified by column chromatography, thereby obtaining Intermediate 148-2 (1.15 g, yield: 39 %).

**Synthesis of Compound 148**

Intermediate 148-2 (2.86 g), N-([1,1'-biphenyl]-2-yl)-9-phenyl-9H-carbazol-2-amine (4.10 g), $Pd_2(dba)_3$ (0.46 g), $P(t\text{-}Bu)_3$ (0.21 g), and $NaO^tBu$ (2.44 g) were dissolved in o-xylene (50 mL), and then stirred at a temperature of 160 °C for 1 hour. After the reaction solution was cooled to room temperature, the reaction was terminated by utilizing water. Then, an extraction process was performed thereon three times by utilizing ethyl ether. An organic layer extracted therefrom was dried by utilizing anhydrous magnesium sulfate and distilled under reduced pressure, and a residue thus obtained was separated and purified by column chromatography, thereby obtaining Compound 148 (5.08 g, yield: 77 %).

**Synthesis Example 10: Synthesis of Compound 170**

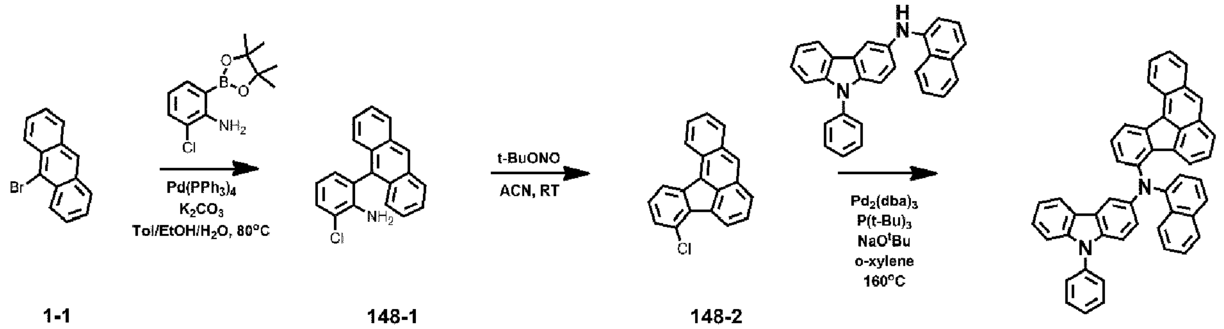


1-1 148-1 148-2 170

**Synthesis of Compound 170**

Intermediate 148-2 (2.86 g), N-(naphthalen-1-yl)-9-phenyl-9H-carbazol-3-amine (3.84 g), $Pd_2(dba)_3$ (0.46 g), $P(t\text{-}Bu)_3$ (0.21 g), and $NaO^tBu$ (2.44 g) were dissolved in o-xylene (50 mL), and then stirred at a temperature of 160 °C for 1 hour. After the reaction solution was cooled to room temperature, the reaction was terminated by utilizing water. Then, an extraction process was performed thereon three times by utilizing ethyl ether. An organic layer extracted therefrom was dried by utilizing anhydrous magnesium sulfate and distilled under reduced pressure, and a residue thus obtained was separated and purified by column chromatography, thereby obtaining Compound 170 (4.12 g, yield: 65 %).

**Synthesis Example 11: Synthesis of Compound 188**

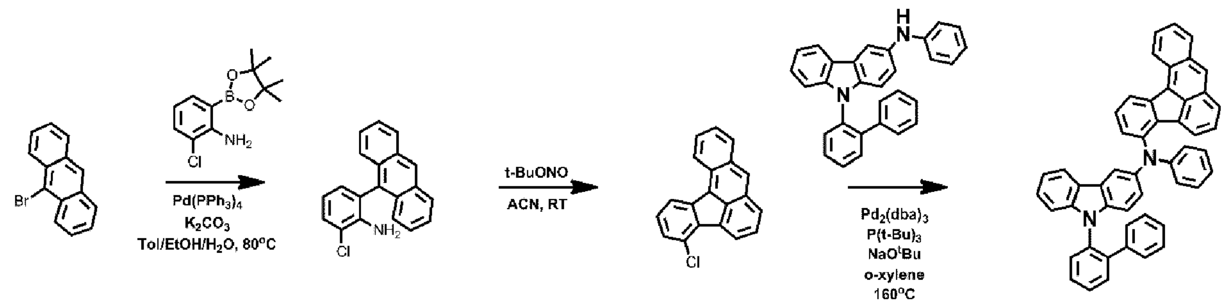


1-1 148-1 148-2

188

**Synthesis of Compound 188**

Intermediate 148-2 (2.86 g), 9-([1,1'-biphenyl]-2-yl)-N-phenyl-9H-carbazol-3-amine (4.10 g), $Pd_2(dba)_3$ (0.46 g), $P(t\text{-}Bu)_3$ (0.21 g), and $NaO^tBu$ (2.44 g) were dissolved in o-xylene (50 mL), and then stirred at a temperature of 160 °C for 1 hour. After the reaction solution was cooled to room temperature, the reaction was terminated by utilizing water. Then, an extraction process was performed thereon three times by utilizing ethyl ether. An organic layer extracted therefrom was dried by utilizing anhydrous magnesium sulfate and distilled under reduced pressure, and a residue thus obtained was separated and purified by column chromatography, thereby obtaining Compound 188 (5.01 g, yield: 76 %).

Table 1 shows $^1H$ NMR and MS/FAB of the synthesized compounds. Synthesis methods for compounds other than the compounds shown in Table 1 may be easily recognized by those skilled in the technical field by referring to the synthesis paths and source materials described above.

**Table 1**

| Compo und | $^1H$ NMR (δ) | MS/FAB | |
|---|---|---|---|
| | | Calc | Found |
| 1 | 8.55(d, 1H), 8.45(s,1H), 8.24-8.20(m,2H), 8.01-7.94(m,3H), 7.68-7.47 (m, 9H), 7.36-7.00 (m, 12H) | 584.23 | 583.23 |
| 4 | 8.55(d, 1H), 8.45(s,1H), 8.24-8.20(m,2H), 8.10(d,1H), 8.01-7.94(m,3H), 7.68-7.09 (m, 24H) | 660.26 | 659.26 |
| 21 | 8.55(d, 1H), 8.45(s,1H), 8.24-8.15(m,4H), 8.01-7.91(m,5H), 7.81-7.80(m,2H), 7.68-7.09 (m, 21H) | 710.27 | 709.27 |
| 52 | 8.55(d, 1H), 8.45(s,1H), 8.24-8.20(m,2H), 8.10(d,1H), 8.01-7.94(m,3H), 7.68-7.08 (m, 23H), 6.93(s,1H) | 660.26 | 659.26 |
| 68 | 8.55(d, 1H), 8.45(s,1H), 8.24-8.20(m,2H), 8.01-7.91(m,4H), 7.80(t,1H), 7.68 (d, 1H), 7.49-7.00(m,22H) | 660.26 | 659.26 |
| 93 | 8.55(d, 1H), 8.45(s,1H), 8.22-8.15(m,3H), 8.01-7.91(m,5H), 7.81-7.80(m,2H), 7.68-7.33 (m, 15H), 7.19-7.16(m,4H), 6.93(s,1H) | 710.27 | 709.27 |

(continued)

| Compo und | $^1$H NMR (δ) | MS/FAB | |
|---|---|---|---|
| | | Calc | Found |
| 104 | 8.55(d, 1H), 8.45(s,1H), 8.24-8.22(m,2H), 8.01-7.94(m,4H), 7.68-7.16(m, 19H), 6.97-6.93 (m, 2H) | 674.24 | 673.24 |
| 126 | 8.55(d, 1H), 8.45(s,1H), 8.20(d,1H), 8.01-7.94(m,3H), 7.75-7.33(m,23H), 7.18-7.16 (m, 2H), 6.93 (s, 1H) | 660.26 | 659.26 |
| 148 | 8.55(d, 1H), 8.45(s,1H), 8.24-8.20(m,2H), 8.01-7.94(m,3H), 7.68-7.08(m, 25H) | 660.26 | 659.26 |
| 170 | 8.55(d, 1H), 8.45(s,1H), 8.35-8.15(m,3H), 8.01-7.94(m,3H), 7.81 (d, 1H), 7.68-7.47(m,14H), 7.35-7.33(m,3H), 7.18-7.09(m,3H) | 634.24 | 633.24 |
| 188 | 8.55(d, 1H), 8.45(s,1H), 8.20(d,1H), 8.01-7.91(m,5H), 7.80(t, 1H), 7.68 (d,1H), 7.47-7.35(m,7H), 7.24-7.00(m,12H) | 660.26 | 659.26 |

**Example 1**

As an anode, a Corning 15 Ω/cm$^2$ (1,200 Å) ITO glass substrate was cut to a size of 50 mm x 50 mm x 0.7 mm, sonicated with isopropyl alcohol and pure water each for 5 minutes, and then cleaned by exposure to ultraviolet rays and ozone for 30 minutes. The ITO glass substrate was provided to a vacuum deposition apparatus.

2-TNATA was vacuum-deposited on an ITO anode on the glass substrate to form an hole injection layer having a thickness of 600 Å, and Compound 1 was vacuum-deposited on the hole injection layer to form a hole transport layer having a thickness of 300 Å.

DNA as a host and DPAVBi as a dopant were co-deposited at a weight ratio of 98:2 on the hole transport layer to form an emission layer having a thickness of 300 Å.

Subsequently, $Alq_3$ was deposited on the emission layer to form an electron transport layer having a thickness of 300 Å, LiF was deposited on the electron transport layer to form an electron injection layer having a thickness of 10 Å, and Al was vacuum-deposited on thereon to form a LiF/Al electrode having a thickness of 3,000 Å, thereby completing the manufacture of a light-emitting device.

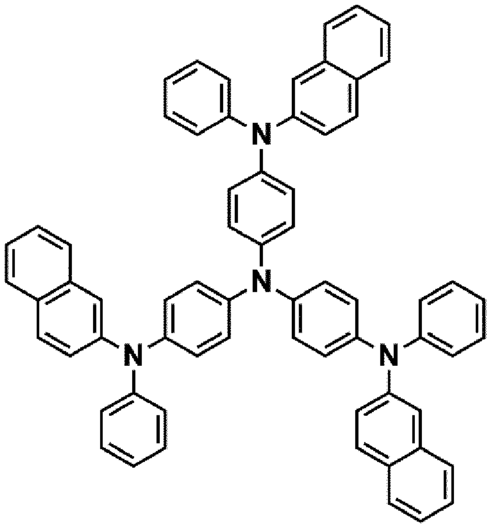

2-TNATA

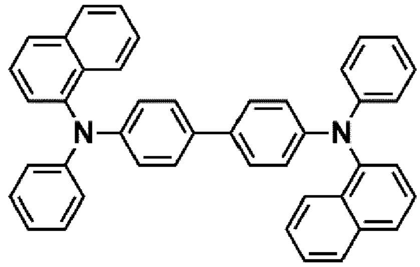

NPB

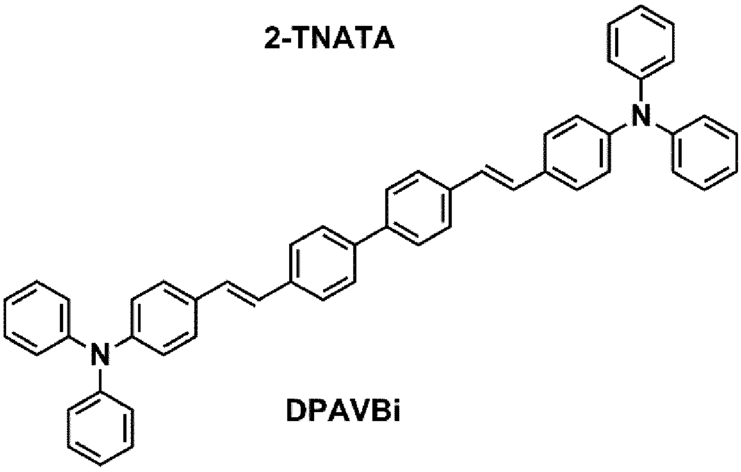

DPAVBi

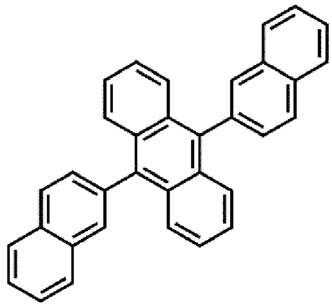

DNA

.

**Examples 2 to 11 and Comparative Examples 1 to 5**

Light-emitting devices were manufactured in substantially the same manner as in Example 1, except that the hole transport layer compounds shown in Table 2 were utilized instead of Compound 1 in forming the hole transport layer.

**Evaluation Example 1**

To evaluate characteristics of the light-emitting devices manufactured according to Examples 1 to 11 and Comparative Examples 1 to 5, the driving voltage at the current density of 50 mA/cm$^2$, luminance, and luminescence efficiency thereof were measured. The driving voltage of each of the light-emitting devices was measured utilizing a source meter (Keithley Instrument Inc., 2400 series), and the luminescence efficiency thereof was measured utilizing a luminescence efficiency measurement apparatus C9920-2-12 of Hamamatsu Photonics Inc. In evaluating the luminescence efficiency, the luminance/current density was measured utilizing a luminance meter that was calibrated for wavelength sensitivity, and the half lifespan was measured as the time taken to reach 50 % of the initial luminance at the current density of 100 mA/cm$^2$. Table 2 shows the evaluation results of the characteristics of the light-emitting devices.

**Table 2**

| | Hole transport layer | Driving voltage (V) | Current density (mA/cm$^2$) | Luminance (cd/m$^2$) | Efficiency (cd/A) | Emission color | Half lifespan (hr @100 mA/cm$^2$) |
|---|---|---|---|---|---|---|---|
| Example 1 | Compound 1 | 4.43 | 50 | 4570 | 9.14 | Blue | 420 |

(continued)

| | Hole transport layer | Driving voltage (V) | Current density (mA/cm2) | Luminance (cd/m2) | Efficiency (cd/A) | Emission color | Half lifespan (hr @100 mA/cm2) |
|---|---|---|---|---|---|---|---|
| Example 2 | Compound 4 | 4.44 | 50 | 4320 | 8.64 | Blue | 410 |
| Example 3 | Compound 21 | 4.50 | 50 | 4270 | 8.54 | Blue | 386 |
| Example 4 | Compound 52 | 4.45 | 50 | 4330 | 8.66 | Blue | 376 |
| Example 5 | Compound 68 | 4.71 | 50 | 4030 | 8.06 | Blue | 398 |
| Example 6 | Compound 93 | 4.42 | 50 | 4190 | 8.38 | Blue | 377 |
| Example 7 | Compound 104 | 4.64 | 50 | 4165 | 8.33 | Blue | 373 |
| Example 8 | Compound 126 | 4.56 | 50 | 4030 | 8.06 | Blue | 399 |
| Example 9 | Compound 148 | 4.35 | 50 | 4230 | 8.66 | Blue | 368 |
| Example 10 | Compound 170 | 4.91 | 50 | 3980 | 7.96 | Blue | 387 |
| Example 11 | Compound 188 | 4.31 | 50 | 4225 | 8.45 | Blue | 365 |
| Comparative Example 1 | NPB | 7.01 | 50 | 2645 | 5.29 | Blue | 258 |
| Comparative Example 2 | Compound A | 4.76 | 50 | 3315 | 6.63 | Blue | 223 |
| Comparative Example 3 | Compound B | 5.43 | 50 | 3615 | 7.23 | Blue | 290 |
| Comparative Example 4 | Compound C | 5.63 | 50 | 4005 | 8.01 | Blue | 346 |
| Comparative Example 5 | Compound D | 6.17 | 50 | 4055 | 8.11 | Blue | 316 |

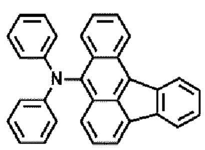

A

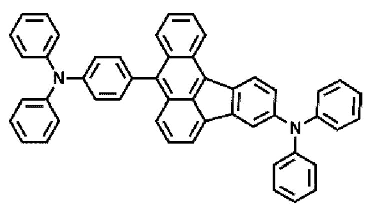

B

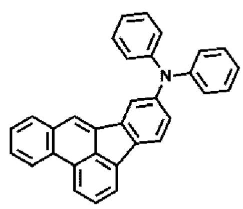

C

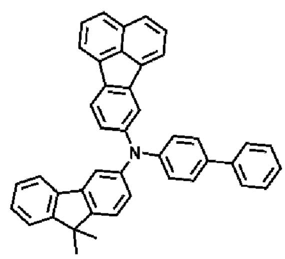

D .

Referring to Table 2, it was confirmed that the light-emitting devices of Examples 1 to 11 exhibited, as compared to the light-emitting devices of Comparative Examples 1 to 5, significantly improved half lifespan and/or low driving voltage, and excellent or suitable luminance and luminescence efficiency.

As described above, according to the one or more embodiments, provided are a novel condensed cyclic compound and a light-emitting device and an electronic apparatus that include the novel condensed cyclic compound, wherein the condensed cyclic compound disclosed herein has high thermal resistance and/or strong stability of structures, so that the light-emitting device and electronic apparatus that include the condensed cyclic compound may have improved luminescence efficiency and/or long lifespan.

Terms such as "substantially," "about," and "~" are used as terms of approximation and not as terms of degree, and are intended to account for the inherent deviations in measured or calculated values that would be recognized by those of ordinary skill in the art. They may be inclusive of the stated value and an acceptable range of deviation as determined by one of ordinary skill in the art, considering the limitations and error associated with measurement of that quantity. For example, "about" may refer to one or more standard deviations, or ± 30%, 20%, 10%, 5% of the stated value.

Numerical ranges disclosed herein include and are intended to disclose all subsumed sub-ranges of the same numerical precision. For example, a range of "1.0 to 10.0" includes all subranges having a minimum value equal to or greater than 1.0 and a maximum value equal to or less than 10.0, such as, for example, 2.4 to 7.6. Applicant therefore reserves the right to amend this specification, including the claims, to expressly recite any sub-range subsumed within the ranges expressly recited herein.

It should be understood that embodiments described herein should be considered in a descriptive sense only and not for purposes of limitation. Descriptions of features or aspects within each embodiment should typically be considered as being available for other similar features or aspects in other embodiments. While one or more embodiments have been described with reference to the drawings, it will be understood by those of ordinary skill in the art that various suitable changes in form and details may be made therein without departing from the scope as defined by the following claims .

**Claims**

**1.** A condensed cyclic compound represented by Formula 1:

**Formula 1**

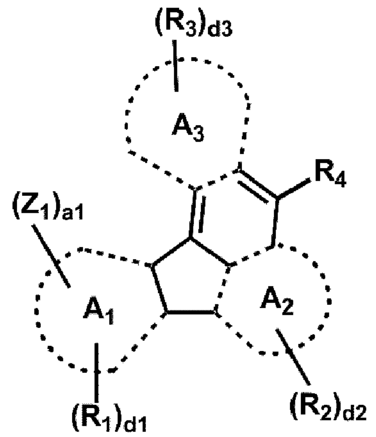

wherein in Formula 1,

$A_1$ to $A_3$ are each independently a $C_3$-$C_{60}$ carbocyclic group or a $C_1$-$C_{60}$ heterocyclic group,
$R_1$ to $R_4$ are each independently hydrogen, deuterium, -F, -Cl, -Br, -I, a hydroxyl group, a cyano group, a nitro group, a $C_1$-$C_{60}$ alkyl group unsubstituted or substituted with at least one $R_{10a}$, a $C_2$-$C_{60}$ alkenyl group unsubstituted or substituted with at least one $R_{10a}$, a $C_2$-$C_{60}$ alkynyl group unsubstituted or substituted with at least one $R_{10a}$, a $C_1$-$C_{60}$ alkoxy group unsubstituted or substituted with at least one $R_{10a}$, a $C_3$-$C_{60}$ carbocyclic group unsubstituted or substituted with at least one $R_{10a}$, a $C_1$-$C_{60}$ heterocyclic group unsubstituted or substituted with at least one $R_{10a}$, a $C_6$-$C_{60}$ aryloxy group unsubstituted or substituted with at least one $R_{10a}$, a $C_6$-$C_{60}$ arylthio group unsubstituted or substituted with at least one $R_{10a}$, -Si($Q_1$)($Q_2$)($Q_3$), -B($Q_1$)($Q_2$), - C(=O)($Q_1$), -S(=O)$_2$($Q_1$), or -P(=O)($Q_1$)($Q_2$),
d1 to d3 are each independently an integer from 0 to 10,
a1 is an integer from 1 to 4,
$Z_1$ is a group represented by Formula 2:

**Formula 2**

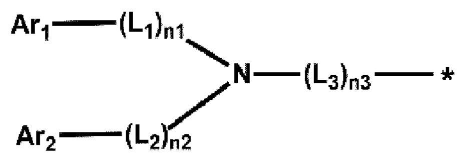

wherein in Formula 2,

$L_1$ to $L_3$ are each independently a single bond, a $C_5$-$C_{60}$ carbocyclic group unsubstituted or substituted with at least one $R_{10a}$, or a $C_1$-$C_{60}$ heterocyclic group unsubstituted or substituted with at least one $R_{10a}$,
n1 to n3 are each independently an integer from 1 to 3,
$Ar_1$ and $Ar_2$ are each independently a $C_5$-$C_{60}$ carbocyclic group unsubstituted or substituted with at least one $R_{10a}$ or a $C_1$-$C_{60}$ heterocyclic group unsubstituted or substituted with at least one $R_{10a}$,
* indicates a binding site to a neighboring atom; and
wherein each $R_{10a}$ is individually:
deuterium, -F, -Cl, -Br, -I, a hydroxyl group, a cyano group, or a nitro group;
a $C_1$-$C_{60}$ alkyl group, a $C_2$-$C_{60}$ alkenyl group, a $C_2$-$C_{60}$ alkynyl group, or a $C_1$-$C_{60}$ alkoxy group, each unsubstituted or substituted with deuterium, -F, -Cl, -Br, -I, a hydroxyl group, a cyano group, a nitro group, a $C_3$-$C_{60}$ carbocyclic group, a $C_1$-$C_{60}$ heterocyclic group, a $C_6$-$C_{60}$ aryloxy group, a $C_6$-$C_{60}$ arylthio group, -Si($Q_{11}$)($Q_{12}$)($Q_{13}$), - N($Q_{11}$)($Q_{12}$), -B($Q_{11}$)($Q_{12}$), -C(=O)($Q_{11}$), -S(=O)$_2$($Q_{11}$), -P(=O)($Q_{11}$)($Q_{12}$), or any combination thereof;
a $C_3$-$C_{60}$ carbocyclic group, a $C_1$-$C_{60}$ heterocyclic group, a $C_6$-$C_{60}$ aryloxy group, or a $C_6$-$C_{60}$ arylthio group, each unsubstituted or substituted with deuterium, -F, -Cl, - Br, -I, a hydroxyl group, a cyano group, a nitro group, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{60}$ carbocyclic group, a $C_1$-$C_{60}$ heterocyclic group, a $C_6$-$C_{60}$ aryloxy group, a $C_6$-$C_{60}$ arylthio group, - Si($Q_{21}$)($Q_{22}$)($Q_{23}$), -N($Q_{21}$)($Q_{22}$), -B($Q_{21}$)($Q_{22}$), -C(=O)($Q_{21}$), -S(=O)$_2$($Q_{21}$), - P(=O)($Q_{21}$)($Q_{22}$), or any combination thereof; or
-Si($Q_{31}$)($Q_{32}$)($Q_{33}$), -N($Q_{31}$)($Q_{32}$), -B($Q_{31}$)($Q_{32}$), -C(=O)($Q_{31}$), -S(=O)$_2$($Q_{31}$), or - P(=O)($Q_{31}$)($Q_{32}$), and
wherein $Q_1$ to $Q_3$, $Q_{11}$ to $Q_{13}$, $Q_{21}$ to $Q_{23}$, and $Q_{31}$ to $Q_{33}$ are each independently: hydrogen; deuterium; -F; -Cl; -Br; -I; a hydroxyl group; a cyano group; a nitro group; a $C_1$-$C_{60}$ alkyl group; a $C_2$-$C_{60}$ alkenyl group; a $C_2$-$C_{60}$ alkynyl group; a $C_1$-$C_{60}$ alkoxy group; or a $C_3$-$C_{60}$ carbocyclic group or a $C_1$-$C_{60}$ heterocyclic group, each unsubstituted or substituted with deuterium, -F, a cyano group, a $C_1$-$C_{60}$ alkyl group, a $C_1$-$C_{60}$ alkoxy group, a phenyl group, a biphenyl group, or any combination thereof.

2. The condensed cyclic compound of claim 1, wherein $A_1$ is a benzene group.

3. The condensed cyclic compound of claim 1 or 2, wherein a1 is 1.

4. The condensed cyclic compound of any one of claims 1 to 3, wherein the condensed cyclic compound represented by Formula 1 is represented by one of Formulae 1-1 to 1-4:

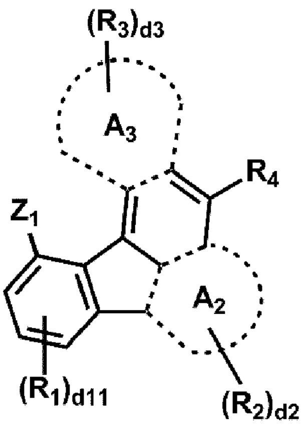

1-1

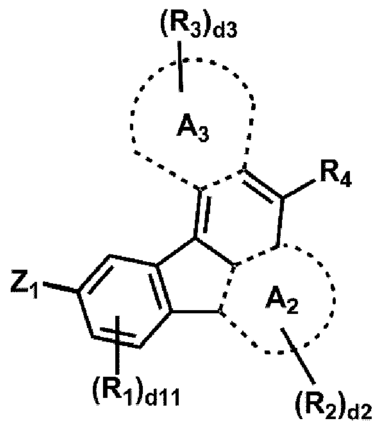

1-2

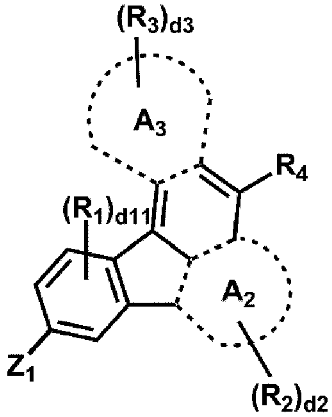

1-3

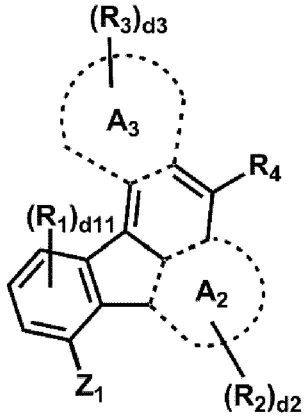

1-4

, and

wherein, in Formulae 1-1 to 1-4,

$Z_1$, $A_2$, $A_3$, $R_1$ to $R_4$, d2, and d3 are each independently same as described in claim 1, and d11 is an integer from 0 to 3.

**5.** The condensed cyclic compound of any one of claims 1 to 4, wherein $L_1$ to $L_3$ are each independently:

a single bond; or
a $\pi$ electron-rich $C_3$-$C_{60}$ cyclic group unsubstituted or substituted with at least one $R_{10a}$, and wherein each $R_{10a}$ is independently the same as described in claim 1

**6.** The condensed cyclic compound of any one of claims 1 to 4, wherein $L_1$ to $L_3$ are each independently:

a single bond; or
a group represented by one of Formulae 3-1 to 3-40:

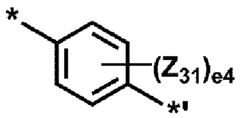

3-1

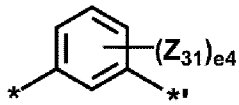

3-2

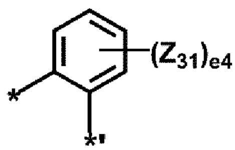

3-3

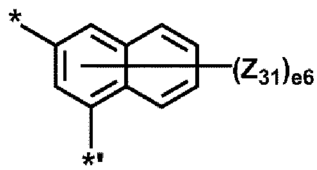

3-4

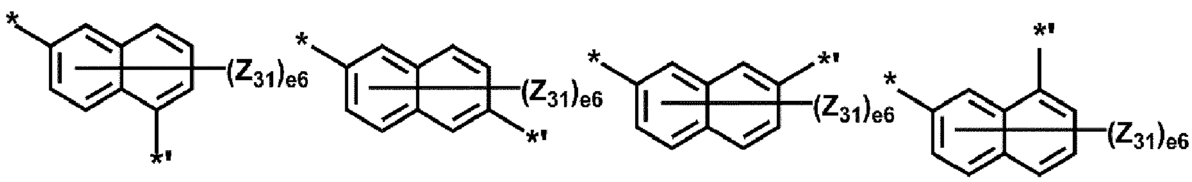

3-5 3-6 3-7 3-8

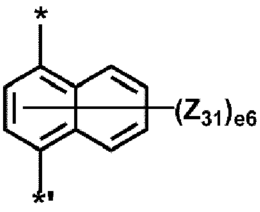

3-9

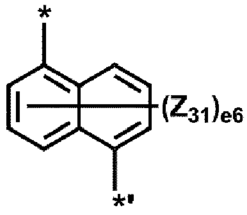

3-10

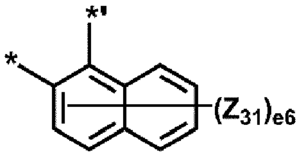

3-11

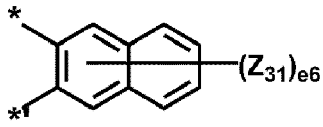

3-12

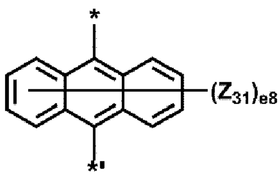

3-13

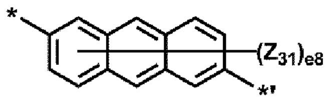

3-14

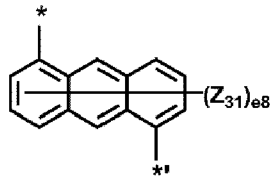

3-15

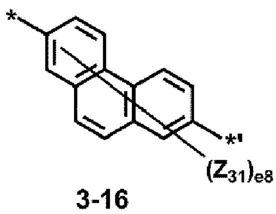

3-16

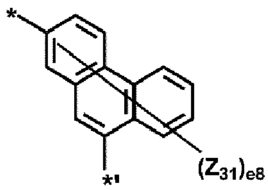

3-17

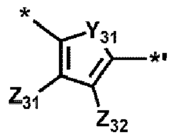

3-18

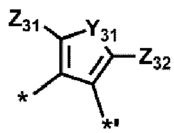

3-19

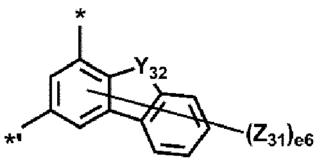

3-20

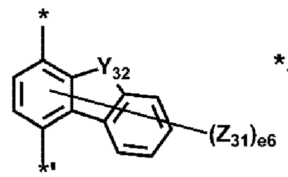

3-21

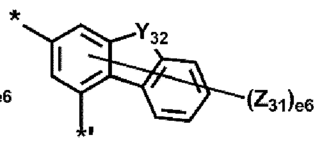

3-22

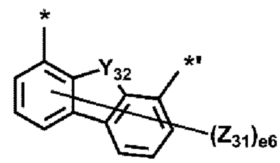

3-23

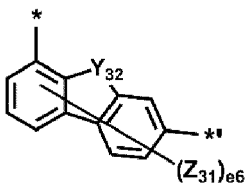

3-24

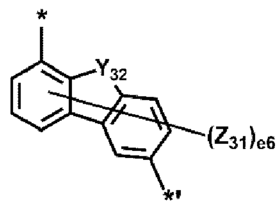

3-25

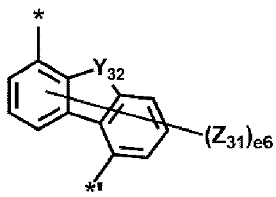

3-26

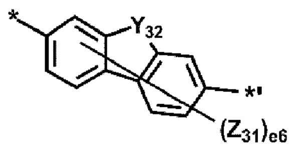

3-27

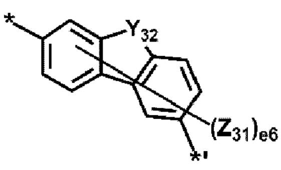

3-28

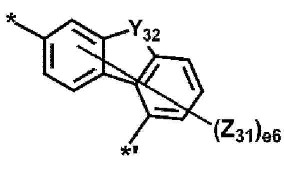

3-29

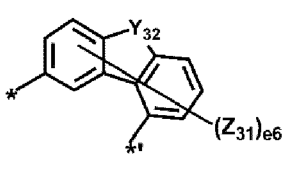

3-30

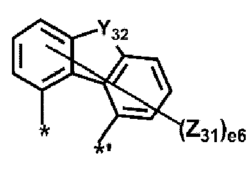

3-31

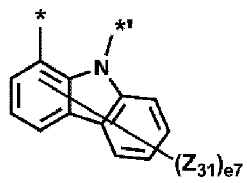 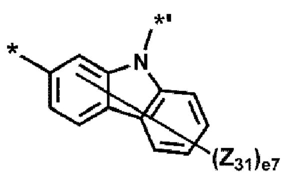 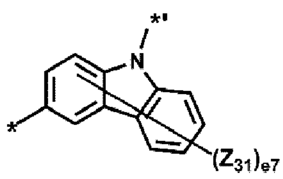 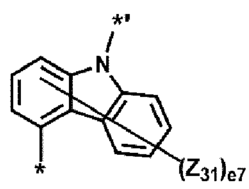

3-32 3-33 3-34 3-35

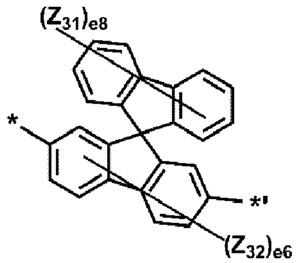 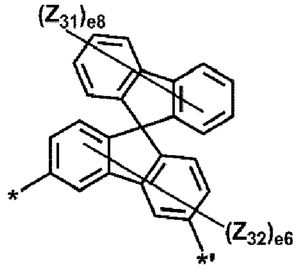 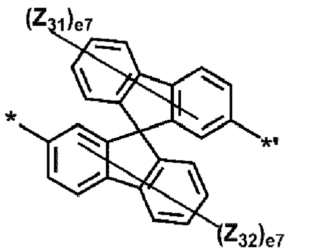 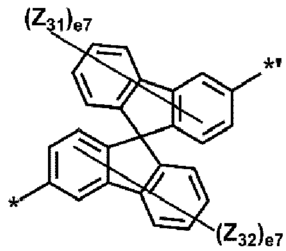

3-36 3-37 3-38 3-39

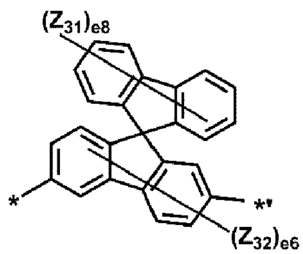

3-40

, and

wherein, in Formulae 3-1 to 3-40,

$Y_{31}$ is O or S,

$Y_{32}$ is O, S, $N(Z_{33})$, $C(Z_{33})(Z_{34})$, or $Si(Z_{33})(Z_{34})$,

$Z_{31}$ to $Z_{34}$ are each independently hydrogen, deuterium, -F, -Cl, -Br, -I, a hydroxyl group, a cyano group, a nitro group, an amidino group, a hydrazino group, a hydrazono group, $-CF_3$, $-CF_2H$, $-CFH_2$, a $C_1$-$C_{20}$ alkyl group, a $C_1$-$C_{20}$ alkoxy group, a cyclopentyl group, a cyclohexyl group, a cycloheptyl group, a cyclopentenyl group, a cyclohexenyl group, a phenyl group, a biphenyl group, a naphthyl group, a fluorenyl group, a spiro-bifluorenyl group, a spiro-fluorene-benzofluorenyl group, a benzofluorenyl group, a dibenzofluorenyl group, a phenalenyl group, a phenanthrenyl group, an anthracenyl group, a fluoranthenyl group, a triphenylenyl group, a pyridinyl group, a pyrimidinyl group, a pyrazinyl group, a triazinyl group, a quinolinyl group, an isoquinolinyl group, a benzoquinolinyl group, a naphthyridinyl group, a quinoxalinyl group, a quinazolinyl group, a phenanthridinyl group, an acridinyl group, a phenanthrolinyl group, a phenazinyl group, a carbazolyl group, a dibenzofuranyl group, a dibenzothiophenyl group, a dibenzosilolyl group, $-Si(Q_{31})(Q_{32})(Q_{33})$, $-N(Q_{31})(Q_{32})$, or - $B(Q_{31})(Q_{32})$,

e4 is an integer from 0 to 4,

e6 is an integer from 0 to 6,

e7 is an integer from 0 to 7,

e8 is an integer from 0 to 8,

$Q_{31}$ to $Q_{33}$ are each independently a $C_1$-$C_{10}$ alkyl group, a $C_1$-$C_{10}$ alkoxy group, a phenyl group, a biphenyl group, a terphenyl group, or a naphthyl group, and

* and *' each indicate a binding site to a neighboring atom.

**7.** The condensed cyclic compound of any one of claims 1 to 6, wherein, in Formula 2, $L_3$ is a single bond, and n3 is 1.

**8.** The condensed cyclic compound of any one of claims 1 to 7, wherein, in Formula 2, at least one of $L_1$ and $L_2$ is not

a single bond.

**9.** The condensed cyclic compound of any one of claims 1 to 8, wherein $Ar_1$ and $Ar_2$ are each independently a π electron-rich $C_3$-$C_{60}$ cyclic group unsubstituted or substituted with at least one $R_{10a}$, and

wherein each $R_{10a}$ is individually the same as described in claim 1; and/or wherein at least one of $Ar_1$ and $Ar_2$ is a carbazole group, a dibenzosilole group, a dibenzothiophene group, or a dibenzofuran group, each unsubstituted or substituted with at least one $R_{10a}$, and
each $R_{10a}$ is individually the same as described in claim 1.

**10.** The condensed cyclic compound of any one of claims 1 to 9, wherein $Ar_1$ and $Ar_2$ are each independently represented by one of Formulae 4-1 to 4-36:

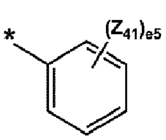
4-1

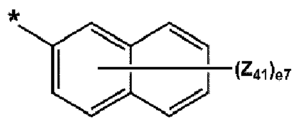
4-2

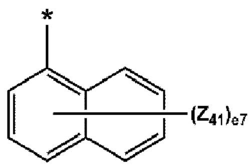
4-3

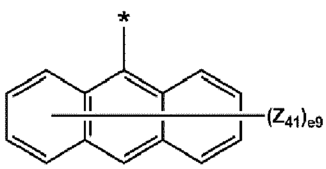
4-4

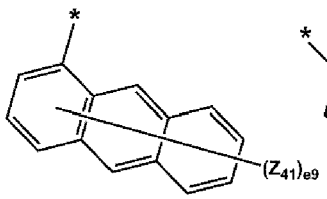
4-5

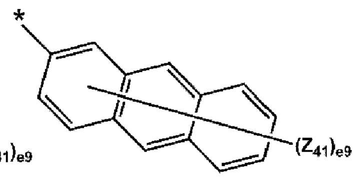
4-6

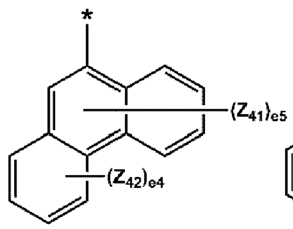
4-7

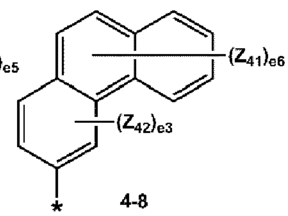
4-8

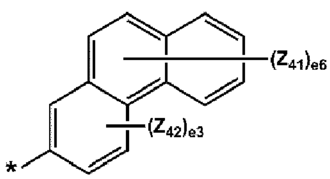
4-9

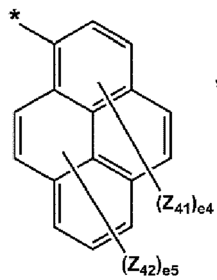
4-10

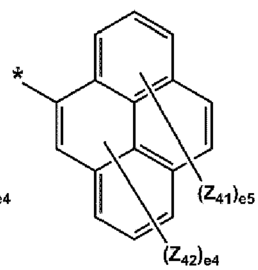
4-11

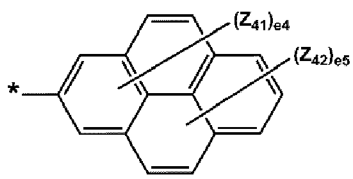
4-12

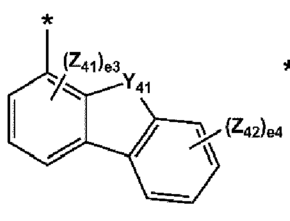
4-13

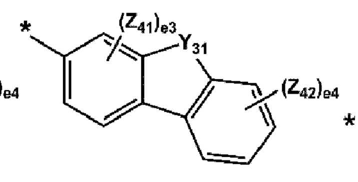
4-14

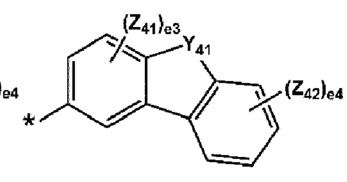
4-15

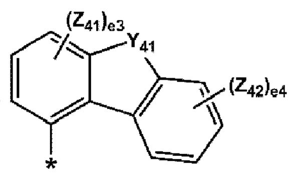
4-16

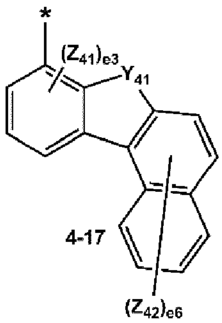
4-17

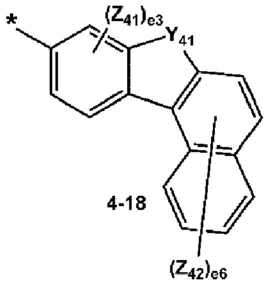
4-18

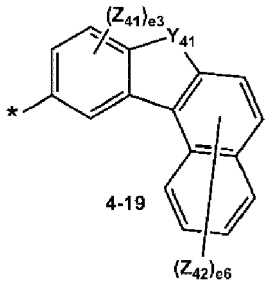
4-19

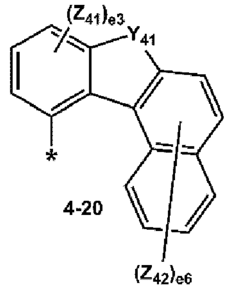
4-20

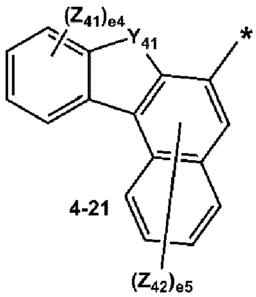
4-21

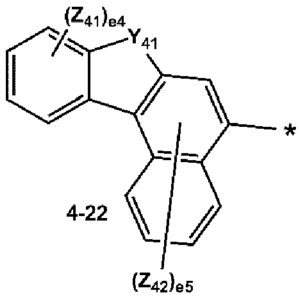
4-22

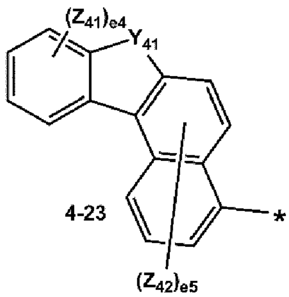
4-23

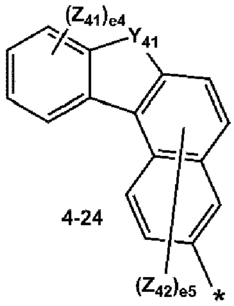
4-24

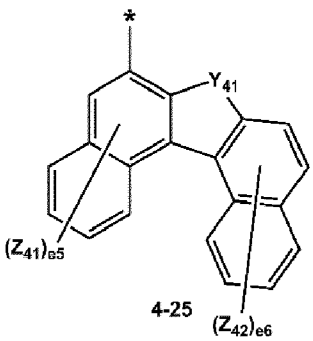
4-25

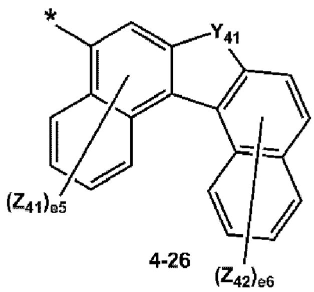
4-26

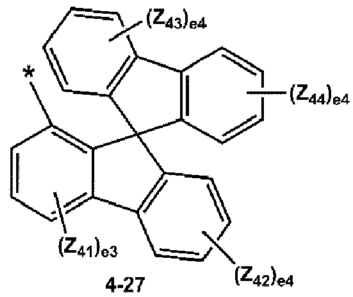
4-27

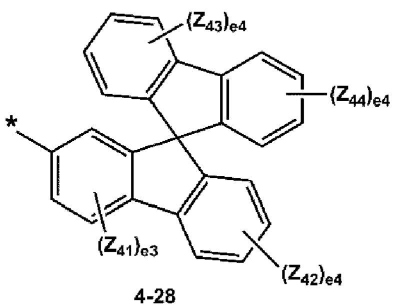
4-28

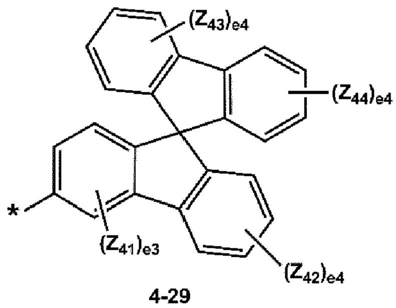
4-29

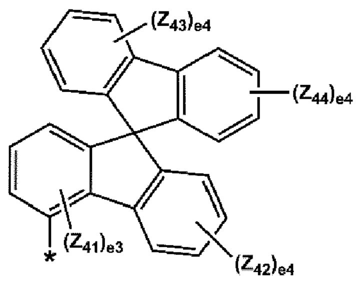
4-30

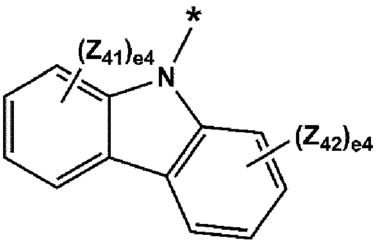
4-31

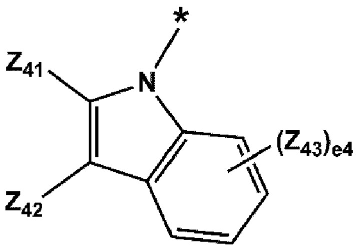
4-32

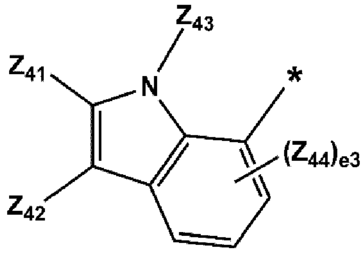
4-33

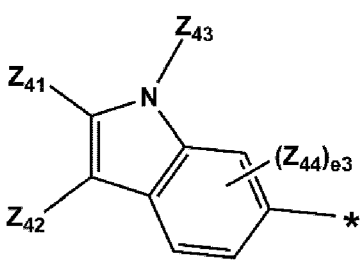

4-34

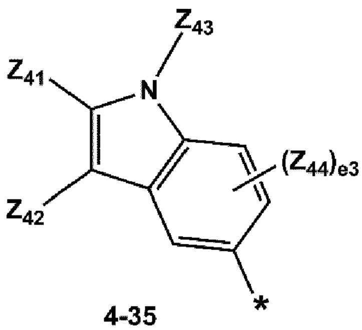

4-35

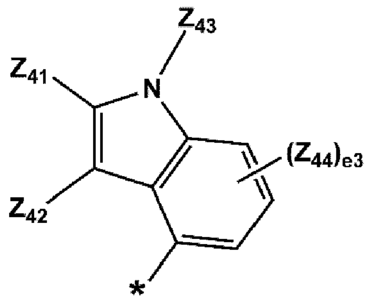

4-36

, and
wherein, in Formulae 4-1 to 4-36,

Y41 is O, S, N(Z45), C(Z45)(Z46), or Si(Z45)(Z46),
$Z_{41}$ to $Z_{46}$ are each independently hydrogen, deuterium, -F, -Cl, -Br, -I, a hydroxyl group, a cyano group, a nitro group, an amidino group, a hydrazino group, a hydrazono group, $-CF_3$, $-CF_2H$, $-CFH_2$, a $C_1$-$C_{20}$ alkyl group, a $C_1$-$C_{20}$ alkoxy group, a cyclopentyl group, a cyclohexyl group, a cycloheptyl group, a cyclopentenyl group, a cyclohexenyl group, a phenyl group, a biphenyl group, a naphthyl group, a fluorenyl group, a spiro-bifluorenyl group, a spiro-fluorene-benzofluorenyl group, a benzofluorenyl group, a dibenzofluorenyl group, a phenalenyl group, a phenanthrenyl group, an anthracenyl group, a fluoranthenyl group, a triphenylenyl group, a pyridinyl group, a pyrimidinyl group, a pyrazinyl group, a triazinyl group, a quinolinyl group, an isoquinolinyl group, a benzoquinolinyl group, a naphthyridinyl group, a quinoxalinyl group, a quinazolinyl group, a phenanthridinyl group, an acridinyl group, a phenanthrolinyl group, a phenazinyl group, a carbazolyl group, a dibenzofuranyl group, a dibenzothiophenyl group, a dibenzosilolyl group, $-Si(Q_{31})(Q_{32})(Q_{33})$, $-N(Q_{31})(Q_{32})$, or - $B(Q_{31})(Q_{32})$,
e2 is an integer from 0 to 2,
e3 is an integer from 0 to 3,
e4 is an integer from 0 to 4,
e5 is an integer from 0 to 5,
e6 is an integer from 0 to 6,
e7 is an integer from 0 to 7,
e9 is an integer from 0 to 9,
$Q_{31}$ to $Q_{33}$ are each independently a $C_1$-$C_{10}$ alkyl group, a $C_1$-$C_{10}$ alkoxy group, a phenyl group, a biphenyl group, a terphenyl group, or a naphthyl group, and
* indicates a binding site to a neighboring atom.

**11.** The condensed cyclic compound of any one of claims 1 to 10, wherein, in Formula 2, $*-(L_1)_{n1}-Ar_1$ and $*-(L_2)_{n2}-Ar_2$ are different from each other, and
* indicates a binding site to N in Formula 2.

**12.** The condensed cyclic compound of any one of claims 1 to 11, wherein $R_1$ to $R_4$ are each independently: hydrogen, deuterium, -F, -Cl, -Br, -I, a hydroxyl group, a cyano group, a nitro group, an amidino group, a hydrazine group, a hydrazone group, a $C_1$-$C_{20}$ alkyl group, or a $C_1$-$C_{20}$ alkoxy group;

a $C_1$-$C_{20}$ alkyl group or a $C_1$-$C_{20}$ alkoxy group, each substituted with deuterium, - F, -Cl, -Br, -I, $-CD_3$, $-CD_2H$, $-CDH_2$, $-CF_3$, $-CF_2H$, $-CFH_2$, a hydroxyl group, a cyano group, a nitro group, an amidino group, a hydrazine group, a hydrazone group, a $C_1$-$C_{10}$ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a biphenyl group, a naphthyl group, a pyridinyl group, a pyrimidinyl group, or any combination thereof;
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a biphenyl group, a $C_1$-$C_{10}$ alkylphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group,

a quinolinyl group, an isoquinolinyl group, a benzoquinolinyl group, a quinoxalinyl group, a quinazolinyl group, a cinnolinyl group, a carbazolyl group, a phenanthrolinyl group, a benzoimidazolyl group, a benzofuranyl group, a benzothiophenyl group, a benzoisothiazolyl group, a benzoxazolyl group, an benzoisoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, an azacarbazolyl group, an azadibenzofuranyl group, an azadibenzothiophenyl group, an azafluorenyl group, or an azadibenzosilolyl group, each unsubstituted or substituted with deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amidino group, a hydrazine group, a hydrazone group, a $C_1$-$C_{20}$ alkyl group, a $C_1$-$C_{20}$ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a biphenyl group, a $C_1$-$C_{10}$ alkylphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzoimidazolyl group, a benzofuranyl group, a benzothiophenyl group, a benzoisothiazolyl group, a benzoxazolyl group, an benzoisoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, an azacarbazolyl group, an azadibenzofuranyl group, an azadibenzothiophenyl group, an azafluorenyl group, an azadibenzosilolyl group, $-Si(Q_{31})(Q_{32})(Q_{33})$, $-B(Q_{31})(Q_{32})$, $-P(Q_{31})(Q_{32})$, $-C(=O)(Q_{31})$, $-S(=O)_2(Q_{31})$, $-P(=O)(Q_{31})(Q_{32})$, or any combination thereof; or

$-Si(Q_1)(Q_2)(Q_3)$, $-B(Q_1)(Q_2)$, $-C(=O)(Q_1)$, $-S(=O)_2(Q_1)$, or $-P(=O)(Q_1)(Q_2)$, and

$Q_1$ to $Q_3$ and $Q_{31}$ to $Q_{33}$ are each independently:

-CH3, $-CD_3$, $-CD_2H$, $-CDH_2$, $-CH_2CH_3$, $-CH_2CD_3$, $-CH_2CD_2H$, $-CH_2CDH_2$, - $CHDCH_3$, $-CHDCD_2H$, $-CHDCDH_2$, $-CHDCD_3$, $-CD_2CD_3$, $-CD_2CD_2H$, or $-CD_2CDH_2$; or

an n-propyl group, an isopropyl group, an n-butyl group, an isobutyl group, a sec-butyl group, a tert-butyl group, an n-pentyl group, an isopentyl group, a sec-pentyl group, a tert-pentyl group, a phenyl group, a naphthyl group, a pyridinyl group, a pyrimidinyl group, a pyridazinyl group, a pyrazinyl group, a triazinyl group, a carbazolyl group, a dibenzofuranyl group, or a dibenzothiophenyl group, each unsubstituted or substituted with deuterium, a $C_1$-$C_{10}$ alkyl group, a phenyl group, a biphenyl group, a pyridinyl group, a pyrimidinyl group, pyridazinyl group, a pyrazinyl group, a triazinyl group, a carbazolyl group, a dibenzofuranyl group, a dibenzothiophenyl group, or any combination thereof.

**13.** The condensed cyclic compound of any one of claims 1 to 12, wherein the condensed cyclic compound is one of Compounds 1 to 192:

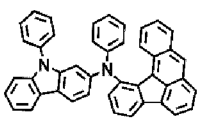
1

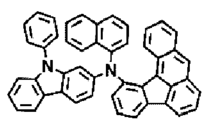
2

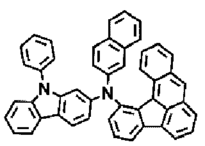
3

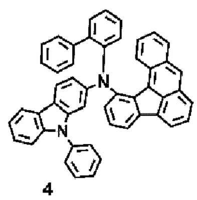
4

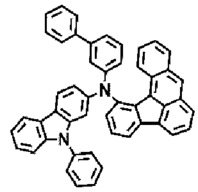
5

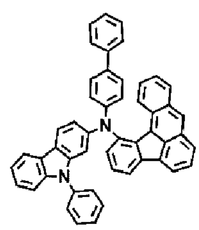
6

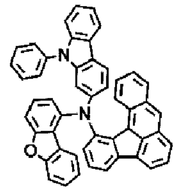
7

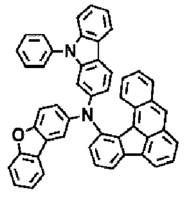
8

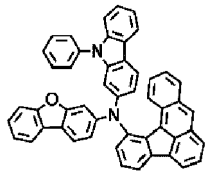
9

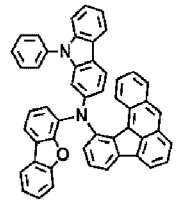
10

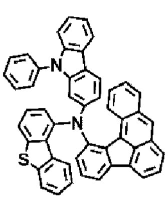
11

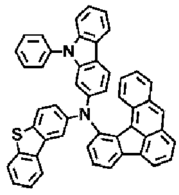
12

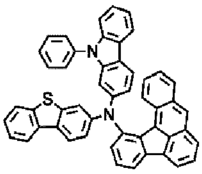
13

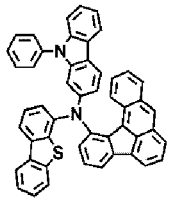
14

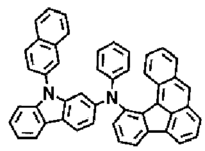
15

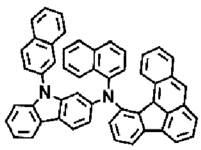
16

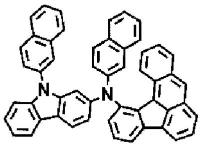
17

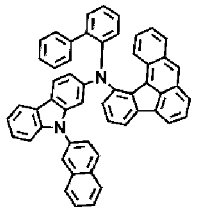
18

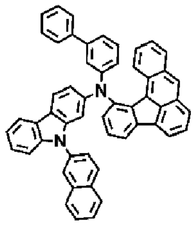
19

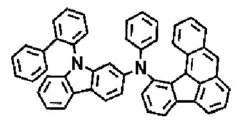
20

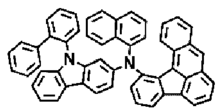
21

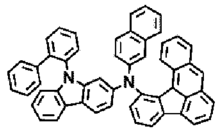
22

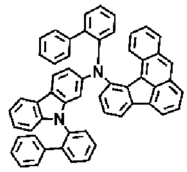
23

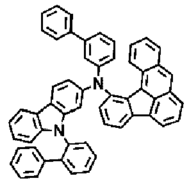
24

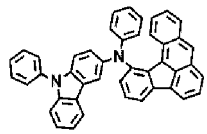
25

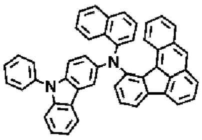
26

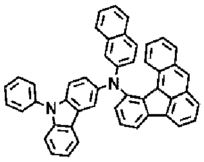
27

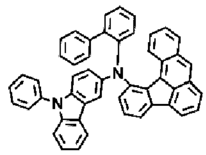
28

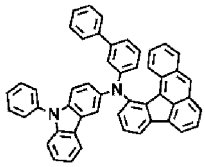
29

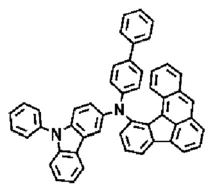
30

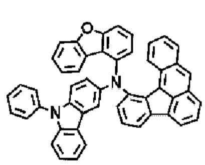
31

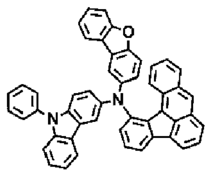
32

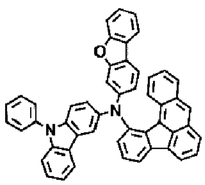
33

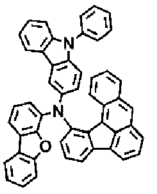
34

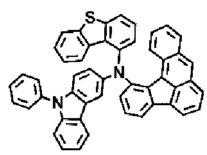
35

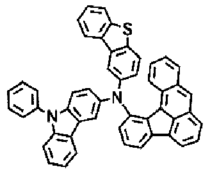
36

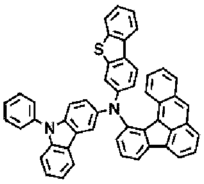
37

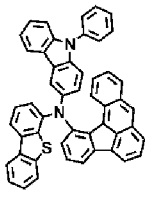
38

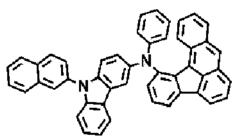
39

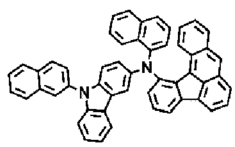
40

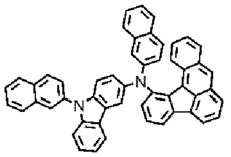

41

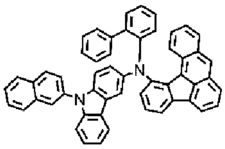

42

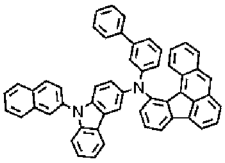

43

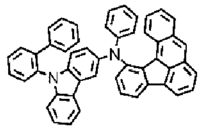

44

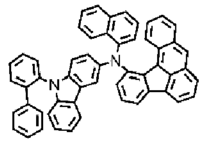

45

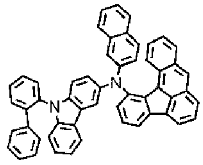

46

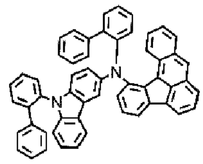

47

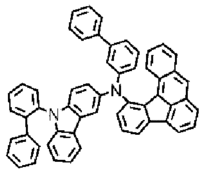

48

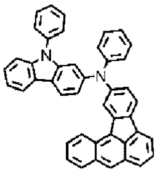

49

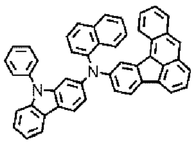

50

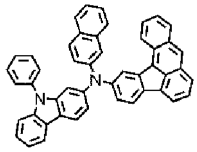

51

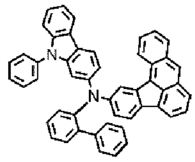

52

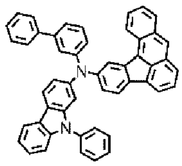

53

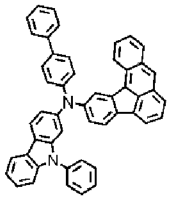

54

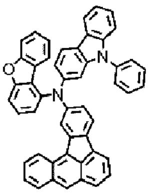

55

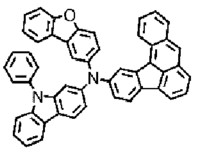

56

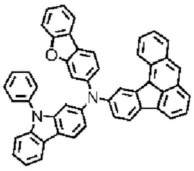

57

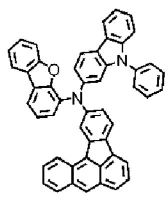

58

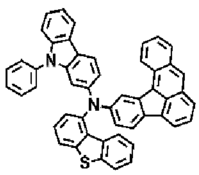

59

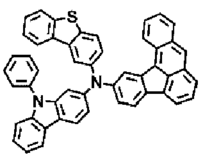

60

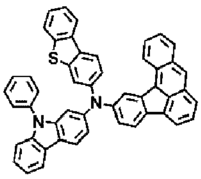

61

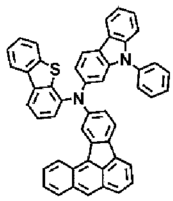

62

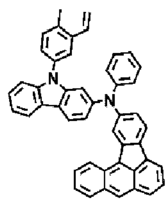

63

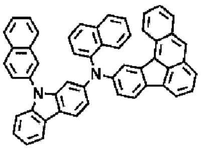

64

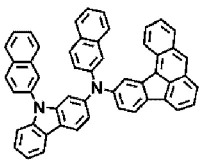

65

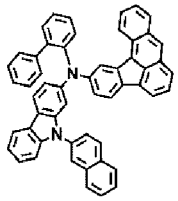

66

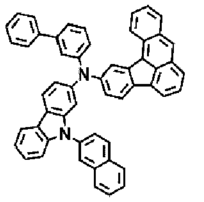

67

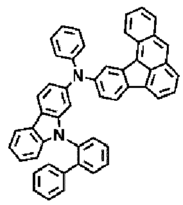

68

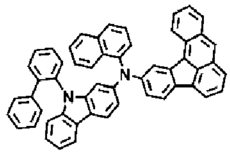
69

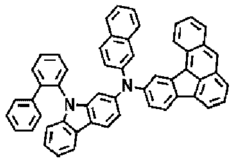
70

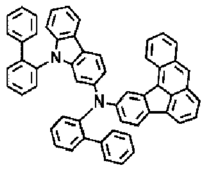
71

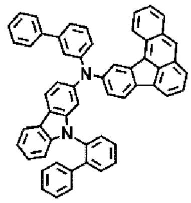
72

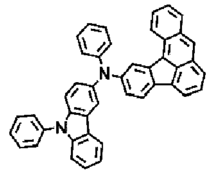
73

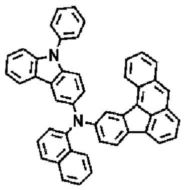
74

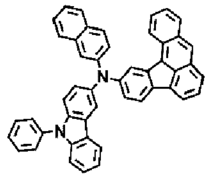
75

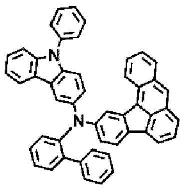
76

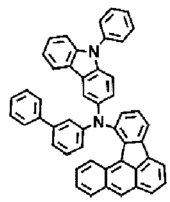
77

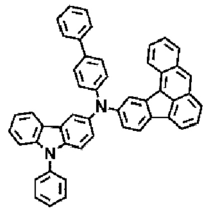
78

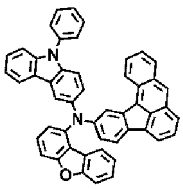
79

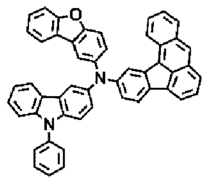
80

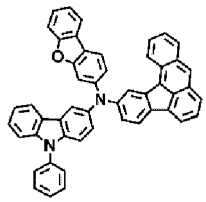
81

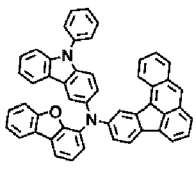
82

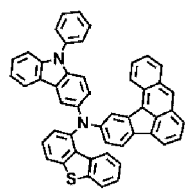
83

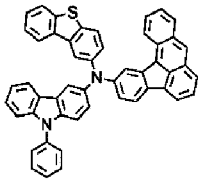
84

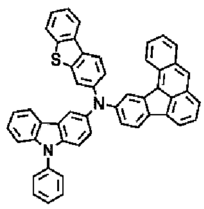
85

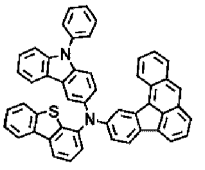
86

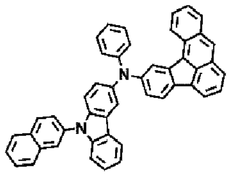
87

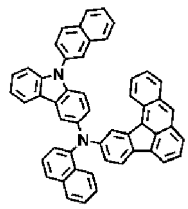
88

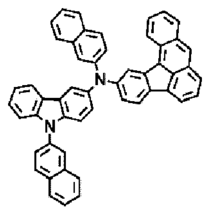
89

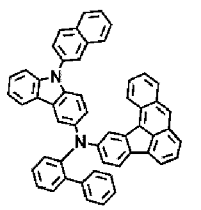
90

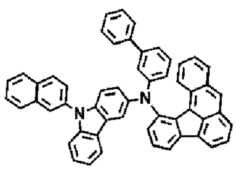
91

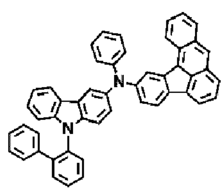
92

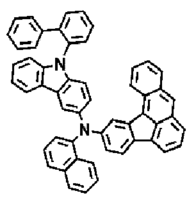
93

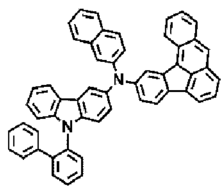
94

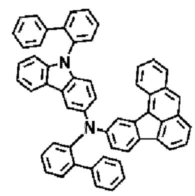
95

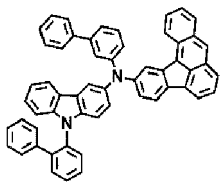
96

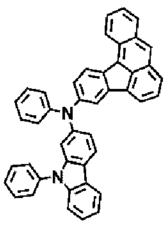
97

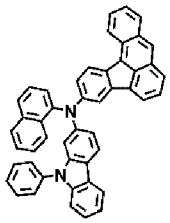
98

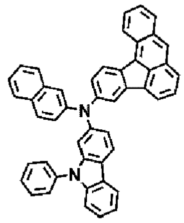
99

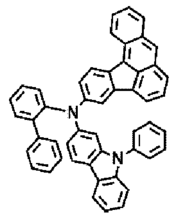
100

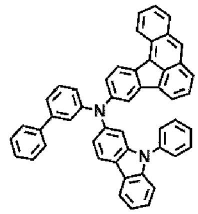
101

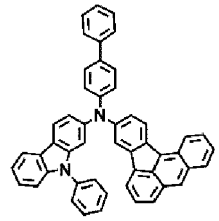
102

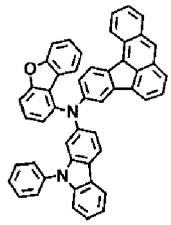
103

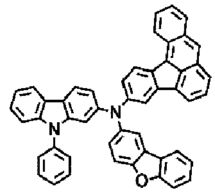
104

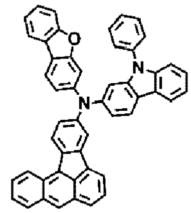
105

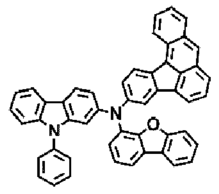
106

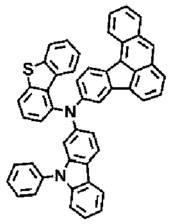
107

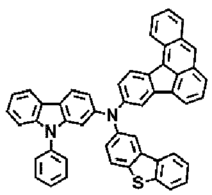
108

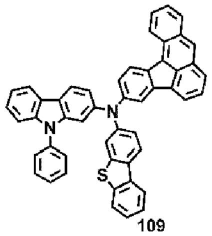
109

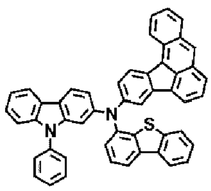
110

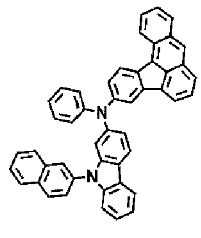
111

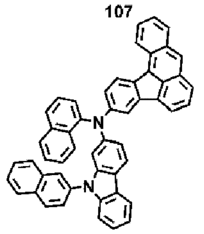
112

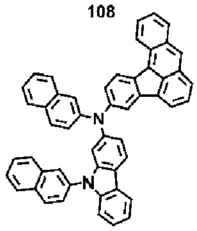
113

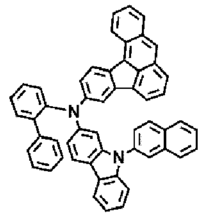
114

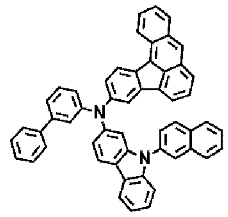
115

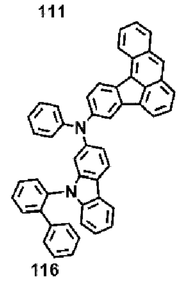
116

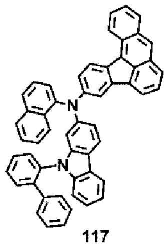
117
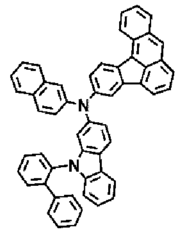
118
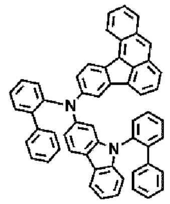
119
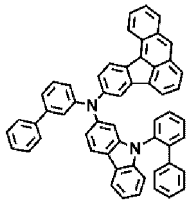
120
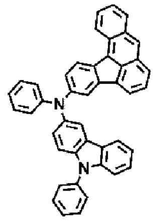
121

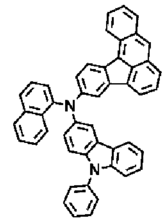
122
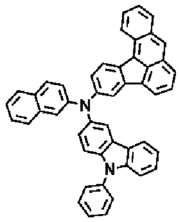
123
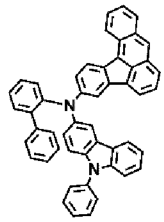
124
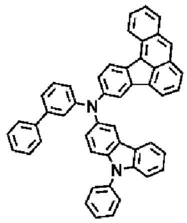
125
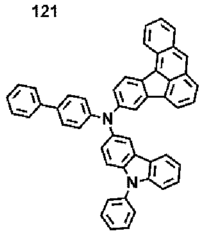
126

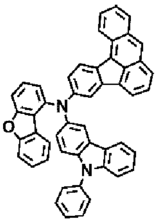
127
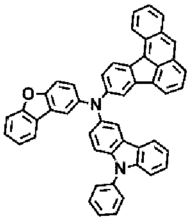
128
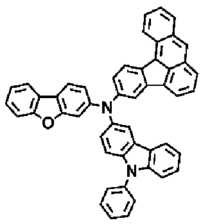
129
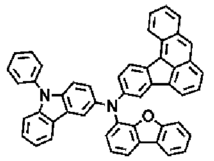
130
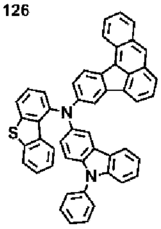
131

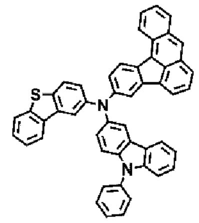
132
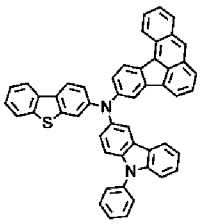
133
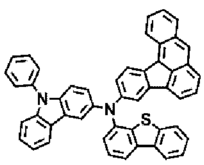
134
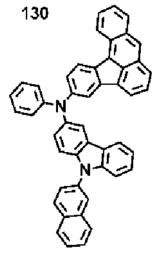
135
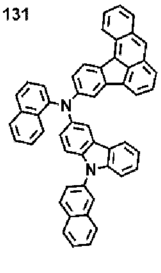
136

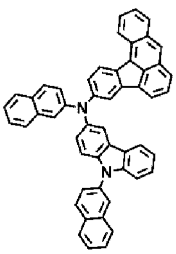
137
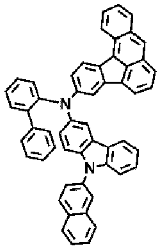
138
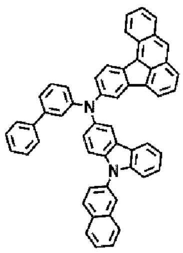
139
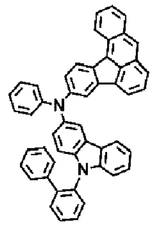
140
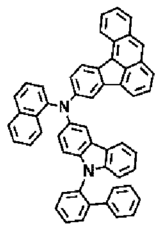
141

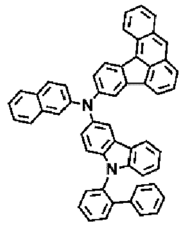
142

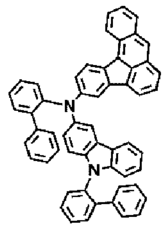
143

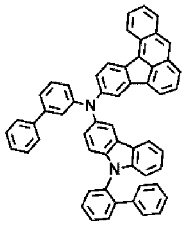
144

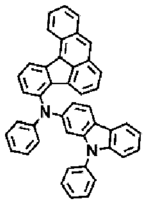
145

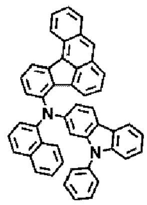
146

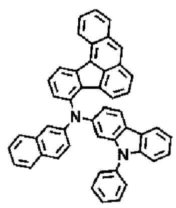
147

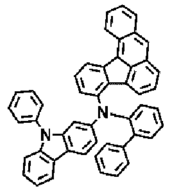
148

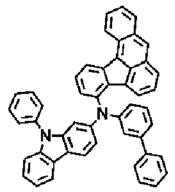
149

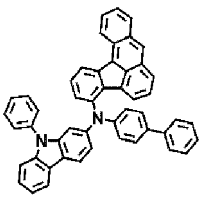
150

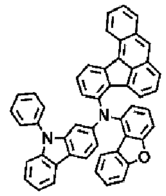
151

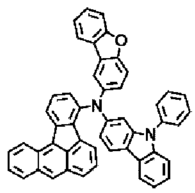
152

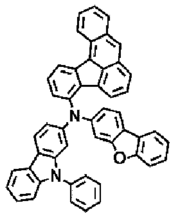
153

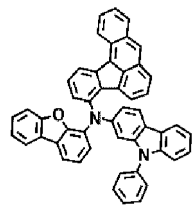
154

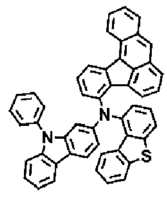
155

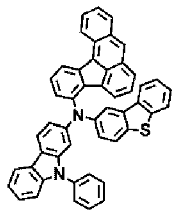
156

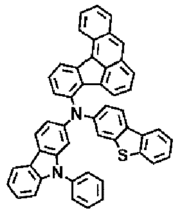
157

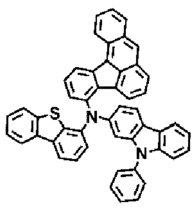
158

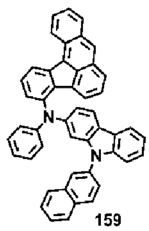
159

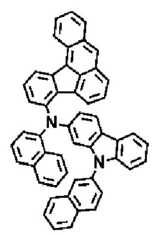
160

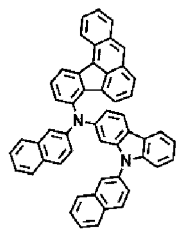
161

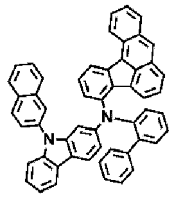
162

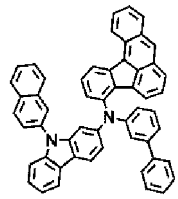
163

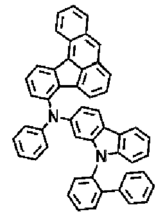
164

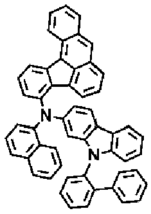
165

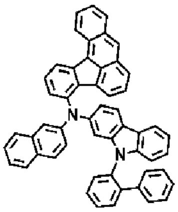
166

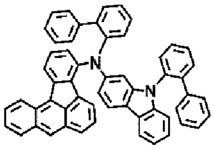
167

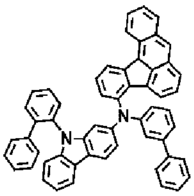
168

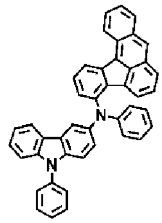
169

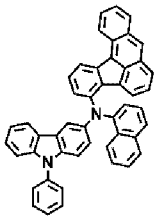
170

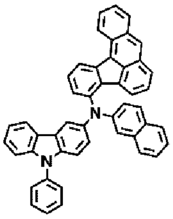
171

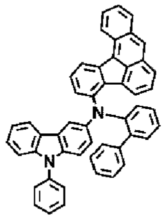
172

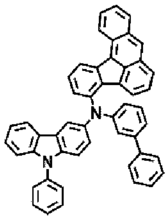
173

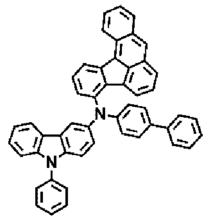
174

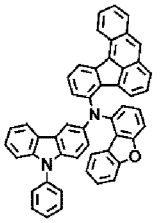
175

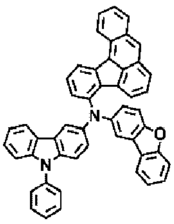
176

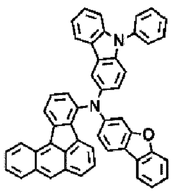
177

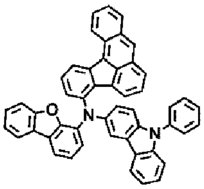
178

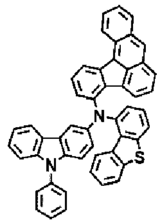
179

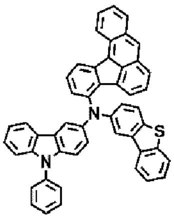
180

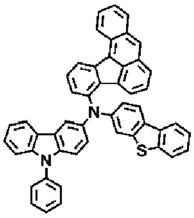
181

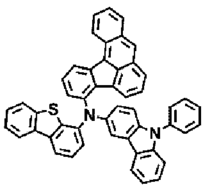
182

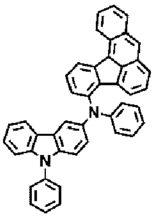
183

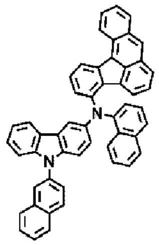
184

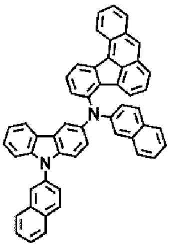
185

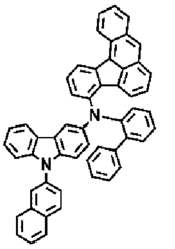
186

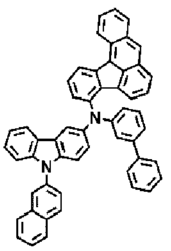
187

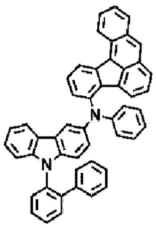
188

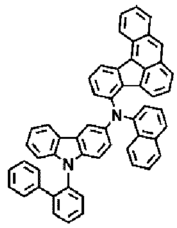
189

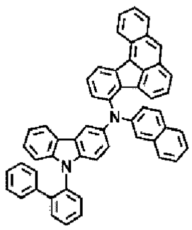

190

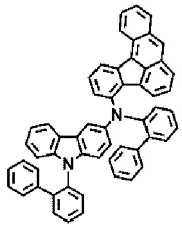

191

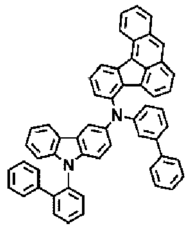

192

**14.** A light-emitting device comprising:

a first electrode;
a second electrode facing the first electrode; and
an interlayer between the first electrode and the second electrode and comprising an emission layer,
wherein the interlayer comprises at least one condensed cyclic compound according to any one of claims 1 to 13; preferably:
wherein the first electrode is an anode,
the second electrode is a cathode,
the interlayer further comprises a hole transport region between the emission layer and the first electrode,
the interlayer further comprises an electron transport region between the emission layer and the second electrode,
the hole transport region comprises a hole injection layer, a hole transport layer, an emission auxiliary layer, an electron blocking layer, or any combination thereof, and
the electron transport region comprises a buffer layer, a hole blocking layer, an electron control layer, an electron transport layer, an electron injection layer, or any combination thereof; and/or
wherein the hole transport region comprises the at least one condensed cyclic compound; and/or
wherein the emission layer is configured to emit blue light or blue-green light.

**15.** An electronic apparatus comprising:

the light-emitting device of claim 14; and
a thin-film transistor, wherein:

the thin-film transistor comprises a source electrode and a drain electrode, and
the first electrode of the light-emitting device is electrically connected to the source electrode or the drain electrode; preferably:

wherein the electronic apparatus further comprises a color filter, a color conversion layer, a touch screen layer, a polarizing layer, or any combination thereof.

FIG. 1

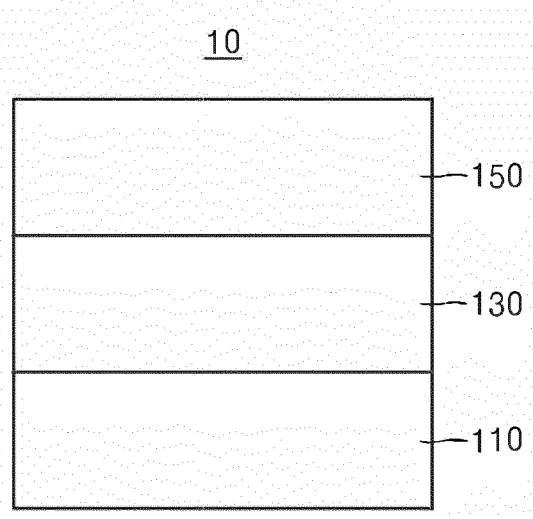

FIG. 2

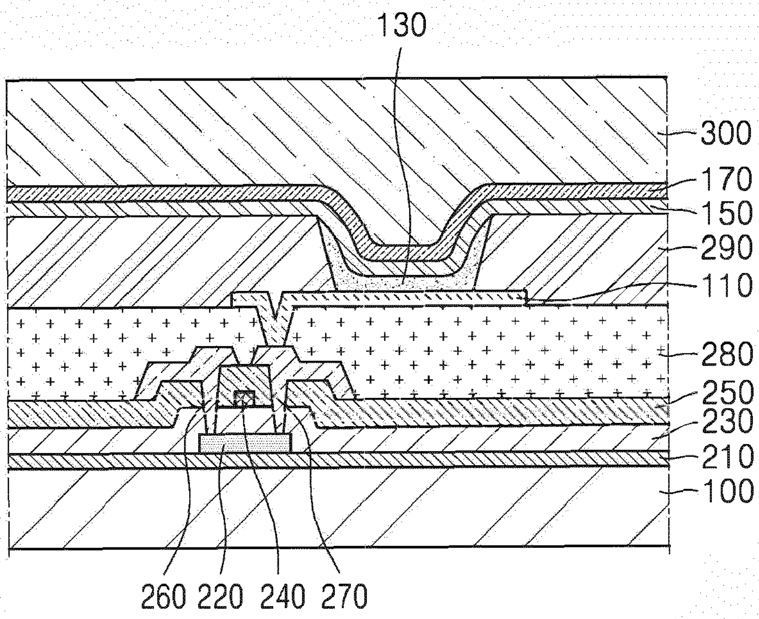

FIG. 3

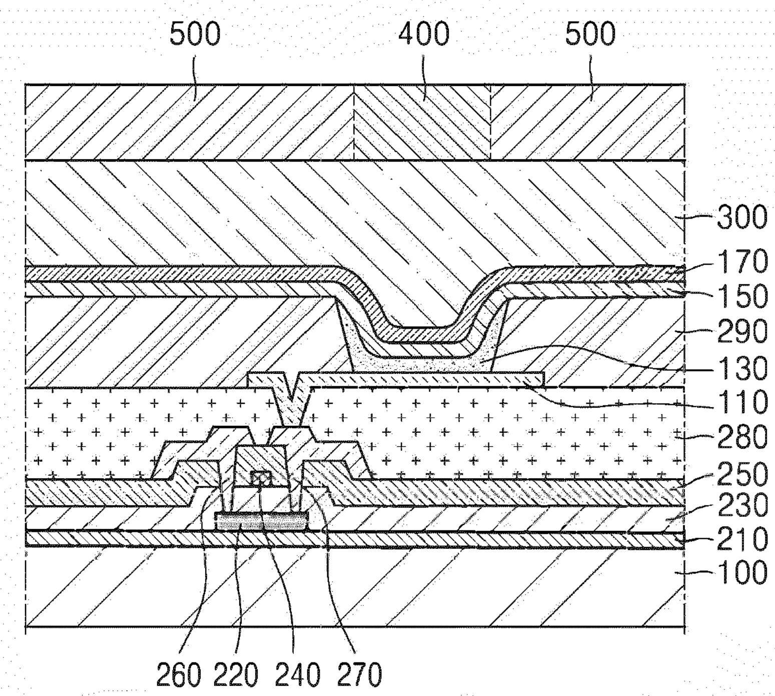
500
400
500
300
170
150
290
130
110
280
250
230
210
100
260 220 240 270

Europäisches Patentamt
European Patent Office
Office européen des brevets

# EUROPEAN SEARCH REPORT

Application Number
EP 22 16 5019

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br>A | WO 2008/140132 A1 (CANON KK [JP]; YAMADA NAOKI [JP] ET AL.) 20 November 2008 (2008-11-20)<br>* compound B-6 in page 12, claims 1 and 3 * | 1-12,14, 15<br><br>13 | INV.<br>C07D209/88<br>C07D405/12<br>C07D409/12<br>H01L51/00<br>H05B45/60 |
| X | EP 3 483 156 A1 (CYNORA GMBH [DE]) 15 May 2019 (2019-05-15)<br>* third compound in page 32, paragraphs [0081]-[0082] * | 1-7, 9-12,14, 15 | |
| X | HUANG T H ET AL: "Benzo.alpha.aceanthrylene Derivatives for Red-Emitting Electroluminescent Materials", CHEMISTRY OF MATERIALS, AMERICAN CHEMICAL SOCIETY, US, vol. 15, no. 25, 1 January 2003 (2003-01-01), pages 4854-4862, XP008104169, ISSN: 0897-4756, DOI: 10.1021/CM034631E [retrieved on 2003-11-14]<br>* compounds 2, 4, 5 and 6 in Figure 1, page 4856 * | 1-7, 9-12,14, 15 | TECHNICAL FIELDS SEARCHED (IPC)<br>C07D<br>H05B<br>H01L |
| X | JP 5 363164 B2 (IDEMITSU KOSAN CO) 11 December 2013 (2013-12-11)<br>* compounds in page (9), claim 1 * | 1-7,9, 10,12, 14,15 | |

The present search report has been drawn up for all claims

3

| Place of search | Date of completion of the search | Examiner |
|---|---|---|
| Munich | 1 September 2022 | Sahagún Krause, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 22 16 5019

This annex lists the patent family members relating to the patent documents cited in the above-mentioned European search report. The members are as contained in the European Patent Office EDP file on
The European Patent Office is in no way liable for these particulars which are merely given for the purpose of information.

01-09-2022

| Patent document cited in search report | Publication date | Patent family member(s) | Publication date |
|---|---|---|---|
| WO 2008140132 A1 | 20-11-2008 | CN 101547877 A | 30-09-2009 |
| | | EP 2160373 A1 | 10-03-2010 |
| | | JP 5361238 B2 | 04-12-2013 |
| | | JP 2008308486 A | 25-12-2008 |
| | | KR 20090122921 A | 01-12-2009 |
| | | US 2011049479 A1 | 03-03-2011 |
| | | WO 2008140132 A1 | 20-11-2008 |
| EP 3483156 A1 | 15-05-2019 | CN 109942551 A | 28-06-2019 |
| | | EP 3483156 A1 | 15-05-2019 |
| | | JP 6720272 B2 | 08-07-2020 |
| | | JP 2019085410 A | 06-06-2019 |
| | | KR 20190052625 A | 16-05-2019 |
| | | KR 20200143337 A | 23-12-2020 |
| | | TW 202017919 A | 16-05-2020 |
| | | US 2019177303 A1 | 13-06-2019 |
| JP 5363164 B2 | 11-12-2013 | JP 5363164 B2 | 11-12-2013 |
| | | JP 2010238924 A | 21-10-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020210087394